(19) Europäisches Patentamt
European Patent Office
Office européen des brevets

(11) EP 4 772 507 A1

(12) EUROPEAN PATENT APPLICATION
published in accordance with Art. 153(4) EPC

(43) Date of publication:
**08.07.2026 Bulletin 2026/28**

(21) Application number: **24872412.2**

(22) Date of filing: **26.09.2024**

(51) International Patent Classification (IPC):
***C07D 471/04*** (2006.01) ***A61K 31/437*** (2006.01)
***A61K 31/496*** (2006.01) ***A61K 31/506*** (2006.01)
***A61K 31/4375*** (2006.01) ***A61K 31/4545*** (2006.01)
***A61K 31/4725*** (2006.01) ***A61P 29/00*** (2006.01)
***A61P 35/00*** (2006.01) ***A61P 35/02*** (2006.01)
***A61P 37/02*** (2006.01) ***A61P 37/04*** (2006.01)
***A61P 37/06*** (2006.01) ***A61P 43/00*** (2006.01)
***C07D 519/00*** (2006.01)

(52) Cooperative Patent Classification (CPC):
**A61K 31/437; A61K 31/4375; A61K 31/4545; A61K 31/4725; A61K 31/496; A61K 31/506; A61P 29/00; A61P 35/00; A61P 35/02; A61P 37/02; A61P 37/04; A61P 37/06; A61P 43/00; C07D 471/04; C07D 519/00**

(86) International application number:
**PCT/JP2024/034463**

(87) International publication number:
**WO 2025/070627 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.09.2023 PCT/JP2023/035221**

(71) Applicant: **Nippon Shinyaku Co., Ltd.**
**Kyoto-shi**
**Kyoto 601-8550 (JP)**

(72) Inventors:
- **NAGASAWA, Tomohiro**
  **Kyoto-shi, Kyoto 601-8550 (JP)**
- **HATA, Kenji**
  **Kyoto-shi, Kyoto 601-8550 (JP)**
- **TANAKA, Toru**
  **Kyoto-shi, Kyoto 601-8550 (JP)**
- **MURATA, Akihiro**
  **Kyoto-shi, Kyoto 601-8550 (JP)**
- **TSUJI, Takashi**
  **Kyoto-shi, Kyoto 601-8550 (JP)**
- **NISHIBAYASHI, Kazuya**
  **Kyoto-shi, Kyoto 601-8550 (JP)**
- **KANEKO, Hiroki**
  **Kyoto-shi, Kyoto 601-8550 (JP)**
- **YAMADA, Kaori**
  **Kyoto-shi, Kyoto 601-8550 (JP)**

(74) Representative: **Hoffmann Eitle**
**Patent- und Rechtsanwälte PartmbB**
**Arabellastraße 30**
**81925 München (DE)**

# (54) AZAINDOLE COMPOUND AND MEDICAL DRUG

(57) The present invention relates to a compound represented by the following General Formula (1) or a pharmaceutically acceptable salt thereof. In General Formula (1), $R^1$ represents a group represented by General Formula (2) or General Formula (3); $R^2$ represents a hydrogen atom, or the like; $R^3$ represents a substituted or unsubstituted aryl group, or the like; $R^4$ represents halogen; $R^5$ represents a substituted or unsubstituted aryl group, or the like; $R^6$ and $R^7$ each independently represent a hydrogen atom, or the like; $R^8$ represents a substituted or unsubstituted aryl group, or the like; $R^9$ and $R^{10}$ each independently represent a substituted or unsubstituted alkyl group, or the like, and $R^9$ and $R^{10}$ may be linked to each other to form a ring; and $R^{11}$ represents a substituted or unsubstituted aryl group, or the like.

EP 4 772 507 A1

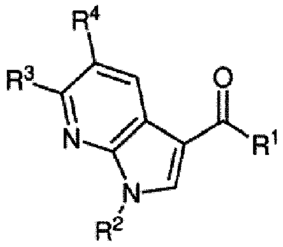

(1)

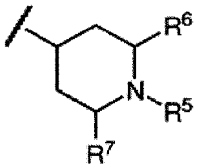

(2)

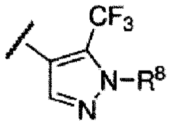

(3)

## Description

### Technical Field

The present invention relates to an azaindole compound and a medical drug.

### Background Art

Mucosa-associated lymphoid tissue lymphoma translocation protein 1 (MALT1) is a key signaling component of the NF-κB pathway. MALT1 is the only paracaspase in humans and transduces signals from the B-cell receptor and the T-cell receptor. MALT1 functions as a subunit of the activated CBM (CARD11/BCL10/MALT1) complex and activates the NF-κB pathway through two distinct mechanisms. The first is recruiting proteins such as TRAF6, TAK1, and IKKα/β as a scaffold protein, and the second is activating the NF-κB pathway through the cleavage of negative regulators of the NF-κB pathway, such as RelB, A20, and CYLD, as a cysteine protease (see, for example, Non Patent Literature 1).

Examples of diseases in which the NF-κB pathway is constitutively activated by mutations in the B-cell receptor pathway, such as CD79A/B, CARD11, MYD88, or A20, include activated B-cell-like diffuse large B-cell lymphoma (ABC-DLBCL) and primary central nervous system lymphoma (PCNSL) (see, for example, Non Patent Literature 2 and Non Patent Literature 3). BTK inhibitors such as ibrutinib have been reported to be clinically effective against ABC-DLBCL and PCNSL because these agents inhibit the B-cell receptor pathway (see, for example, Non Patent Literature 4 and Non Patent Literature 5). While ibrutinib is ineffective against ABC-DLBCL with mutations in downstream CARD11 (see, for example, Non Patent Literature 4), MALT1 protease inhibitors are effective even against ABC-DLBCL with CARD11 mutations because MALT1 is located downstream of BTK signaling (see, for example, Non Patent Literature 6). MALT1 protease inhibitors have demonstrated efficacy in multiple non-clinical ABC-DLBCL models (see, for example, Non Patent Literature 6).

MALT1 plays a key role in innate immunity and adaptive immunity. MALT1-deficient mice grow and reproduce normally, but exhibit impaired immune responses in lymphocytes (see, for example, Non Patent Literature 7). In humans with MALT1 deficiency mutations, the number of lymphocytes is normal, but impaired immune responses are observed (see, for example, Non Patent Literature 8). MALT1 protease inhibitors inhibit IL-2 production from T cells and suppress T cell proliferation (see, for example, Non Patent Literature 9). MALT1 protease inhibitors also reduce the onset and progression of multiple sclerosis (see, for example, Non Patent Literature 10).

On the other hand, mice with loss of MALT1 protease function develop autoimmune-like pathologies accompanied by a reduction in regulatory T cells (see, for example, Non Patent Literature 11). Furthermore, since MALT1 protease inhibitors enhance the tumor-suppressive activity of anti-PD-1 antibodies via alteration of regulatory T cells in malignant melanoma, MALT1 protease inhibitors are considered to possess tumor-immune activating effects (see, for example, Non Patent Literature 12).

Accordingly, MALT1 inhibitors are expected to exhibit efficacy against various inflammatory diseases, hematological tumors, and solid tumors. Specifically, examples of the inflammatory diseases include diseases, such as multiple sclerosis (see, for example, Non Patent Literature 10), rheumatoid arthritis, psoriasis (see, for example, Patent Literature 13), immune thrombocytopenia (see, for example, Non Patent Literature 14), and spinal cord injury (see, for example, Non Patent Literature 15). In addition, since such inhibitors suppress T-cell activation, similar to cyclosporine, efficacy is expected against graft-versus-host disease associated with bone marrow transplantation, rejection associated with organ transplantation, aplastic anemia, Behcet's disease, nephrotic syndrome, generalized myasthenia gravis, and atopic dermatitis. For hematological tumors, efficacy can be expected against diffuse large B-cell lymphoma, MALT lymphoma, mantle cell lymphoma, follicular lymphoma, marginal zone B-cell lymphoma, Burkitt's lymphoma, multiple myeloma, BENTA syndrome, adult T-cell leukemia/lymphoma, peripheral T-cell lymphoma, Sezary's syndrome, primary effusion lymphoma (see, for example, Non Patent Literature 16), chronic lymphocytic leukemia/small lymphocytic lymphoma (see, for example, Non Patent Literature 17), primary central nervous system malignant lymphoma (see, for example, Non Patent Literature 3), intraocular malignant lymphoma, primary macroglobulinemia, and lymphoplasmacytic lymphoma. For solid tumors, efficacy against brain tumors is expected (see, for example, Non Patent Literature 18). Furthermore, through tumor immune activation, efficacy is also expected against malignant melanoma (see, for example, Non Patent Literature 12), as well as non-small cell lung cancer, renal cell carcinoma, head and neck cancer, gastric cancer, malignant pleural mesothelioma, colon and rectal cancer, and esophageal cancer, for which anti-PD-1 antibodies exhibit efficacy.

### Citation List

### Non Patent Literatures

Non Patent Literature 1: Jaworski et al., Cellular and Molecular Life Sciences, 2016, 73, 459-473.
Non Patent Literature 2: Staudt et al., Cold Spring Harbor Perspectives in Biology, 2010, 2, 1-30.
Non Patent Literature 3: Chihara et al., Clinical Lymphoma, Myeloma & Leukemia, 2020,21,73-79.
Non Patent Literature 4: Wilson et al., Nature Medicine, 2015, 21, 922-927.
Non Patent Literature 5: Low et al., CNS Oncology, 2020, 9, CNS51.
Non Patent Literature 6: Fontan et al., The Journal of Clinical Investigation, 2018, 128, 4397-4412.
Non Patent Literature 7: Ruefli-Brasse et al., Science, 2003, 302, 1581-1584.
Non Patent Literature 8: Jabara et al., Journal of Allergy and Clinical Immunology, 2003, 132, 151-158.
Non Patent Literature 9: Bardet et al., Immunology & Cell Biology, 2018, 96, 81-99.
Non Patent Literature 10: Brustle et al., The Journal of Clinical Investigation, 2012, 122, 4698-4709.
Non Patent Literature 11: Jaworski et al., The EMBO Journal, 2014, 33, 2765-2781.
Non Patent Literature 12: Pilato et al., Nature, 2019, 570, 112-116.
Non Patent Literature 13: Demeyer et al., Trends in Molecular Medicine, 2016, 22, 135-150.
Non Patent Literature 14: Nakamura et al., International Immunopharmacology, 2018, 56, 193-196.
Non Patent Literature 15: Zhang et al., Science Bulletin, 2019, 64, 1179-1194.
Non Patent Literature 16: Juilland et al., Oncotarget, 2018, 9, 12542-12543.
Non Patent Literature 17: Saba et al., Cancer Research, 2017, 77, 7038-7048.
Non Patent Literature 18: Liu et al., Journal of Cellular and Molecular Medicine, 2020, 24, 7550-7562.

## Summary of Invention

### Technical Problem

An object of the present invention is to provide a compound having MALT1 inhibitory activity, or a pharmaceutically acceptable salt thereof.

### Solution to Problem

That is, the present invention includes the following inventions (Clause 1) to (Clause 11).

(Clause 1)

A compound represented by General Formula (1) or a pharmaceutically acceptable salt thereof (hereinafter, collectively referred to as the "compound of the present invention"):

[Chem. 1]

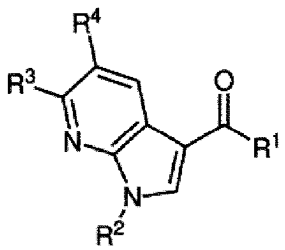

(1)

in General Formula (1), $R^1$ represents a group represented by General Formula (2) or General Formula (3);

[Chem. 2]

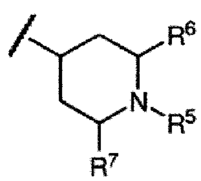

(2)

[Chem. 3]

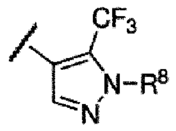

( 3 )

$R^2$ represents a hydrogen atom, a substituted or unsubstituted alkyl group, a substituted or unsubstituted cycloalkyl group, or a substituted or unsubstituted heterocyclic group;
$R^3$ represents a substituted or unsubstituted aryl group, a substituted or unsubstituted heteroaryl group, a substituted or unsubstituted alkoxy group, a substituted or unsubstituted aryloxy group, a substituted or unsubstituted heteroaryloxy group, a group represented by General Formula (4), or a group represented by General Formula (5);

[Chem. 4]

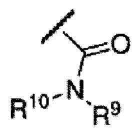

( 4 )

[Chem. 5]

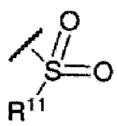

( 5 )

$R^4$ represents halogen;
$R^5$ represents a substituted or unsubstituted aryl group or a substituted or unsubstituted heteroaryl group;
$R^6$ and $R^7$ each independently represent a hydrogen atom or a substituted or unsubstituted alkyl group;
$R^8$ represents a substituted or unsubstituted aryl group, a substituted or unsubstituted heteroaryl group, or a substituted or unsubstituted heterocyclic group;
$R^9$ and $R^{10}$ each independently represent a substituted or unsubstituted alkyl group, a substituted or unsubstituted aryl group, or a substituted or unsubstituted heteroaryl group, and $R^9$ and $R^{10}$ may be linked to each other to form a ring;
$R^{11}$ represents a substituted or unsubstituted aryl group, a substituted or unsubstituted heteroaryl group, or a substituted or unsubstituted heterocyclic group; and
in General Formula (2), General Formula (3), General Formula (4), and General Formula (5), a wavy line represents a bond.

(Clause 2)

The compound or a pharmaceutically acceptable salt thereof according to (Clause 1), wherein the compound is a compound represented by General Formula (6),

[Chem. 6]

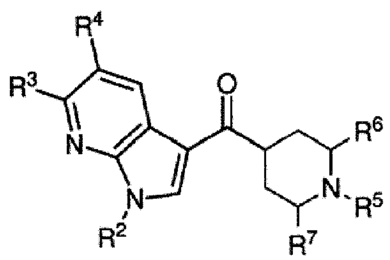

( 6 )

where $R^3$ represents a substituted or unsubstituted aryl group, a substituted or unsubstituted heteroaryl group, a group represented by General Formula (4), or a group represented by General Formula (5).

(Clause 3)

The compound or a pharmaceutically acceptable salt thereof according to (Clause 1), wherein the compound is a compound represented by General Formula (7),

[Chem. 7]

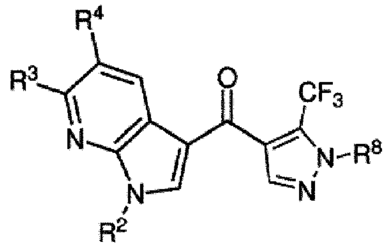

(7)

where $R^3$ represents a substituted or unsubstituted aryl group, a substituted or unsubstituted heteroaryl group, a substituted or unsubstituted alkoxy group, a substituted or unsubstituted aryloxy group, or a substituted or unsubstituted heteroaryloxy group.

(Clause 4)

A compound or a pharmaceutically acceptable salt thereof, wherein the compound is selected from the group consisting of the compounds (1) to (36) below:

(1) [5-chloro-1-(oxetan-3-yl)-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(2) [5-chloro-1-methyl-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(3) {5-chloro-3-[(2R,6R)-1-(5-fluoro-3-iodopyridin-2-yl)-2,6-dimethylpiperidine-4-carbonyl]-6-(2H-1,2,3-triazol-2-yl)-1H-pyrrolo[2,3-b]pyridin-1-yl}acetonitrile;
(4) [5-chloro-1-(2-hydroxyethyl)-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(5) 1-(3-{5-chloro-3-[(2R,6R)-1-(5-fluoro-3-iodopyridin-2-yl)-2,6-dimethylpiperidine-4-carbonyl]-6-(2H-1,2,3-triazol-2-yl)-1H-pyrrolo[2,3-b]pyridin-1-yl}azetidin-1-yl)ethan-1-one;
(6) [5-chloro-1-methyl-6-(2H-1,2,3-triazol-2-yl)-1H-pyrrolo[2,3-b]pyridin-3-yl][1-(8-fluoroisoquinolin-4-yl)-5-(trifluoromethyl)-1H-pyrazol-4-yl]methanone;
(7) [5-chloro-1-ethyl-6-(2H-1,2,3-triazol-2-yl)-1H-pyrrolo[2,3-b]pyridin-3-yl][1-(8-fluoroisoquinolin-4-yl)-5-(trifluoromethyl)-1H-pyrazol-4-yl]methanone;
(8) [5-chloro-1-propyl-6-(2H-1,2,3-triazol-2-yl)-1H-pyrrolo[2,3-b]pyridin-3-yl][1-(8-fluoroisoquinolin-4-yl)-5-(trifluoromethyl)-1H-pyrazol-4-yl]methanone;
(9) [5-chloro-1-(3-hydroxypropyl)-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(10) [5-chloro-1-(oxetan-3-yl)-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(11) [5-chloro-1-methyl-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(12) [5-chloro-1-methyl-6-(pyrimidin-2-yl)-1H-pyrrolo[2,3-b]pyridin-3-yl][1-(8-fluoroisoquinolin-4-yl)-5-(trifluoromethyl)-1H-pyrazol-4-yl]methanone;
(13) [5-chloro-1-ethyl-6-(pyrimidin-2-yl)-1H-pyrrolo[2,3-b]pyridin-3-yl][1-(8-fluoroisoquinolin-4-yl)-5-(trifluoromethyl)-1H-pyrazol-4-yl]methanone;
(14) [5-chloro-1-ethyl-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(15) [5-chloro-1-(3-hydroxypropyl)-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;

(16) [5-chloro-1-cyclopropyl-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(17) [5-chloro-1-isopropyl-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(18) [5-chloro-1-ethyl-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(19) [5-chloro-1-(1,1-dioxidotetrahydro-2H-thiopyran-4-yl)-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(20) [5-chloro-1-(oxan-4-yl)-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(21) {5-chloro-1-[(3S)-oxolan-3-yl]-6-(2H-1,2,3-triazol-2-yl)-1H-pyrrolo[2,3-b]pyridin-3-yl}[(2R,6R)-1-(5-fluoro-3-iodopyridin-2-yl)-2,6-dimethylpiperidin-4-yl]methanone;
(22) {5-chloro-1-[(3R)-oxolan-3-yl]-6-(2H-1,2,3-triazol-2-yl)-1H-pyrrolo[2,3-b]pyridin-3-yl}[(2R,6R)-1-(5-fluoro-3-iodopyridin-2-yl)-2,6-dimethylpiperidin-4-yl]methanone;
(23) [5-chloro-6-(2H-1,2,3-triazol-2-yl)-1H-pyrrolo[2,3-b]pyridin-3-yl][1-(5-fluoronaphthalen-1-yl)-5-(trifluoromethyl)-1H-pyrazol-4-yl]methanone;
(24) [5-chloro-1-methyl-6-(2H-1,2,3-triazol-2-yl)-1H-pyrrolo[2,3-b]pyridin-3-yl][1-(thieno[2,3-b]pyridin-4-yl)-5-(trifluoromethyl)-1H-pyrazol-4-yl]methanone;
(25) [5-chloro-1-(oxetan-3-yl)-6-(2H-1,2,3-triazol-2-yl)-1H-pyrrolo[2,3-b]pyridin-3-yl][1-(thieno[2,3-c]pyridin-4-yl)-5-(trifluoromethyl)-1H-pyrazol-4-yl]methanone;
(26) [5-chloro-1-propyl-6-(pyrimidin-2-yl)-1H-pyrrolo[2,3-b]pyridin-3-yl][1-(8-fluoroisoquinolin-4-yl)-5-(trifluoromethyl)-1H-pyrazol-4-yl]methanone;
(27) [5-chloro-1-methyl-6-(2H-1,2,3-triazol-2-yl)-1H-pyrrolo[2,3-b]pyridin-3-yl][1-(pyrazolo[1,5-a]pyridin-4-yl)-5-(trifluoromethyl)-1H-pyrazol-4-yl]methanone;
(28) [1-(1,2,3-benzothiadiazol-7-yl)-5-(trifluoromethyl)-1H-pyrazol-4-yl][5-chloro-1-methyl-6-(2H-1,2,3-triazol-2-yl)-1H-pyrrolo[2,3-b]pyridin-3-yl]methanone;
(29) [1-(1,2,3-benzothiadiazol-7-yl)-5-(trifluoromethyl)-1H-pyrazol-4-yl] [5-chloro-1-methyl-6-(pyrimidin-2-yl)-1H-pyrrolo[2,3-b]pyridin-3-yl]methanone;
(30) 5-{4-[5-chloro-1-cyclobutyl-6-(2H-1,2,3-triazol-2-yl)-1H-pyrrolo[2,3-b]pyridine-3-carbonyl]-5-(trifluoromethyl)-1H-pyrazol-1-yl}isoquinolin-1(2H)-one;
(31) 9-{4-[5-chloro-1-methyl-6-(2H-1,2,3-triazol-2-yl)-1H-pyrrolo[2,3-b]pyridine-3-carbonyl]-5-(trifluoromethyl)-1H-pyrazol-1-yl}-4H-quinolizin-4-one;
(32) (5-chloro-6-methoxy-1-methyl-1H-pyrrolo[2,3-b]pyridin-3-yl)[1-(8-fluoroisoquinolin-4-yl)-5-(trifluoromethyl)-1H-pyrazol-4-yl]methanone;
(33) [(2RS,4RS)-1-(5-bromopyrimidin-4-yl)-2-methylpiperidin-4-yl][5-chloro-1-methyl-6-(2H-1,2,3-triazol-2-yl)-1H-pyrrolo[2,3-b]pyridin-3-yl]methanone;
(34) [(2RS,4RS)-1-(2-amino-4-fluorophenyl)-2-methylpiperidin-4-yl][5-chloro-1-methyl-6-(2H-1,2,3-triazol-2-yl)-1H-pyrrolo[2,3-b]pyridin-3-yl]methanone;
(35) {5-chloro-3-[(2RS,4RS)-1-(3-chloro-5-fluoropyridin-2-yl)-2-methylpiperidine-4-carbonyl]-1H-pyrrolo[2,3-b]pyridin-6-yl}(piperazin-1-yl)methanone; and
(36) [(2RS,4RS)-1-(3-chloro-5-fluoropyridin-2-yl)-2-methylpiperidin-4-yl][5-chloro-1-methyl-6-(piperazin-1-sulfonyl)-1H-pyrrolo[2,3-b]pyridin-3-yl]methanone.

(Clause 5)

A pharmaceutical composition comprising, as an active ingredient, the compound or a pharmaceutically acceptable salt thereof according to any one of (Clause 1) to (Clause 4).

(Clause 6)

A MALT1 inhibitor comprising, as an active ingredient, the compound or a pharmaceutically acceptable salt thereof according to any one of (Clause 1) to (Clause 4).

(Clause 7)

A method for inhibiting MALT1, comprising administering the compound or a pharmaceutically acceptable salt thereof according to any one of (Clause 1) to (Clause 4) to a subject in need thereof.

(Clause 8)

The compound or a pharmaceutically acceptable salt thereof according to any one of (Clause 1) to (Clause 4), for use in inhibiting MALT1.

(Clause 9)

A use of the compound or a pharmaceutically acceptable salt thereof according to any one of (Clause 1) to (Clause 4) in the manufacture of a MALT1 inhibitor.

(Clause 10)

A prophylactic or therapeutic agent for a disease involving MALT1, the agent comprising, as an active ingredient, the compound or a pharmaceutically acceptable salt thereof according to any one of (Clause 1) to (Clause 4).

(Clause 11)

A method for preventing or treating a disease involving MALT1, comprising administering the compound or a pharmaceutically acceptable salt thereof according to any one of (Clause 1) to (Clause 4) to a subject in need thereof.

(Clause 12)

The compound of the present invention or a pharmaceutically acceptable salt thereof, for use in preventing or treating a disease involving MALT1.

(Clause 13)

A use of the compound of the present invention or a pharmaceutically acceptable salt thereof in the manufacture of a prophylactic or therapeutic agent for a disease involving MALT1.

(Clause 14)

A therapeutic agent for multiple sclerosis, rheumatoid arthritis, psoriasis, immune thrombocytopenia, spinal cord injury, graft-versus-host disease associated with bone marrow transplantation, rejection associated with organ transplantation, aplastic anemia, Behcet's disease, nephrotic syndrome, generalized myasthenia gravis, atopic dermatitis, diffuse large B-cell lymphoma, MALT lymphoma, mantle cell lymphoma, follicular lymphoma, marginal zone B-cell lymphoma, Burkitt lymphoma, multiple myeloma, BENTA syndrome, adult T-cell leukemia/lymphoma, peripheral T-cell lymphoma, Sezary's syndrome, primary effusion lymphoma, chronic lymphocytic leukemia/small lymphocytic lymphoma, primary central nervous system malignant lymphoma, intraocular lymphoma, primary macroglobulinemia, lymphoplasmacytic lymphoma, brain tumor, malignant melanoma, non-small cell lung cancer, renal cell carcinoma, head and neck cancer, gastric cancer, malignant pleural mesothelioma, colon and rectal cancer, or esophageal cancer,
the agent comprising, as an active ingredient, the compound or a pharmaceutically acceptable salt thereof according to any one of (Clause 1) to (Clause 4).

## Description of Embodiments

Hereinafter, embodiments for carrying out the present invention will be described in detail. However, the present invention is not limited to the following embodiments.

As used herein, the term "alkyl group" refers to a monovalent group obtained by removing one hydrogen atom from a saturated hydrocarbon. The alkyl group may be linear or branched. The alkyl group may be a $C_1$-$C_6$ alkyl group, may be a $C_1$-$C_4$ alkyl group, or may be a $C_1$-$C_3$ alkyl group. Note that, as used herein, "$C_a$-$C_b$" means that the number of carbon atoms is from a to b. Specific examples of the alkyl group include a methyl group, an ethyl group, an n-propyl group, an isopropyl group (1-methylethyl group), an n-butyl group, a sec-butyl group, an isobutyl group (2-methylpropyl group), a tert-butyl group, an n-pentyl group, a neopentyl group, an isopentyl group, a sec-pentyl group, a 3-pentyl group, a tert-pentyl group, an n-hexyl group, a 1,1,2-trimethylpropyl group, a 1,2,2-trimethylpropyl group, a 1,1,2,2-tetramethylpropyl group, a 1,1-dimethylbutyl group, a 1,2-dimethylbutyl group, a 1,3-dimethylbutyl group, a 2,2-dimethylbutyl group, a 2,3-dimethylbutyl group, a 3,3-dimethylbutyl group, a 1-ethylbutyl group, and a 2-ethylbutyl group.

As used herein, the term "substituted alkyl group" refers to a group in which one or more hydrogen atoms of an

alkyl group are substituted with a substituent. Examples of the substituent in the substituted alkyl group include a hydroxy group, a cyano group, halogen, an amino group, a mono-substituted amino group, a di-substituted amino group, a nitro group, an alkyl group, an alkoxy group, an aryloxy group, a heteroaryloxy group, a saturated heterocyclic group, an aryl group, a heteroaryl group, an acyl group, and an oxo group. These substituents may be further substituted with other substituents, and may be taken together to form a ring.

As used herein, the term "cycloalkyl group" refers to a monovalent group obtained by removing one hydrogen atom from a cyclic saturated hydrocarbon. The cycloalkyl group may be a $C_3$-$C_8$ cycloalkyl group, a $C_3$-$C_6$ cycloalkyl group, or a $C_3$-$C_4$ cycloalkyl group. Specific examples of the cycloalkyl group include a cyclopropyl group, a cyclobutyl group, a cyclopentyl group, and a cyclohexyl group.

As used herein, the term "substituted cycloalkyl group" refers to a group in which one or more hydrogen atoms of a cycloalkyl group are substituted with a substituent. Examples of the substituent in the substituted cycloalkyl group include the substituents as described above.

As used herein, the term "heterocyclic group" refers to a nonaromatic cyclic monovalent group containing a heteroatom in the ring. The number of heteroatoms contained in the ring of the heterocyclic group may be, for example, 1 to 5, 1 to 3, 1 to 2, or 1. The heterocyclic group may be monocyclic or may be a fused ring. The heterocyclic group may have an unsaturated bond in the ring (unsaturated heterocyclic group) or may not have an unsaturated bond (saturated heterocyclic group). Specific examples of the heterocyclic group include an oxetanyl group, a tetrahydrofuranyl group, a tetrahydropyranyl group, an azetidinyl group, a tetrahydro-1,1-dioxide-2H-thiopyranyl group, and a quinolizinone group.

As used herein, the term "substituted heterocyclic group" refers to a group in which one or more elements (elements not constituting the ring) of a heterocyclic group are substituted with a substituent. As the substituent in the substituted heterocyclic group, the substituents described above can be exemplified.

As used herein, the term "aryl group" refers to a monovalent group of a cyclic aromatic hydrocarbon. The aryl group may be a single ring or a fused ring. Specific examples of the aryl group include a phenyl group and a naphthyl group.

As used herein, the term "substituted aryl group" refers to a group in which one or more elements (elements not constituting the ring) of an aryl group are substituted with a substituent. Examples of substituents in the substituted aryl group include the substituents described above.

As used herein, the term "heteroaryl group" refers to a monovalent cyclic aromatic group containing a heteroatom in the ring. The number of heteroatoms contained in the ring of the heteroaryl group may be, for example, 1 to 5, 1 to 3, 1 to 2, or 1. The heteroaryl group may be a single ring or a fused ring. Specific examples of heteroaryl groups include pyrrolyl, imidazolyl, pyrazolyl, triazolyl, pyridyl, pyrimidinyl, isoquinolyl, quinolizinyl, 1,2,3-benzothiadiazolyl, thieno[2,3-b]pyridyl, thieno[2,3-c]pyridyl, 1(2H)-oxoisoquinolyl, pyrazolo[1,5-a]pyridyl, and 2,7-naphthyridinyl.

As used herein, the term "substituted heteroaryl group" refers to a group in which one or more elements (elements not constituting the ring) of a heteroaryl group are substituted with a substituent. Examples of substituents for the substituted heteroaryl group include the substituents described above.

As used herein, the term "alkoxy group" refers to an oxy group bonded to an alkyl group. Specific examples of alkoxy groups include methoxy, ethoxy, 1-propoxy, 2-propoxy, n-butoxy, i-butoxy, s-butoxy, t-butoxy, pentyloxy, and 3-methylbutoxy.

As used herein, the term "substituted alkoxy group" refers to a group in which one or more hydrogen atoms of an alkoxy group are substituted with a substituent. Examples of substituents for the substituted alkoxy group include the substituents described above.

As used herein, the term "aryloxy group" refers to an oxy group bonded to an aryl group. Specific examples of aryloxy groups include phenoxy and naphthyloxy.

As used herein, the term "substituted aryloxy group" refers to a group in which one or more elements (elements not constituting the ring) of an aryloxy group are substituted with a substituent. Examples of substituents for the substituted aryloxy group include the substituents described above.

As used herein, the term "heteroaryloxy group" refers to an oxy group bonded to a heteroaryl group. Specific examples of heteroaryloxy groups include furyloxy, thienyloxy, pyrrolyloxy, imidazolyloxy, pyrazolyloxy, thiazolyloxy, and piperazinyloxy.

As used herein, the term "substituted heteroaryloxy group" refers to a group in which one or more elements (elements not constituting the ring) of a heteroaryloxy group are substituted with a substituent. Examples of substituents for the substituted heteroaryloxy group include the substituents described above.

As used herein, the term "halogen" refers to a group consisting of a halogen atom (halogeno group). Examples of the halogen include a fluoro group, a chloro group, a bromo group, and an iodo group.

The compound of the present invention is a compound represented by General Formula (1), or a pharmaceutically acceptable salt thereof.

[Chem. 8]

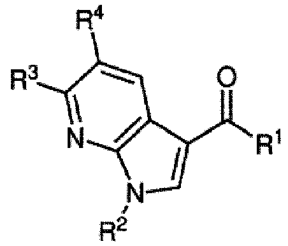

(1)

In General Formula (1), $R^1$ represents a group represented by General Formula (2) or General Formula (3).

[Chem. 9]

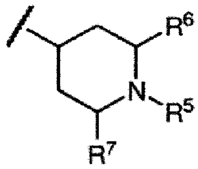

(2)

[Chem. 10]

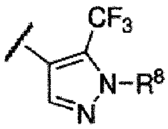

(3)

In General Formula (1), $R^2$ represents a hydrogen atom, a substituted or unsubstituted alkyl group, a substituted or unsubstituted cycloalkyl group, or a substituted or unsubstituted heterocyclic group; preferably, a hydrogen atom, a substituted or unsubstituted $C_1$-$C_6$ alkyl group, a substituted or unsubstituted $C_3$-$C_8$ cycloalkyl group, or a substituted or unsubstituted monocyclic saturated heterocyclic group; more preferably, a hydrogen atom, a $C_1$-$C_6$ alkyl group, a $C_1$-$C_6$ alkyl group substituted with a hydroxy group or a cyano group, a $C_3$-$C_6$ cycloalkyl group, a monocyclic saturated heterocyclic group substituted with an acetyl or oxo group, or an unsubstituted monocyclic saturated heterocyclic group; even more preferably, a hydrogen atom, a methyl group, an ethyl group, an n-propyl group, a 1-methylethyl group, a 2-hydroxyethyl group, a 3-hydroxypropyl group, a cyanomethyl group, an oxetanyl group (for example, 3-oxetanyl group), a tetrahydrofuranyl group (for example, 3-tetrahydrofuranyl group), a tetrahydropyranyl group (for example, 4-tetrahydropyranyl group), a cyclopropyl group, a cyclobutyl group, a 1-acetylazetidinyl group (for example, 3-(1-acetylazetidinyl) group), or a tetrahydro-1,1-dioxide-2H-thiopyranyl group (for example, 4-(tetrahydro-1,1-dioxide-2H-thiopyranyl) group); still more preferably, a methyl group, an ethyl group, or an n-propyl group; and particularly preferably, a methyl group.

In General Formula (1), $R^3$ represents a substituted or unsubstituted aryl group, a substituted or unsubstituted heteroaryl group, a substituted or unsubstituted alkoxy group, a substituted or unsubstituted aryloxy group, a substituted or unsubstituted heteroaryloxy group, a group represented by General Formula (4), or a group represented by General Formula (5).

[Chem. 11]

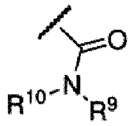

(4)

In General Formula (4), $R^9$ and $R^{10}$ each independently represent a substituted or unsubstituted alkyl group, a substituted or unsubstituted aryl group, or a substituted or unsubstituted heteroaryl group, and $R^9$ and $R^{10}$ may be linked to each other to form a ring.

[Chem. 12]

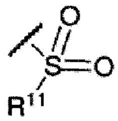

( 5 )

In General Formula (5), $R^{11}$ represents a substituted or unsubstituted aryl group, a substituted or unsubstituted heteroaryl group, or a substituted or unsubstituted heterocyclic group.

$R^3$ is preferably an aryl group, a heteroaryl group, an alkoxy group, an aryloxy group, a heteroaryloxy group, a group represented by general formula (4), or a group represented by general formula (5); more preferably, a heteroaryl group, an alkoxy group, a group represented by general formula (4), or a group represented by general formula (5); even more preferably, a 1,2,3-triazolyl group (for example, a 2-1,2,3-triazolyl group), a pyrimidinyl group (for example, a 2-pyrimidinyl group), a methoxy group, a group represented by general formula (4), or a group represented by general formula (5); and still more preferably, a 1,2,3-triazolyl group or a pyrimidinyl group.

In General Formula (4), $R^9$ and $R^{10}$ each independently represent a substituted or unsubstituted alkyl group, a substituted or unsubstituted aryl group, or a substituted or unsubstituted heteroaryl group. $R^9$ and $R^{10}$ may be linked to each other to form a ring. It is preferable that $R^9$ and $R^{10}$ are linked to each other to form a ring, and it is more preferable that $R^9$ and $R^{10}$ form a piperazine ring together with the nitrogen atom to which $R^9$ and $R^{10}$ are bonded.

In General Formula (5), $R^{11}$ represents a substituted or unsubstituted aryl group, or a substituted or unsubstituted heteroaryl group, or a substituted or unsubstituted heterocyclic group, and is preferably a saturated heterocyclic group, and more preferably a piperazinyl group (for example, 1-piperazinyl group).

In General Formula (1), $R^4$ represents halogen, and is preferably a chloro group.

In General Formula (2), $R^5$ represents a substituted or unsubstituted aryl group, or a substituted or unsubstituted heteroaryl group, preferably a substituted aryl group or a substituted heteroaryl group, preferably an aryl group substituted with a bromo, chloro, fluoro, amino, or iodo group, or a heteroaryl group substituted with a bromo, chloro, fluoro, amino, or iodo group, still more preferably a phenyl group substituted with a bromo, chloro, fluoro, amino, or iodo group, or a pyridinyl group substituted with a bromo, chloro, fluoro, or iodo group, and even still more preferably a 1-amino-3-fluorophenyl group (for example, 6-1-amino-3-fluorophenyl), a 5-bromopyrimidinyl group (for example, 6-5-bromopyrimidinyl), or a 3-iodo-5-fluoropyridinyl group (for example, 2-3-iodo-5-fluoropyridinyl), and particularly preferably a 3-iodo-5-fluoropyridinyl group.

In General Formula (2), $R^6$ and $R^7$ each independently represent a hydrogen atom or a substituted or unsubstituted alkyl group, preferably a substituted or unsubstituted alkyl group, more preferably a $C_1$-$C_6$ alkyl group, still more preferably a $C_1$-$C_3$ alkyl group, and even more preferably a methyl group. $R^6$ and $R^7$ are each particularly preferably a methyl group. When $R^6$ is not a hydrogen atom, the carbon atom to which $R^6$ is bonded is an asymmetric center. In this case, the steric configuration may be R or S, but is preferably R. Similarly, when $R^7$ is not a hydrogen atom, the carbon atom to which $R^7$ is bonded is an asymmetric center. In this case, the steric configuration may be R or S, but is preferably R. When both the carbon atom to which $R^6$ is bonded and the carbon atom to which $R^7$ is bonded are asymmetric centers, it is particularly preferred that both have the R steric configuration.

In General Formula (3), $R^8$ represents a substituted or unsubstituted aryl group, a substituted or unsubstituted heteroaryl group, or a substituted or unsubstituted heterocyclic group; preferably, a substituted or unsubstituted fused aryl group, a substituted or unsubstituted fused heteroaryl group, or a substituted or unsubstituted fused heterocyclic group; more preferably, a fused aryl group substituted with a fluoro group, a fused heteroaryl group substituted with a fluoro group, an unsubstituted fused heteroaryl group, a fused heterocyclic group substituted with an oxo group, or an unsubstituted fused heterocyclic group; even more preferably, an 8-fluoroisoquinolyl group (for example, 4-8-fluoroisoquinolyl), a 4-quinolizinonyl group (for example, 8-4-quinolizinonyl), a 1-fluoronaphthyl group (for example, 5-1-fluoronaphthyl), a 1,2,3-benzothiadiazolyl group (for example, 7-1,2,3-benzothiadiazolyl), a pyrazolo[1,5-a]pyridyl group (for example, 4-pyrazolo [1,5-a]pyridyl), a thieno[2,3-b]pyridyl group (for example, 4-thieno[2,3-b]pyridyl), a thieno[2,3-c]pyridyl group (for example, 3-thieno[2,3-c]pyridyl), or a 1(2H)-oxoisoquinolyl group (for example, 5-1(2H)-oxoisoquinolyl); and still more preferably, an 8-fluoroisoquinolyl group.

In General Formulae (1), (2), (3), (4), and (5), a wavy line represents a bond.

The compound according to the present embodiment may be a compound represented by General Formula (6).

[Chem. 13]

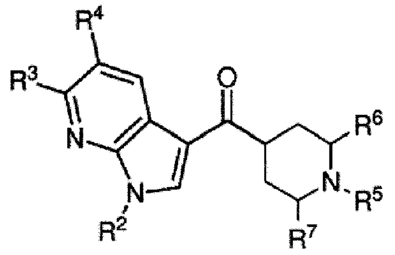

( 6 )

In General Formula (6), $R^2$, $R^3$, $R^4$, $R^5$, $R^6$, and $R^7$ are as defined in General Formula (1).

The compound according to the present embodiment may be a compound represented by General Formula (7).

[Chem. 14]

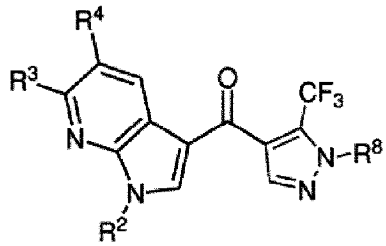

( 7 )

In General Formula (7), $R^2$, $R^3$, $R^4$ and $R^8$ are as defined in General Formula (1).

Specific examples of the compound represented by General Formula (1) include the following Compounds (1) to (36).

(1) [5-chloro-1-(oxetan-3-yl)-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(2) [5-chloro-1-methyl-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(3) {5-chloro-3-[(2R,6R)-1-(5-fluoro-3-iodopyridin-2-yl)-2,6-dimethylpiperidine-4-carbonyl]-6-(2H-1,2,3-triazol-2-yl)-1H-pyrrolo[2,3-b]pyridin-1-yl}acetonitrile;
(4) [5-chloro-1-(2-hydroxyethyl)-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(5) 1-(3-{5-chloro-3-[(2R,6R)-1-(5-fluoro-3-iodopyridin-2-yl)-2,6-dimethylpiperidine-4-carbonyl]-6-(2H-1,2,3-triazol-2-yl)-1H-pyrrolo[2,3-b]pyridin-1-yl}azetidin-1-yl)ethan-1-one;
(6) [5-chloro-1-methyl-6-(2H-1,2,3-triazol-2-yl)-1H-pyrrolo[2,3-b]pyridin-3-yl][1-(8-fluoroisoquinolin-4-yl)-5-(trifluoromethyl)-1H-pyrazol-4-yl]methanone;
(7) [5-chloro-1-ethyl-6-(2H-1,2,3-triazol-2-yl)-1H-pyrrolo[2,3-b]pyridin-3-yl][1-(8-fluoroisoquinolin-4-yl)-5-(trifluoromethyl)-1H-pyrazol-4-yl]methanone;
(8) [5-chloro-1-propyl-6-(2H-1,2,3-triazol-2-yl)-1H-pyrrolo[2,3-b]pyridin-3-yl][1-(8-fluoroisoquinolin-4-yl)-5-(trifluoromethyl)-1H-pyrazol-4-yl]methanone;
(9) [5-chloro-1-(3-hydroxypropyl)-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(10) [5-chloro-1-(oxetan-3-yl)-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(11) [5-chloro-1-methyl-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(12) [5-chloro-1-methyl-6-(pyrimidin-2-yl)-1H-pyrrolo[2,3-b]pyridin-3-yl][1-(8-fluoroisoquinolin-4-yl)-5-(trifluoromethyl)-1H-pyrazol-4-yl]methanone;
(13) [5-chloro-1-ethyl-6-(pyrimidin-2-yl)-1H-pyrrolo[2,3-b]pyridin-3-yl][1-(8-fluoroisoquinolin-4-yl)-5-(trifluoromethyl)-1H-pyrazol-4-yl]methanone;
(14) [5-chloro-1-ethyl-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;

(15) [5-chloro-1-(3-hydroxypropyl)-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;

(16) [5-chloro-1-cyclopropyl-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;

(17) [5-chloro-1-isopropyl-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;

(18) [5-chloro-1-ethyl-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone;

(19) [5-chloro-1-(1,1-dioxidotetrahydro-2H-thiopyran-4-yl)-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;

(20) [5-chloro-1-(oxan-4-yl)-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;

(21) {5-chloro-1-[(3S)-oxolan-3-yl]-6-(2H-1,2,3-triazol-2-yl)-1H-pyrrolo[2,3-b]pyridin-3-yl}[(2R,6R)-1-(5-fluoro-3-iodopyridin-2-yl)-2,6-dimethylpiperidin-4-yl]methanone;

(22) {5-chloro-1-[(3R)-oxolan-3-yl]-6-(2H-1,2,3-triazol-2-yl)-1H-pyrrolo[2,3-b]pyridin-3-yl}[(2R,6R)-1-(5-fluoro-3-iodopyridin-2-yl)-2,6-dimethylpiperidin-4-yl]methanone;

(23) [5-chloro-6-(2H-1,2,3-triazol-2-yl)-1H-pyrrolo[2,3-b]pyridin-3-yl][1-(5-fluoronaphthalen-1-yl)-5-(trifluoromethyl)-1H-pyrazol-4-yl]methanone;

(24) [5-chloro-1-methyl-6-(2H-1,2,3-triazol-2-yl)-1H-pyrrolo[2,3-b]pyridin-3-yl][1-(thieno[2,3-b]pyridin-4-yl)-5-(trifluoromethyl)-1H-pyrazol-4-yl]methanone;

(25) [5-chloro-1-(oxetan-3-yl)-6-(2H-1,2,3-triazol-2-yl)-1H-pyrrolo[2,3-b]pyridin-3-yl][1-(thieno[2,3-c]pyridin-4-yl)-5-(trifluoromethyl)-1H-pyrazol-4-yl]methanone;

(26) [5-chloro-1-propyl-6-(pyrimidin-2-yl)-1H-pyrrolo[2,3-b]pyridin-3-yl][1-(8-fluoroisoquinolin-4-yl)-5-(trifluoromethyl)-1H-pyrazol-4-yl]methanone;

(27) [5-chloro-1-methyl-6-(2H-1,2,3-triazol-2-yl)-1H-pyrrolo[2,3-b]pyridin-3-yl][1-(pyrazolo[1,5-a]pyridin-4-yl)-5-(trifluoromethyl)-1H-pyrazol-4-yl]methanone;

(28) [1-(1,2,3-benzothiadiazol-7-yl)-5-(trifluoromethyl)-1H-pyrazol-4-yl][5-chloro-1-methyl-6-(2H-1,2,3-triazol-2-yl)-1H-pyrrolo[2,3-b]pyridin-3-yl]methanone;

(29) [1-(1,2,3-benzothiadiazol-7-yl)-5-(trifluoromethyl)-1H-pyrazol-4-yl][5-chloro-1-methyl-6-(pyrimidin-2-yl)-1H-pyrrolo[2,3-b]pyridin-3-yl]methanone;

(30) 5-{4-[5-chloro-1-cyclobutyl-6-(2H-l,2,3-triazol-2-yl)-1H-pyrrolo[2,3-b]pyridine-3-carbonyl]-5-(trifluoromethyl)-1H-pyrazol-1-yl}isoquinolin-1(2H)-one;

(31) 9-{4-[5-chloro-1-methyl-6-(2H-1,2,3-triazol-2-yl)-1H-pyrrolo[2,3-b]pyridine-3-carbonyl]-5-(trifluoromethyl)-1H-pyrazol-1-yl}-4H-quinolizin-4-one;

(32) (5-chloro-6-methoxy-1-methyl-1H-pyrrolo[2,3-b]pyridin-3-yl)[1-(8-fluoroisoquinolin-4-yl)-5-(trifluoromethyl)-1H-pyrazol-4-yl]methanone;

(33) [(2RS,4RS)-1-(5-bromopyrimidin-4-yl)-2-methylpiperidin-4-yl][5-chloro-1-methyl-6-(2H-1,2,3-triazol-2-yl)-1H-pyrrolo[2,3-b]pyridin-3-yl]methanone;

(34) [(2RS,4RS)-1-(2-amino-4-fluorophenyl)-2-methylpiperidin-4-yl][5-chloro-1-methyl-6-(2H-1,2,3-triazol-2-yl)-1H-pyrrolo[2,3-b]pyridin-3-yl]methanone;

(35) {5-chloro-3-[(2RS,4RS)-1-(3-chloro-5-fluoropyridin-2-yl)-2-methylpiperidine-4-carbonyl]-1H-pyrrolo[2,3-b]pyridin-6-yl}(piperazin-1-yl)methanone; and

(36) [(2RS,4RS)-1-(3-chloro-5-fluoropyridin-2-yl)-2-methylpiperidin-4-yl][5-chloro-1-methyl-6-(piperazin-1-sulfonyl)-1H-pyrrolo[2,3-b]pyridin-3-yl]methanone.

The compound of the present invention can be produced using known compounds or intermediates that can be readily synthesized from known compounds, for example, according to the methods described below, the examples described later, or known methods. In the production of the compound of the present invention, in a case where a starting material has a substituent that affects the reaction, it is common to carry out the reaction after protecting the starting material in advance with an appropriate protecting group by a known method. The protecting group can be removed after the reaction by a known method.

The meanings of the terms used in the present specification are described below. Unless otherwise specified, each term is used with the same meaning whether used alone or in combination with other terms.

Abbreviations used in the present specification represent the following meanings.

In Examples, the following abbreviations are used.

Pd-C: Palladium-carbon

$Pd(PPh_3)_4$: Tetrakistriphenylphosphine palladium
$PdCl_2(PPh_3)_2$: Bis(triphenylphosphine)palladium(II) dichloride
$Pd(OAc)_2$: Palladium (II) acetate
$Pd(dppf)_2Cl_2$: [1,1'-Bis(diphenylphosphino)ferrocene]dichloropalladium(II) dichloromethane adduct
$PPh_3$: Triphenylphosphine
$Boc_2O$: Di-tert-butyl dicarbonate
HATU: O-(7-Azabenzotriazol-1-yl)-N,N,N',N'-tetramethyluronium hexafluorophosphate
HBTU: O-(Benzotriazol-1-yl)-N,N,N',N'-tetramethyluronium hexafluorophosphate
EDCI: 1-Ethyl-3-(3-dimethylaminopropyl)carbodiimide
HOBt: 1-Hydroxybenzotriazole
THF: Tetrahydrofuran
DMF: Dimethylformamide
DMSO: Dimethyl sulfoxide
DIPA: Diisopropylamine
DIPEA: N,N-diisopropylethylamine
TEA: Triethylamine
Boc: tert-Butoxycarbonyl
Cbz: Benzyloxycarbonyl
Bn: Benzyl
MS: Mass spectrometry
LCMS: High-performance liquid chromatography-mass spectrometry
ESI: Electron Spray Ionization
M: Molar concentration (mol/L)

Examples of pharmaceutically acceptable salts of the compounds of the present invention include mineral acid salts such as hydrochloride, hydrobromide, sulfate, and phosphate; organic acid salts such as acetate, malate, lactate, citrate, tartrate, maleate, succinate, fumarate, p-toluenesulfonate, benzenesulfonate, and methanesulfonate; alkali metal salts such as lithium, potassium, and sodium salts; alkaline earth metal salts such as magnesium and calcium salts; and organic base salts such as ammonium salts. These salts can be formed by a method commonly used in the art.

For example, when the compound of the present invention is a hydrochloride salt, it can be obtained by dissolving the free base of the compound in a solution of hydrogen chloride in alcohol, ethyl acetate, 1,4-dioxane, cyclopentyl methyl ether, or diethyl ether.

The compound of the present invention may form a solvate by incorporating solvent molecules when left in the air or upon recrystallization, and such solvates are also included within the scope of the compound of the present invention. Examples of such solvates include solvates with solvents such as methanol, ethanol, isopropyl alcohol, butanol, dimethyl sulfoxide, and acetonitrile, as well as monohydrates and dihydrates.

Some of the compounds of the present invention have asymmetric carbon atoms, and all optical isomers and mixtures thereof are included in the present invention. Stereoisomers may be separated, for example, from racemates by optical resolution using known methods that exploit their basicity and employ optically active acids such as tartaric acid, dibenzoyltartaric acid, mandelic acid, or 10-camphorsulfonic acid, or may be produced using optically active compounds prepared in advance as starting materials. In addition, they may also be produced by optical resolution using a chiral column or by asymmetric synthesis. Furthermore, in the compounds of the present invention, although the structural formula of a compound may, for convenience, represent a specific isomer, the present invention encompasses isomers such as geometric isomers, optical isomers based on asymmetric carbon atoms, stereoisomers, and tautomers, as well as isomer mixtures, which arise due to the structure of the compound. In addition, unless otherwise specifically defined in a particular claim, the description of formulas for convenience does not limit the invention, and all variations are included within the scope of the present invention.

The compound of the present invention has a MALT1 inhibitory activity as illustrated in Test Examples described later.

Therefore, in one embodiment of the present invention, there is provided a MALT1 inhibitor containing the compound of the present invention.

Furthermore, in one embodiment of the present invention, there is provided a method for inhibiting MALT1 including administering the compound of the present invention to a subject in need thereof.

Furthermore, in one embodiment of the present invention, there is provided the compound of the present invention for use in inhibiting MALT1.

Furthermore, in one embodiment of the present invention, there is provided a use of the compound of the present invention in the manufacture of a MALT1 inhibitor.

In one embodiment of the present invention, there is provided a prophylactic or therapeutic agent for a disease

involving MALT1, which contains the compound of the present invention.

Furthermore, in one embodiment of the present invention, there is provided a method for preventing or treating a disease involving MALT1, the method including administering the compound of the present invention to a subject in need thereof.

Furthermore, in one embodiment of the present invention, there is provided the compound of the present invention for use in preventing or treating a disease involving MALT1.

Furthermore, in one embodiment of the present invention, there is provided a use of the compound of the present invention in the manufacture of a prophylactic or therapeutic agent for a disease involving MALT1.

Examples of the diseases to which the compound of the present invention can be applied include multiple sclerosis, rheumatoid arthritis, psoriasis, immune thrombocytopenia, spinal cord injury, graft-versus-host disease associated with bone marrow transplantation, rejection associated with organ transplantation, aplastic anemia, Behcet's disease, nephrotic syndrome, generalized myasthenia gravis, atopic dermatitis, diffuse large B-cell lymphoma, MALT lymphoma, mantle cell lymphoma, follicular lymphoma, marginal zone B-cell lymphoma, Burkitt lymphoma, multiple myeloma, BENTA syndrome, adult T-cell leukemia/lymphoma, peripheral T-cell lymphoma, Sezary's syndrome, primary effusion lymphoma, chronic lymphocytic leukemia/small lymphocytic lymphoma, primary central nervous system malignant lymphoma, intraocular lymphoma, primary macroglobulinemia, lymphoplasmacytic lymphoma, brain tumor, malignant melanoma, non-small cell lung cancer, renal cell carcinoma, head and neck cancer, gastric cancer, malignant pleural mesothelioma, colon and rectal cancer, or esophageal cancer.

The term "subject" refers to a human or non-human animal having or suspected of having a disease involving MALT1. In one embodiment of the present invention, the subject is a mammal. In one embodiment of the present invention, the subject is a human.

The compound of the present invention may be used as a therapeutic agent for the aforementioned various diseases in mammals such as humans, mice, rats, rabbits, dogs, cats, cattle, horses, pigs, and monkeys, either as is or in the form of a pharmaceutical composition containing the compound in an amount of, for example, 0.001% to 99.5%, preferably 0.1% to 90%, mixed with a pharmacologically acceptable carrier or the like.

As the carrier, one or more conventionally used solid, semi-solid, or liquid diluents, fillers, and other excipients that are pharmaceutically acceptable may be used. It is desirable that the pharmaceutical composition according to the present invention be administered in a unit dosage form. The pharmaceutical composition may be administered by intratissue administration, oral administration, intravenous administration, topical administration (such as transdermal, ocular, intraperitoneal, or intrathoracic administration), or rectal administration. The pharmaceutical composition according to the present invention is administered in a dosage form suitable for these routes of administration.

The dosage for use as a pharmaceutical is preferably adjusted in consideration of the patient's condition, including age, body weight, type and severity of disease, route of administration, type of compound of the present invention, whether it is in the form of a salt or not, and the type of salt. However, for oral administration in adults, the usual effective amount of the compound of the present invention or a pharmaceutically acceptable salt thereof is in the range of 0.01 mg to 5 g per adult per day, preferably in the range of 1 mg to 500 mg per adult per day. In some cases, a lower dose may be sufficient, while in other cases, a higher dose may be required. Typically, administration may be carried out once daily or in divided doses, or in the case of intravenous administration, the compound may be administered either by rapid injection or by continuous infusion within 24 hours.

One or more hydrogen, carbon, and/or other atoms of the compound of the present invention may be substituted with isotopes of hydrogen, carbon, and/or other atoms, respectively. Examples of such isotopes include $^{2}H$, $^{3}H$, $^{11}C$, $^{13}C$, $^{14}C$, $^{15}N$, $^{18}O$, $^{17}O$, $^{31}P$, $^{32}P$, $^{35}S$, $^{18}F$, $^{123}I$ and $^{36}Cl$, that is, isotopes of hydrogen, carbon, nitrogen, oxygen, phosphorus, sulfur, fluorine, iodine, and chlorine. Compounds substituted with such isotopes are also useful as pharmaceuticals, and all radiolabeled forms of the compounds of the present invention are encompassed.

The compounds of the present invention can be produced from compounds that are themselves known or from intermediates that can be easily prepared from known compounds, for example, according to the following methods, the examples described below, or known methods.

In each step of the following production methods, commercially available solvents, reagents, and starting materials may be used in the original form thereof when available on the market. In addition, the compounds obtained in each step of the production methods described below, as well as the starting materials used, may form salts, and can be converted into other types of salts or into the free form by known methods. Conversely, when the compounds obtained in each step of the following production methods or the starting materials used are in the free form, they can be converted into the desired salt by known methods. Examples of such salts include the same types of salts as those used for the compounds of the present invention described above.

In the production of the compound of the present invention, when the starting material has a substituent that may affect the reaction, a protecting group may be introduced into such a substituent in advance by a known method, and the target compound can be obtained by removing the protecting group after the reaction, as necessary. Examples of such protecting groups include those described in "Greene's Protective Groups in Organic Synthesis", 4th Edition, by Wuts and

Greene, John Wiley & Sons Inc., 2006, or in "Protecting Groups", 3rd Edition, by P.J. Kocienski, Thieme, 2005, and may be appropriately selected and used according to the reaction conditions.

The compounds obtained in each step of the following production methods may be isolated or purified by conventional methods such as solvent extraction, concentration, distillation, sublimation, recrystallization, reprecipitation, or chromatography, or may be used in the next step in the form of a reaction mixture or crude product.

Unless otherwise specified, the reactions in the steps of the following production methods are carried out by appropriately modifying or combining known methods, for example, those described in "Comprehensive Organic Transformations: A Guide to Functional Group Preparations, 2nd Edition" by R.C. Larock, John Wiley & Sons Inc., 1999; "The Fourth Series of Experimental Chemistry" edited by the Chemical Society of Japan, Maruzen, 1992; "Strategic Applications of Named Reactions in Organic Synthesis" by L. Kuerti and B. Czako, supervised and translated by Kiyoshi Tomioka, Kagaku Dojin, 2006; and "Modern Organic Synthesis: An Introduction" by G.S. Zweifel and M.H. Nantz, translated by Tametsugu Hiyama, Kagaku Dojin, 2009, or the methods described in the Examples.

[1] Synthesis of Compound (1)

[Chem. 15]

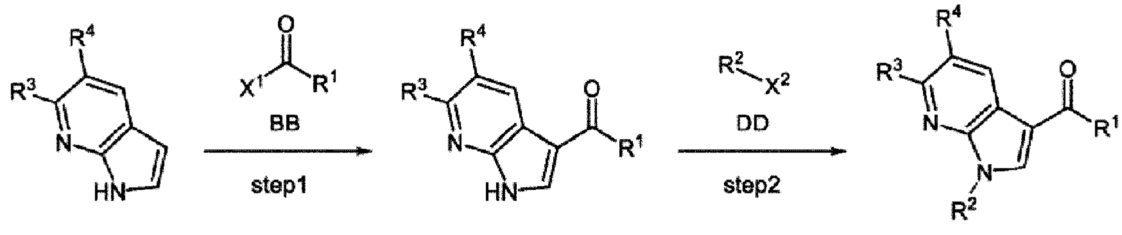


AA CC (1)

(where $R^1$, $R^3$, $R^3$ and $R^4$ are as defined above. $X^1$ represents chloro or bromo, and $X^2$ represents chloro, bromo, mesylate, or triflate.)

step 1

This step is a step of obtaining Compound (1) by subjecting a compound AA, which is commercially available or synthesized by a known method, to a Friedel-Crafts acylation reaction with an acid halide BB in the presence of a Lewis acid such as aluminum chloride in a solvent such as methylene chloride or dichloroethane at 0°C to 150°C, preferably 60°C to 120°C, for 1 hour to 48 hours, preferably 12 hours to 24 hours.

The acid halide BB can be obtained by reacting a compound LL or a compound PP described later, with oxalyl chloride or oxalyl bromide in a solvent such as methylene chloride or dichloroethane in the presence of a catalytic amount of dimethylformamide at 0°C to 50°C, preferably 0°C to room temperature, for 1 hour to 48 hours, preferably 1 hour to 12 hours.

step2

This step is a step of obtaining Compound (1) by alkylating a compound CC with an alkylating agent DD in the presence of a base, and it can be carried out in accordance with a known method for an alkylation reaction. The reaction can be carried out to obtain Compound (1) by reacting the compound CC with the alkylating agent DD in a solvent such as dimethylformamide or tetrahydrofuran in the presence of a base such as sodium hydride, potassium hydride, potassium carbonate, sodium carbonate, or cesium carbonate at 0°C to 120°C, preferably room temperature to 120°C, for 1 hour to 48 hours, preferably 1 hour to 12 hours.

[2] Synthesis of Compound AAa

[Chem. 16]

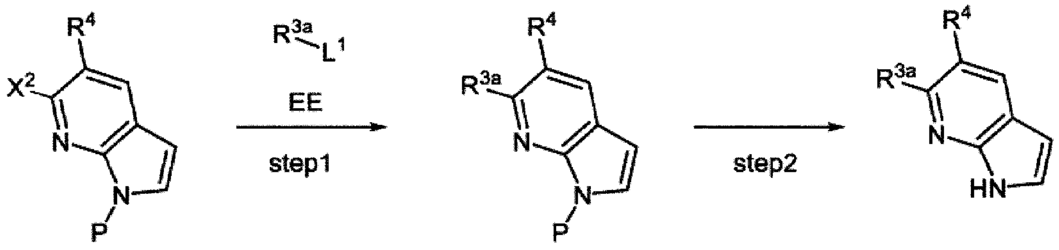


CC FF AAa

(where $R^4$ is as defined above. $R^{3a}$ represents a substituted or unsubstituted aryl group, a substituted or unsubstituted heteroaryl group, a substituted or unsubstituted alkoxy group, a substituted or unsubstituted aryloxy group, or a substituted or unsubstituted heteroaryloxy group. $X^2$ represents chloro, bromo, or triflate. $L^1$ represents boronic acid, boronic acid ester, alkyltin, zinc halide, or the like. P represents a protecting group, and examples thereof include Boc, Cbz, and Bn.)

step 1

This step is a step for obtaining a compound FF by a coupling reaction between a compound CC and a compound EE. Examples of the reaction can include Suzuki cross-coupling, Ullmann cross-coupling, Negishi cross-coupling, and Stille coupling. The compound FF can be obtained by reacting the compound CC with a boronic acid, a boronic acid ester, a trialkyltin, or a zinc halide, which is commercially available or synthesized by a known method, in a solvent such as dioxane, toluene, DMSO, DMF, DME, THF, or water, or a mixed solvent thereof, in the presence of a palladium catalyst such as $Pd(PPh_3)_4$, $Pd(OAc)_2$, $PdCl_2(PPh_3)_2$, or $Pd(dppf)_2Cl_2$, and a base such as potassium carbonate, sodium carbonate, or potassium phosphate, under a nitrogen atmosphere at 0°C to 150°C, preferably 60°C to 120°C, for 0.5 hours to 24 hours, preferably 1 hour to 12 hours.

Note that the protecting group (P) of the compound CC can be introduced with reference to "Greene's Protective Groups in Organic Synthesis", 4th Edition, by Wuts and Greene, John Wiley & Sons Inc., 2006, or "Protecting Groups", 3rd Edition, by P. J. Kocienski, Thiemes, 2005.

step2

This step is a step for obtaining AAa by deprotecting the protecting group of the compound FF, and it can be introduced with reference to "Greene's Protective Groups in Organic Synthesis", 4th Edition, by Wuts and Greene, John Wiley & Sons Inc., 2006, or "Protecting Groups", 3rd Edition, by P. J. Kocienski, Thiemes, 2005.

[3] Synthesis of Compound AAb

[Chem. 17]

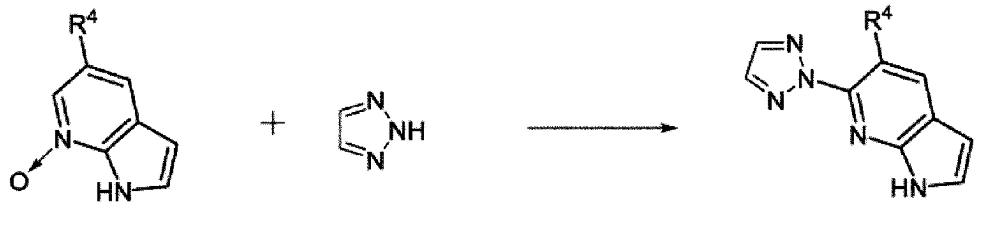

GG HH AAb

(where $R^4$ is as defined in General Formula (1).)

This step is a step for obtaining a compound AAb by subjecting a compound GG, which is commercially available or synthesized by a known method, to an addition reaction with HH in the presence of a base such as potassium carbonate in a solvent such as acetonitrile at 0°C to 100°C for 1 hour to 48 hours, preferably 12 hours to 24 hours.

[4] Synthesis of Compound LL

[Chem. 18]

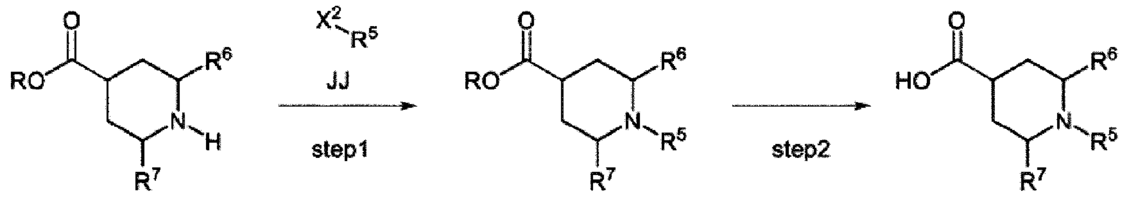


II KK LL

(where $R^5$, $R^6$, $R^7$, and $X^2$ are as defined above. R represents alkyl such as methyl or ethyl.)

step 1

This step is a step for obtaining a compound KK by performing an aromatic nucleophilic substitution reaction ($S_NAr$) on a compound II, which is commercially available or synthesized by a known method, in a solvent such as DMSO or DMF in the presence of TEA, DIPEA, and a compound JJ at 0°C to 150°C, preferably 120°C to 150°C, for 1 hour to 48 hours, preferably 1 hour to 10 hours.

step2

This step is a step for obtaining a compound LL by hydrolyzing an ester of a compound KK, and the compound LL can be synthesized by referring to "Greene's Protective Groups in Organic Synthesis" by Wuts and Greene, 4th edition, John Wiley & Sons Inc. 2006, or "Protecting Groups" by P. J. Kocienski, 3rd edition, Thiemes, 2005.

[5] Synthesis of Compound (PP)

[Chem. 19]

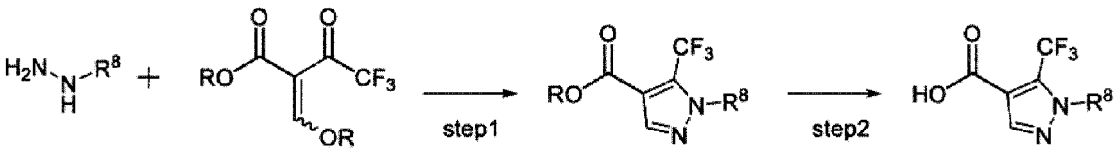


MM NN OO PP

(where $R^8$ is as defined above. R represents alkyl such as methyl or ethyl.)

step 1

This step is a step for obtaining a compound OO by reacting a compound MM and a compound NN, which are commercially available or synthesized by known methods, in a solvent such as ethanol or ether at 0°C to 80°C, and preferably in ethanol at 60°C to 80°C, for 1 hour to 48 hours, preferably 1 hour to 10 hours.

step2

This step is a step for obtaining a compound PP by hydrolyzing an ester of the compound OO, and the compound PP can be synthesized by referring to "Greene's Protective Groups in Organic Synthesis" by Wuts and Greene, 4th edition, John Wiley & Sons Inc. 2006, or "Protecting Groups" by P. J. Kocienski, 3rd edition, Thiemes, 2005.

## Examples

The present invention will be described in further detail below with reference to Reference Examples, Examples, and Test Examples, but the present invention is not limited thereto.
MS was measured by LCMS. As the ionization method, the ESI method was used. Observed mass spectrometry values are expressed in terms of m/z.
Measurement conditions of LCMS are as follows.

Analytical instrument: ACQUITY UPLC MS/PDA system (manufactured by Waters Corporation) mass spectrometer: Waters 3100 MS detector
Photodiode array detector: ACQUITY PDA detector (UV detection wavelength: 210 to 400 nm)
Column: Acquity BEH C18, 1.7 μm, 2.1 × 50 mm
Flow rate: 0.5 mL/min
Column temperature: 40°C
Solvent;
Mobile phase A: 0.1% formic acid/$H_2O$ (v/v; the same applies hereinafter)
Mobile phase B: 0.1% formic acid/acetonitrile

Measurement conditions of chiral column chromatography are as follows.

Analytical instrument: i-Series UHPLC model LC-2060C 3D (PDA model) (manufactured by Shimadzu Corporation)
Detection wavelength: 220 nm
Column: CHIRALPAK IB, 0.46 cmI.D. × 25 cmL
Flow rate: 1.0 mL/min
Column temperature: 40°C
Mobile phase: n-Hexane/IPA = 92/8 (v/v)

$^1$H NMR spectra were measured using the JNM-ECS400 nuclear magnetic resonance spectrometer (manufactured by JEOL RESONANCE Inc.). Observed peaks are expressed as chemical shift values δ (ppm) (s = singlet, d = doublet, t = triplet, q = quartet, brs = broad singlet, m = multiplet, dd = double doublet, and dt = double triplet).
For microwave experiments, Initiator 60 (manufactured by Biotage LLC) was used. It is capable of achieving temperatures ranging from 40°C to 250°C and reaching pressures of up to 20 bar.
The measurement conditions for specific rotation $[\alpha]_{589}$ are as follows.
Analytical instrument: Automatic polarimeter SEPA-500 (manufactured by HORIBA, Ltd.).
Optical path length: 50 mm
The melting point was measured using a micro melting point apparatus (Buchi, model B-565).
The compound names used in the present specification are those assigned using the nomenclature software ACD/NAME (registered trademark, Advanced Chemistry Development Inc.), ChemBioDraw (version 19.1, manufactured by Cambridge Soft), or named in accordance with IUPAC nomenclature.
The lowercase letters "r" and "s" in the compound names indicate the stereochemistry of pseudoasymmetric carbon atoms in accordance with IUPAC rules.

Reference Example 1: Synthesis of 5-chloro-1H-pyrrolo[2,3-b]pyridine-7-oxide

To a solution of 5-chloro-1H-pyrrolo[2,3-b]pyridine (30 g) in diethyl ether (1.4 L) was added m-chloroperbenzoic acid (94 g) in three portions, and the mixture was stirred at room temperature for 18 hours. The reaction mixture was filtered, and the resulting yellowish-green residue was suspended in water (0.72 L), followed by the addition of a 23% (w/v) aqueous potassium carbonate solution (0.31 kg), and the mixture was stirred at room temperature overnight. The reaction mixture was filtered, and the collected solid was dried to afford 25 g of the target compound as a grayish-brown solid.

Reference Example 2: Synthesis of 5-chloro-6-(2H-1,2,3-triazol-2-yl)-1H-pyrrolo[2,3-b]pyridine

To a suspension of 5-chloro-1H-pyrrolo[2,3-b]pyridine-7-oxide (2.6 g) in acetonitrile (80 mL) was added m-chlorobenzoic acid (2.4 g), and after stirring at room temperature for 30 minutes, dimethyl sulfate (1.5 mL) was added thereto, followed by stirring at an external temperature of 75°C for 20 hours. After allowing the reaction mixture to cool, potassium carbonate (11 g), 1,2,3-triazole (3.2 g), and acetonitrile (60 mL) were sequentially added, and the mixture was stirred at an external temperature of 60°C for 6 hours. After allowing the reaction mixture to cool, the mixture was diluted with water and subjected to extraction with ethyl acetate. The organic phase was washed with saturated brine and dried

over magnesium sulfate, and the solvent was then distilled off under reduced pressure. The resulting residue was purified by column chromatography to give a crude residue as a pink solid. This residue was slurry-washed with ethyl acetate (10 mL) to afford 1.6 g of the target compound as a pink solid.

Reference Example 3: Synthesis of 1-benzoyl-6-bromo-5-chloro-1H-pyrrolo[2,3-b]pyridine

To an ice-cooled solution of benzoyl bromide (3.5 mL) in toluene (60 mL) was added a pre-mixed suspension of bis(trimethylsilyl)amine (2.9 mL), 5-chloro-1H-pyrrolo[2,3-b]pyridine-7-oxide (2.0 g), and toluene (60 mL), followed by stirring at room temperature for 2 hours. After adding a saturated aqueous sodium bicarbonate solution to the reaction mixture, the mixture was diluted with water and subjected to extraction with ethyl acetate. The organic phase was washed with saturated brine and dried over magnesium sulfate, and the solvent was then distilled off under reduced pressure. The resulting residue was purified by column chromatography to afford 1.7 g of the target compound as a white solid.

Reference Example 4: Synthesis of 6-bromo-5-chloro-1H-pyrrolo[2,3-b]pyridine

To a solution of 1-benzoyl-6-bromo-5-chloro-1H-pyrrolo[2,3-b]pyridine (3.9 g) in a mixture of tetrahydrofuran (38 mL) and methanol (76 mL) was added an aqueous sodium hydroxide solution (2.0 M, 12 mL), followed by stirring at room temperature overnight. The reaction mixture was diluted with an aqueous sodium hydroxide solution (2.0 M, 0.10 L) and subjected to extraction with chloroform. The organic phase was washed with saturated brine and dried over magnesium sulfate, and the solvent was distilled off under reduced pressure to afford 2.7 g of the target compound as a light yellow solid.

Reference Example 5: Synthesis of tert-butyl 6-bromo-5-chloro-1H-pyrrolo[2,3-b]pyridine-1-carboxylate

To a solution of 6-bromo-5-chloro-1H-pyrrolo[2,3-b]pyridine (1.0 g) in N,N-dimethylformamide (10 mL) were sequentially added di-tert-butyl dicarbonate (hereinafter, referred to as "$Boc_2O$") (1.5 mL), triethylamine (1.8 mL), and 4-dimethylaminopyridine (hereinafter, referred to as "DMAP") (53 mg), and the mixture was stirred at room temperature overnight. The reaction mixture was directly purified by column chromatography to afford 1.4 g of the target compound as a colorless oil.

Reference Example 6: Synthesis of 5-chloro-6-(pyrimidin-2-yl)-1H-pyrrolo[2,3-b]pyridine

To a solution of tert-butyl 6-bromo-5-chloro-1H-pyrrolo[2,3-b]pyridine-1-carboxylate (1.4 g) in dimethyl sulfoxide (8.3 mL) were sequentially added tetrakis(triphenylphosphine)palladium(O) (0.96 g), lithium chloride (0.53 g), copper(I) chloride (82 mg), and 2-(tributylstannyl)pyrimidine (1.7 g), and the mixture was stirred at an external temperature of 80°C overnight. After allowing the reaction mixture to cool, the mixture was directly purified by column chromatography, followed by further purification by reverse-phase column chromatography ($H_2O$/MeOH) to afford 0.45 g of the target compound as a light yellow solid.

Reference Example 7: Synthesis of tert-butyl (7R)-7-methyl-1,4-dioxa-8-azaspiro[4.5]decane-8-carboxylate

To a solution of tert-butyl (2R)-2-methyl-4-oxopiperidine-1-carboxylate (5.2 g) in toluene (0.10 L) were sequentially added ethylene glycol (6.8 mL) and pyridinium p-toluenesulfonate (hereinafter, referred to as "PPTS") (1.5 g), and the mixture was stirred under heating and reflux at an external temperature of 145°C for 15 hours. After allowing the reaction mixture to cool, the mixture was neutralized with a saturated aqueous sodium bicarbonate solution. Subsequently, the mixture was diluted with water and subjected to extraction with ethyl acetate. The organic phase was washed with water, followed by saturated brine, and dried over magnesium sulfate, and the solvent was distilled off under reduced pressure. The resulting residue was purified by column chromatography to afford 4.6 g of the target compound as a colorless oil.

Reference Example 8: Synthesis of tert-butyl (7R,9R)-7,9-dimethyl-1,4-dioxa-8-azaspiro[4.5]decane-8-carboxylate

The compound was synthesized according to the method of Feringa et al. (Org. Biomol. Chem., 2008, vol. 6, pp. 3464-3466).

To a solution of tert-butyl (7R)-7-methyl-1,4-dioxa-8-azaspiro[4.5]decane-8-carboxylate (15 g) in diethyl ether (0.16 L) was added tetramethylethylenediamine (hereinafter, referred to as "TMEDA") (13 mL). After cooling the reaction mixture to -78°C in a dry ice-acetone bath, a solution of sec-butyllithium in hexane (1.22 M, 72 mL) was added dropwise, and the mixture was stirred at the same temperature for 1 hour. Subsequently, a solution of methyl iodide (7.3 mL) in diethyl ether (15 mL) was added dropwise, and the mixture was stirred at -78°C for 1 hour and then at room temperature for 16

hours. The reaction mixture was diluted with water and subjected to extraction with ethyl acetate. The organic phase was dried over magnesium sulfate, and the solvent was distilled off under reduced pressure. The resulting residue was purified by column chromatography to afford 8.5 g of the target compound (trans-isomer : cis-isomer = 10 : 1) as a white solid.

$$\text{Specific optical rotation } [\alpha]_D^{21} = +4.79° \text{ (c = 1.0, chloroform)}$$

Reference Example 9: Synthesis of tert-butyl (2R,6R)-2,6-dimethyl-4-oxopiperidine-1-carboxylate

To a solution of tert-butyl (7R,9R)-7,9-dimethyl-1,4-dioxa-8-azaspiro[4.5]decane-8-carboxylate (12 g) in acetone (0.61 L) was added p-toluenesulfonic acid monohydrate (hereinafter, referred to as "PTSA·$H_2O$") (9.1 g), and the mixture was stirred at room temperature for 1.5 hours. After ice-cooling the reaction mixture, an aqueous solution (69 g) in which sodium carbonate (6.3 g) was dissolved was added to neutralize the mixture, and the reaction solvent was distilled off under reduced pressure. The resulting residue was diluted with water and subjected to extraction with ethyl acetate. The organic phase was dried over magnesium sulfate, and the solvent was distilled off under reduced pressure. The resulting residue was purified by column chromatography to afford 8.9 g of the target compound (trans-isomer : cis-isomer = 10 : 1) as a white solid.

Reference Example 10: Synthesis of tert-butyl (2R,6R)-4-cyano-2,6-dimethylpiperidine-1-carboxylate

To a solution of tert-butyl (2R,6R)-2,6-dimethyl-4-oxopiperidine-1-carboxylate (8.9 g) in a mixture of tert-butyl alcohol (47 mL) and 1,2-dimethoxyethane (0.19 L) was added toluenesulfonylmethyl isocyanide (hereinafter, referred to as "TosMIC") (12 g). After ice-cooling the reaction mixture, potassium tert-butoxide (13 g) was added, and the mixture was stirred at the same temperature for 30 minutes and then stirred at room temperature for 15 hours. After ice-cooling the reaction mixture, the mixture was diluted with water, and the reaction solvent was distilled off under reduced pressure. The mixture was subjected to extraction with ethyl acetate, and the organic phase was washed with water, followed by saturated brine, and dried over magnesium sulfate, and the solvent was distilled off under reduced pressure. The resulting residue was purified by column chromatography to afford 5.4 g of the target compound as a light yellow solid.

Reference Example 11: Synthesis of ethyl (2R,6R)-2,6-dimethylpiperidine-4-carboxylate

Step 1

To a solution of tert-butyl (2R,6R)-4-cyano-2,6-dimethylpiperidine-1-carboxylate (13 g) in a mixture of ethanol (0.11 L) and water (0.11 L) was added potassium hydroxide (18 g), and the mixture was stirred under heating and reflux at an external temperature of 95°C for 20 hours. After allowing the reaction mixture to cool, the mixture was diluted with water and neutralized with a 20% (w/v) aqueous citric acid solution to adjust the pH to 5. The mixture was subjected to extraction with ethyl acetate, and the organic phase was washed with water, followed by saturated brine, and dried over magnesium sulfate, and the solvent was distilled off under reduced pressure to afford the residue (14 g) as a white solid.

Step 2

The residue (13 g) obtained in Step 1 was dissolved in a 2.0 M solution of hydrogen chloride in ethanol (0.68 L), and the mixture was stirred under heating and reflux at an external temperature of 120°C for 30 hours. After allowing the reaction mixture to cool, the reaction solvent was distilled off under reduced pressure. After ice-cooling the resulting residue, an aqueous sodium hydroxide solution (2.0 M) was added to adjust the pH to 13. The mixture was subjected to extraction with chloroform, and the organic phase was washed with saturated brine and dried over magnesium sulfate, and the solvent was distilled off under reduced pressure to afford 8.9 g of the target compound as a yellow oil.

$$\text{Specific optical rotation } [\alpha]_D^{20} = -16.66° \text{ (c = 1.0, chloroform)}$$

Reference Example 12: Synthesis of ethyl (2R,6R)-1-(5-fluoro-3-nitropyridin-2-yl)-2,6-dimethylpiperidine-4-carboxylate

To a solution of ethyl (2R,6R)-2,6-dimethylpiperidine-4-carboxylate (2.2 g) in dimethyl sulfoxide (24 mL) were sequentially added N,N-diisopropylethylamine (hereinafter, referred to as "DIPEA") (4.0 mL) and 2,5-difluoro-3-nitropyridine (2.1 g), and the mixture was stirred at an external temperature of 140°C for 3 hours. After allowing the reaction mixture to cool, the mixture was directly purified by column chromatography to afford 2.9 g of the target compound as an orange oil.

Reference Example 13: Synthesis of ethyl (2R,6R)-1-(3-amino-5-fluoropyridin-2-yl)-2,6-dimethylpiperidine-4-carboxylate

To a solution of ethyl (2R,6R)-1-(5-fluoro-3-nitropyridin-2-yl)-2,6-dimethylpiperidine-4-carboxylate (3.4 g) in methanol (0.10 L) was added Pt/C type STD (Pt 3%) (1.1 g), and the mixture was stirred at an external temperature of 45°C under a hydrogen gas atmosphere (5.0 atm) for 4 hours. After allowing the reaction mixture to cool, the mixture was filtered through Celite, and the solvent was distilled off under reduced pressure. The resulting residue was purified by column chromatography to afford 3.0 g of the target compound as a white solid.

Reference Example 14: Synthesis of ethyl (2R,6R)-1-(5-fluoro-3-iodopyridin-2-yl)-2,6-dimethylpiperidine-4-carboxylate

To a solution of ethyl (2R,6R)-1-(3-amino-5-fluoropyridin-2-yl)-2,6-dimethylpiperidine-4-carboxylate (8.0 g) in diiodomethane (80 mL) was added isoamyl nitrite (6.4 mL), and the mixture was stirred at an external temperature of 80°C for 7 hours. After allowing the reaction mixture to cool, the mixture was directly purified by column chromatography to afford 4.5 g of the target compound as a light yellow oil.

Reference Example 15: Synthesis of (2R,6R)-1-(5-fluoro-3-iodopyridin-2-yl)-2,6-dimethylpiperidine-4-carboxylic acid

To a solution of ethyl (2R,6R)-1-(5-fluoro-3-iodopyridin-2-yl)-2,6-dimethylpiperidine-4-carboxylate (4.5 g) in a mixture of tetrahydrofuran (55 mL), methanol (22 mL), and water (33 mL) was added lithium hydroxide monohydrate (1.2 g), and the mixture was stirred at room temperature overnight. After ice-cooling the reaction mixture, an aqueous hydrochloric acid solution (1.0 M) was added to adjust the pH to 2. The mixture subjected to extraction with ethyl acetate, the organic phase was washed with saturated brine and dried over magnesium sulfate, and the solvent was then distilled off under reduced pressure. The resulting residue was purified by column chromatography to afford 4.1 g of the target compound as a white solid.

Reference Example 16: Synthesis of [5-chloro-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone

Step 1

To a solution of (2R,6R)-1-(5-fluoro-3-iodopyridin-2-yl)-2,6-dimethylpiperidine-4-carboxylic acid (0.12 g) in dichloromethane (2.0 mL) were added N,N-dimethylformamide (2.5 μL) and oxalyl chloride (35 μL), and the mixture was stirred at room temperature for 30 minutes. The reaction mixture was distilled off under reduced pressure to afford a yellow amorphous solid.

Step 2

To a solution of 5-chloro-6-(2H-1,2,3-triazol-2-yl)-1H-pyrrolo[2,3-b]pyridine (70 mg) in 1,2-dichloroethane (2.0 mL) was added aluminum trichloride (0.21 g), and the mixture was stirred at room temperature for 10 minutes. Subsequently, a solution of the light yellow amorphous solid obtained in Step 1 in 1,2-dichloroethane (4.0 mL) was added dropwise to the reaction mixture, and the mixture was stirred at an external temperature of 50°C for 2 hours. After ice-cooling the reaction mixture, the mixture was diluted with water and subjected to extraction with ethyl acetate. The organic phase was washed with saturated brine and dried over magnesium sulfate, and the solvent was then distilled off under reduced pressure. The resulting residue was purified by column chromatography to afford 0.17 g of the target compound as a white solid.

Reference Example 17: Synthesis of [1-(2-{[tert-butyl(dimethyl)silyl]oxy}ethyl)-5-chloro-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone

To a solution of [5-chloro-6-(2H-1,2,3-triazol-2-yl)-1H-pyrrolo[2,3-b]pyridin-3-yl] [(2R,6R)-1-(5-fluoro-3-iodopyridin-2-yl)-2,6-dimethylpiperidin-4-yl]methanone (30 mg) in N,N-dimethylformamide (0.50 mL) were sequentially added potassium carbonate (21 mg) and (2-bromoethoxy)(tert-butyl)dimethylsilane (37 mg), and the mixture was stirred at an external temperature of 50°C for 1 hour. 2-Bromoethoxy-tert-butyldimethylsilane (37 mg) was added again, and the

mixture was stirred at an external temperature of 50°C for 1 hour. After allowing the reaction mixture to cool, the mixture was directly purified by column chromatography to afford 42 mg of the target compound as a colorless oil.

Reference Example 18: Synthesis of tert-butyl 3-{5-chloro-3-[(2R,6R)-1-(5-fluoro-3-iodopyridin-2-yl)-2,6-dimethylpiperidine-4-carbonyl]-6-(2H-1,2,3-triazol-2-yl)-1H-pyrrolo[2,3-b]pyridin-1-yl}azetidine-1-carboxylate

To a solution of [5-chloro-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone (45 mg) in N,N-dimethylformamide (1.0 mL) were sequentially added potassium carbonate (27 mg) and tert-butyl 3-iodoazetidine-1-carboxylate (0.11 g), and the mixture was stirred at an external temperature of 100°C for 10 hours. After allowing the reaction mixture to cool, the mixture was directly purified by reverse-phase column chromatography ($H_2O$/MeOH) to afford 59 mg of the target compound as a light brown solid.

Reference Example 19: Synthesis of [5-chloro-6-(2H-1,2,3-triazol-2-yl)-1H-pyrrolo[2,3-b]pyridin-3-yl][1-(8-fluoroisoquinolin-4-yl)-5-(trifluoromethyl)-1H-pyrazol-4-yl]methanone

Step 1

To a solution of 1-(8-fluoroisoquinolin-4-yl)-5-(trifluoromethyl)-1H-pyrazole-4-carboxylic acid (0.10 g) (synthesized according to WO2018/119036) in dichloromethane (2.5 mL) were added N,N-dimethylformamide (2.4 $\mu$L) and oxalyl chloride (33 $\mu$L), and the mixture was stirred at room temperature for 30 minutes. The reaction mixture was distilled off under reduced pressure to afford a light yellow solid.

Step 2

To a solution of 5-chloro-6-(2H-1,2,3-triazol-2-yl)-1H-pyrrolo[2,3-b]pyridine (60 mg) in 1,2-dichloroethane (2.5 mL) was added aluminum trichloride (0.18 g), and the mixture was stirred at room temperature for 10 minutes. Subsequently, a solution of the light yellow solid obtained in Step 1 in 1,2-dichloroethane (2.5 mL) was added dropwise to the reaction mixture, and the mixture was stirred at an external temperature of 75°C for 3 hours. After ice-cooling the reaction mixture, the mixture was diluted with water and subjected to extraction with ethyl acetate. The organic phase was washed with saturated brine and dried over magnesium sulfate, and the solvent was then distilled off under reduced pressure. The resulting residue was slurry-washed with methanol to afford 0.13 g of the target compound as a light brown solid.

Reference Example 20: Synthesis of [5-chloro-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone

Step 1

To a solution of (2R,6R)-1-(5-fluoro-3-iodopyridin-2-yl)-2,6-dimethylpiperidine-4-carboxylic acid (0.18 g) in dichloromethane (3.0 mL) were added N,N-dimethylformamide (3.6 $\mu$L) and oxalyl chloride (59 $\mu$L), and the mixture was stirred at room temperature for 30 minutes. The reaction mixture was distilled off under reduced pressure to afford a yellow amorphous solid.

Step 2

To a solution of 5-chloro-6-(pyrimidin-2-yl)-1H-pyrrolo[2,3-b]pyridine (0.10 g) in 1,2-dichloroethane (3.0 mL) was added aluminum trichloride (0.29 g), and the mixture was stirred at room temperature for 10 minutes. Subsequently, a solution of the yellow amorphous solid obtained in Step 1 in 1,2-dichloroethane (3.0 mL) was added dropwise to the reaction mixture, and the mixture was stirred at an external temperature of 50°C for 2 hours. Furthermore, the mixture was stirred at an external temperature of 70°C overnight. After ice-cooling the reaction mixture, the mixture was diluted with water and subjected to extraction with ethyl acetate. The organic phase was washed with a saturated aqueous sodium bicarbonate solution, dried over magnesium sulfate, and the solvent was distilled off under reduced pressure. The resulting residue was purified by column chromatography to afford 0.10 g of the target compound as a white solid.

Reference Example 21: Synthesis of [1-(3-{[tert-butyl(dimethyl)silyl]oxy}propyl)-5-chloro-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone

To a solution of [5-chloro-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone (30 mg) in N,N-dimethylformamide (0.50 mL) were sequentially added potassium carbonate (35 mg) and (3-bromopropoxy)(tert-butyl)dimethylsilane (64 mg), and the mixture was stirred at an external temperature of 50°C for 3 hours. After allowing the reaction mixture to cool, the mixture was directly purified by column chromatography to afford 47 mg of the target compound as a colorless oil.

Reference Example 22: Synthesis of [5-chloro-6-(pyrimidin-2-yl)-1H-pyrrolo[2,3-b]pyridin-3-yl][1-(8-fluoroisoquinolin-4-yl)-5-(trifluoromethyl)-1H-pyrazol-4-yl]methanone

Step 1

To a solution of 1-(8-fluoroisoquinolin-4-yl)-5-(trifluoromethyl)-1H-pyrazole-4-carboxylic acid (0.15 g) (synthesized according to WO2018/119036) in dichloromethane (3.0 mL) were added N,N-dimethylformamide (3.6 μL) and oxalyl chloride (49 μL), and the mixture was stirred at room temperature for 30 minutes. The reaction mixture was distilled off under reduced pressure to afford a light yellow solid.

Step 2

To a solution of 5-chloro-6-(pyrimidin-2-yl)-1H-pyrrolo[2,3-b]pyridine (90 mg) in 1,2-dichloroethane (2.0 mL) was added aluminum trichloride (0.26 g), and the mixture was stirred at room temperature for 10 minutes. Subsequently, a solution of the light yellow solid obtained in Step 1 in 1,2-dichloroethane (2.0 mL) was added dropwise to the reaction mixture, and the mixture was stirred at an external temperature of 75°C for 4 hours. The mixture was further stirred under heating and reflux at an external temperature of 100°C for 2 hours. After ice-cooling the reaction mixture, the mixture was diluted with water and subjected to extraction with ethyl acetate. The organic phase was washed twice with an aqueous sodium hydroxide solution (2.0 M), followed by washing with a saturated aqueous sodium bicarbonate solution, and finally with saturated brine, then dried over magnesium sulfate, and the solvent was distilled off under reduced pressure. The resulting residue was purified by column chromatography to give a crude residue. The resulting residue was suspended in methanol and collected by filtration to afford 46 mg of the target compound as a light yellow solid.

Reference Example 23: Synthesis of 1,1-dioxotetrahydro-2H-thiopyran-4-yl methanesulfonate

To a solution of 4-hydroxytetrahydro-2H-thiopyran 1,1-dioxide (0.20 g) in dichloromethane (3.3 mL) were sequentially added triethylamine (0.37 mL) and methanesulfonyl chloride (0.15 mL), and the mixture was stirred at room temperature for 4 hours. The reaction mixture was directly purified by column chromatography to afford 0.28 g of the target compound as a white solid.

Reference Example 24: Synthesis of oxan-4-yl methanesulfonate

To a solution of oxan-4-ol (0.20 g) in dichloromethane (4.8 mL) were sequentially added triethylamine (0.54 mL) and methanesulfonyl chloride (0.22 mL), and the mixture was stirred at room temperature for 4 hours. The reaction mixture was directly purified by column chromatography to afford 0.34 g of the target compound as a white solid.

Reference Example 25: Synthesis of (3R)-oxolan-3-yl methanesulfonate

To a solution of (3R)-oxolan-3-ol (0.20 g) in dichloromethane (5.6 mL) were sequentially added triethylamine (0.63 mL) and methanesulfonyl chloride (0.26 mL), and the mixture was stirred at room temperature for 2 hours. The reaction mixture was directly purified by column chromatography to afford 0.36 g of the target compound as a light yellow oil.

Reference Example 26: Synthesis of (3S)-oxolan-3-yl methanesulfonate

To a solution of (3S)-oxolan-3-ol (0.30 g) in dichloromethane (8.5 mL) were sequentially added triethylamine (0.94 mL) and methanesulfonyl chloride (0.39 mL), and the mixture was stirred at room temperature for 3 hours. The reaction mixture was directly purified by column chromatography to afford 0.40 g of the target compound as a light yellow oil.

Reference Example 27: Synthesis of [5-chloro-6-(2H-1,2,3-triazol-2-yl)-1H-pyrrolo[2,3-b]pyridin-3-yl][1-(thieno[2,3-b]pyridin-4-yl)-5-(trifluoromethyl)-1H-pyrazol-4-yl]methanone

Step 1

To a solution of 1-(thieno[2,3-b]pyridin-4-yl)-5-(trifluoromethyl)-1H-pyrazole-4-carboxylic acid (65 mg) (synthesized according to WO2018/119036) in dichloromethane (1.5 mL) and tetrahydrofuran (1.5 mL) were added N,N-dimethylformamide (1.6 μL) and oxalyl chloride (32 μL), and the mixture was stirred at room temperature for 30 minutes. The reaction mixture was distilled off under reduced pressure to afford a light brown solid.

Step 2

To a solution of 5-chloro-6-(2H-1,2,3-triazol-2-yl)-1H-pyrrolo[2,3-b]pyridine (40 mg) in 1,2-dichloroethane (2.5 mL) was added aluminum trichloride (0.12 g), and the mixture was stirred at room temperature for 10 minutes. Subsequently, a solution of the light brown solid obtained in Step 1 in 1,2-dichloroethane (2.5 mL) was added dropwise to the reaction mixture, and the mixture was stirred at an external temperature of 75°C for 4 hours. After ice-cooling the reaction mixture, the mixture was diluted with water and subjected to extraction with ethyl acetate. The organic phase was washed with saturated brine and dried over magnesium sulfate, and the solvent was then distilled off under reduced pressure. The resulting residue was slurry-washed with ethyl acetate and methanol to afford 0.062 g of the target compound as a light brown solid.

Reference Example 28: Synthesis of [5-chloro-6-(2H-1,2,3-triazol-2-yl)-1H-pyrrolo[2,3-b]pyridin-3-yl][1-(pyrazolo[1,5-a]pyridin-4-yl)-5-(trifluoromethyl)-1H-pyrazol-4-yl]methanone

Step 1

To a solution of 1-(pyrazolo[1,5-a]pyridin-4-yl)-5-(trifluoromethyl)-1H-pyrazole-4-carboxylic acid (65 mg) (synthesized according to WO2018/119036) in dichloromethane (1.5 mL) and tetrahydrofuran (1.5 mL) were added N,N-dimethylformamide (1.7 μL) and oxalyl chloride (23 μL), and the mixture was stirred at room temperature for 30 minutes. The reaction mixture was distilled off under reduced pressure to afford a light brown oil.

Step 2

To a solution of 5-chloro-6-(2H-1,2,3-triazol-2-yl)-1H-pyrrolo[2,3-b]pyridine (40 mg) in 1,2-dichloroethane (2.5 mL) was added aluminum trichloride (120 mg), and the mixture was stirred at room temperature for 10 minutes. Subsequently, a solution of the light brown oil obtained in Step 1 in 1,2-dichloroethane (2.5 mL) was added dropwise to the reaction mixture, and the mixture was stirred at an external temperature of 75°C for 5 hours. After ice-cooling the reaction mixture, the mixture was diluted with water and subjected to extraction with ethyl acetate. The organic phase was washed with saturated brine and dried over magnesium sulfate, and the solvent was then distilled off under reduced pressure. The resulting residue was slurry-washed with methanol to afford 0.081 g of the target compound as a light brown solid.

Reference Example 29: Synthesis of [1-(1,2,3-benzothiadiazol-7-yl)-5-(trifluoromethyl)-1H-pyrazol-4-yl][5-chloro-6-(2H-1,2,3-triazol-2-yl)-1H-pyrrolo[2,3-b]pyridin-3-yl]methanone

Step 1

To a solution of 1-(1,2,3-benzothiadiazol-7-yl)-5-(trifluoromethyl)-1H-pyrazole-4-carboxylic acid (100 mg) (synthesized according to WO2018/119036) in dichloromethane (1.5 mL) and tetrahydrofuran (1.5 mL) were added N,N-dimethylformamide (2.6 μL) and oxalyl chloride (36 μL), and the mixture was stirred at room temperature for 30 minutes. The reaction mixture was distilled off under reduced pressure to afford a light brown solid.

Step 2

To a solution of 5-chloro-6-(2H-1,2,3-triazol-2-yl)-1H-pyrrolo[2,3-b]pyridine (70 mg) in 1,2-dichloroethane (3.1 mL) was added aluminum trichloride (210 mg), and the mixture was stirred at room temperature for 10 minutes. Subsequently, a solution of the light brown solid obtained in Step 1 in 1,2-dichloroethane (2.5 mL) was added dropwise to the reaction mixture, and the mixture was stirred at an external temperature of 75°C for 5 hours. After ice-cooling the reaction mixture, the mixture was diluted with water and subjected to extraction with ethyl acetate. The organic phase was washed with saturated brine and dried over magnesium sulfate, and the solvent was then distilled off under reduced pressure. The resulting residue was slurry-washed with methanol to give a crude residue. The resulting residue was

purified by column chromatography to give a crude residue. The resulting residue was slurry-washed with methanol to afford 120 mg of the target compound as a white solid.

Reference Example 30: Synthesis of 5-chloro-1-methyl-6-(pyrimidin-2-yl)-1H-pyrrolo[2,3-b]pyridine

To a solution of 5-chloro-6-(pyrimidin-2-yl)-1H-pyrrolo[2,3-b]pyridine in N,N-dimethylformamide (3.0 mL) were sequentially added potassium carbonate (930 mg) and methyl iodide (420 μL), and the mixture was stirred at room temperature for 30 minutes. Furthermore, methyl iodide (250 μL) was added, and the mixture was stirred at the same temperature for 30 minutes. The reaction mixture was diluted with water and subjected to extraction with ethyl acetate. The organic phase was washed with saturated brine and dried over magnesium sulfate, and the solvent was then distilled off under reduced pressure. The resulting residue was purified by column chromatography to afford 130 mg of the target compound as a light yellow solid.

Reference Example 31: Synthesis of [5-chloro-6-(2H-1,2,3-triazol-2-yl)-1H-pyrrolo[2,3-b]pyridin-3-yl][1-(1-methoxyisoquinolin-5-yl)-5-(trifluoromethyl)-1H-pyrazol-4-yl]methanone

Step 1

To a solution of 1-(1-methoxyisoquinolin-5-yl)-5-(trifluoromethyl)-1H-pyrazole-4-carboxylic acid (120 mg) (synthesized according to WO2018/119036) in dichloromethane (1.7 mL) and tetrahydrofuran (1.7 mL) were added N,N-dimethylformamide (2.7 μL) and oxalyl chloride (37 μL), and the mixture was stirred at room temperature for 30 minutes. The reaction mixture was distilled off under reduced pressure to afford a light brown solid.

Step 2

To a solution of 5-chloro-6-(2H-1,2,3-triazol-2-yl)-1H-pyrrolo[2,3-b]pyridine (68 mg) in 1,2-dichloroethane (3.0 mL) was added aluminum trichloride (200 mg), and the mixture was stirred at room temperature for 10 minutes. Subsequently, a solution of the light brown solid obtained in Step 1 in 1,2-dichloroethane (2.5 mL) was added dropwise to the reaction mixture, and the mixture was stirred at an external temperature of 75°C for 6 hours. After ice-cooling the reaction mixture, the mixture was diluted with water and subjected to extraction with ethyl acetate. The organic phase was washed with saturated brine and dried over magnesium sulfate, and the solvent was then distilled off under reduced pressure. The resulting residue was slurry-washed with ethyl acetate and methanol to give a crude residue. The resulting residue was purified by column chromatography to afford 68 mg of the target compound as a light yellow solid.

Reference Example 32: Synthesis of 9-bromo-4H-quinolizin-4-one

Step 1

After cooling a solution of lithium diisopropylamide in tetrahydrofuran (2.0 M, 13 mL) to -60°C in a dry ice/40% aqueous methanol bath, a solution of 3-bromo-2-methylpyridine in tetrahydrofuran (3.4 g, 40 mL) was added dropwise, and the mixture was stirred at the same temperature for 1 hour. Subsequently, diethyl ethoxymethylenemalonate (4.0 g) was added dropwise, and the mixture was allowed to warm to -20°C in a dry ice/70% aqueous methanol bath and stirred at the same temperature for 3 hours. After adding an aqueous ammonium chloride solution to the reaction mixture, the mixture was subjected to extraction with ethyl acetate. The organic phase was dried over magnesium sulfate, and the solvent was distilled off under reduced pressure. The resulting residue was purified by column chromatography to afford 4.3 g of a yellow oil.

Step 2

Polyphosphoric acid (25 g) was added to the yellow oil obtained in Step 1, and the mixture was stirred at an external temperature of 140°C for 6 hours. After ice-cooling the reaction mixture, the mixture was diluted with water and extracted with ethyl acetate and tetrahydrofuran. The organic phase was washed with saturated brine and dried over magnesium sulfate, and the solvent was then distilled off under reduced pressure. The resulting residue was purified by column chromatography to give a crude residue. The resulting residue was slurry-washed with a small amount of ice-cooled ethyl acetate to afford 1.5 g of the target compound as a yellow solid.

Reference Example 33: Synthesis of ethyl 1-(4-oxo-4H-quinolizin-9-yl)-5-(trifluoromethyl)-1H-pyrazole-4-carboxylate

Step 1

To a solution of palladium(1-phenylallyl) chloride dimer (57 mg) in 1,4-dioxane (10 mL) was added 4-{2-[di(adamantan-1-yl)phosphanyl]phenyl}morpholine (100 mg), and the mixture was stirred at room temperature under an argon atmosphere for 15 minutes. To the reaction mixture were sequentially added 9-bromo-4H-quinolizin-4-one (500 mg), sodium tert-butoxide (420 mg), and hydrazine hydrate (220 mg), and the mixture was stirred at an external temperature of 50°C under an argon atmosphere for 4 hours. The reaction mixture was filtered through Celite and washed with tetrahydrofuran, and the mother liquor was concentrated to obtain a brown solid.

Step 2

To a suspension of the brown solid obtained in Step 1 in ethanol (10 mL) were added triethylamine (0.62 mL) and ethyl (2Z)-2-(ethoxymethylidene)-4,4,4-trifluoro-3-oxobutanoate, and the mixture was stirred at an external temperature of 80°C for 12 hours. After allowing the reaction mixture to cool, the solvent was distilled off under reduced pressure, and the resulting residue was purified by column chromatography to give a crude residue. The resulting residue was slurry-washed with a small amount of diethyl ether to afford 130 mg of the target compound as an orange solid.

<u>Reference Example 34: Synthesis of 1-(4-oxo-4H-quinolizin-9-yl)-5-(trifluoromethyl)-1H-pyrazole-4-carboxylic acid</u>

To a solution of ethyl 1-(4-oxo-4H-quinolizin-9-yl)-5-(trifluoromethyl)-1H-pyrazole-4-carboxylate (130 mg) in tetrahydrofuran (3.0 mL) were added water (1.5 mL) and lithium hydroxide monohydrate (38 mg), and the mixture was stirred at room temperature for 2 hours. To the reaction mixture was added an aqueous hydrochloric acid solution (1.0 M) to adjust the pH to 5. The solvent of the reaction mixture was distilled off under reduced pressure. The resulting residue was purified by reverse-phase column chromatography to afford 4.1 g of the target compound as a white solid. After allowing the reaction mixture to cool, the mixture was directly purified by column chromatography ($H_2O$/MeOH) to afford 120 mg of the target compound as a yellow solid.

<u>Reference Example 35: Synthesis of 9-{4-[5-chloro-6-(2H-1,2,3-triazol-2-yl)-1H-pyrrolo[2,3-b]pyridine-3-carbonyl]-5-(trifluoromethyl)-1H-pyrazol-1-yl}-4H-quinolizin-4-one</u>

Step 1

To a suspension of 1-(4-oxo-4H-quinolizin-9-yl)-5-(trifluoromethyl)-1H-pyrazole-4-carboxylic acid (120 mg) in dichloromethane (2.5 mL) and tetrahydrofuran (1.5 mL) were added N,N-dimethylformamide (2.7 μL) and oxalyl chloride (39 μL), and the mixture was stirred at room temperature for 30 minutes. The reaction mixture was distilled off under reduced pressure to afford a brown solid.

Step 2

To a solution of 5-chloro-6-(2H-1,2,3-triazol-2-yl)-1H-pyrrolo[2,3-b]pyridine (70 mg) in 1,2-dichloroethane (2.5 mL) was added aluminum trichloride (0.21 g), and the mixture was stirred at room temperature for 10 minutes. Subsequently, a suspension of the brown solid obtained in Step 1 in 1,2-dichloroethane (2.5 mL) was added dropwise to the reaction mixture, and the mixture was stirred at an external temperature of 75°C for 3 hours. Aluminum trichloride (100 mg) was added to the reaction mixture, and the mixture was stirred at the same temperature for 2 hours. After ice-cooling the reaction mixture, the mixture was diluted with water and extracted with ethyl acetate. The organic phase was washed with saturated brine and dried over magnesium sulfate, and the solvent was then distilled off under reduced pressure. The resulting residue was slurry-washed with methanol to afford 120 mg of the target compound as a tan solid.

<u>Reference Example 36: Synthesis of 5-chloro-6-methoxy-1-methyl-1H-pyrrolo[2,3-b]pyridine</u>

Step 1

After ice-cooling a solution of 5-chloro-6-fluoro-1H-pyrrolo[2,3-b]pyridine (300 mg) (synthesized according to WO2021/062316) in tetrahydrofuran (8.7 mL), sodium hydride (100 mg) was added, and the mixture was stirred at the same temperature for 5 minutes. Methyl iodide (0.18 mL) was added to the reaction mixture, and the mixture was stirred at the same temperature for 30 minutes, then allowed to warm to room temperature and stirred for 2 hours. The reaction mixture was diluted with water and subjected to extraction with ethyl acetate. The organic phase was washed with saturated brine and dried over sodium sulfate, and the solvent was distilled off under reduced pressure. The resulting

residue was purified by reverse-phase column chromatography ($H_2O$/MeOH) to afford 290 mg of a white solid.

Step 2

To a suspension of the white solid obtained in Step 1 in methanol (1.2 mL) was added potassium carbonate (100 mg), and the mixture was stirred at an external temperature of 50°C overnight. The reaction mixture was directly purified by column chromatography to afford 16 mg of the target compound as a white solid.

Reference Example 37: Synthesis of ethyl (2RS,4RS)-2-methylpiperidine-4-carboxylate

Step 1

To a suspension of 2-chloro-6-methylpyridine-4-carboxylic acid (10 g) in acetic acid (50 mL) was added platinum dioxide hydrate (0.37 g), and the mixture was stirred at room temperature for 30 minutes. The mixture was stirred under a hydrogen gas atmosphere (0.4 MPa) at an external temperature of 50°C for 8 hours and then at room temperature for 12 hours. Furthermore, the mixture was stirred at an external temperature of 60°C for 8 hours. After ice-cooling the reaction mixture, the mixture was diluted with methanol and filtered through Celite, and the solvent was distilled off under reduced pressure. The resulting residue was slurry-washed with a mixed solvent of ethyl acetate and methanol to afford 10 g of a white solid.

Step 2

To a solution of the white solid (3.6 g) obtained in Step 1 in ethanol (100 mL) was added sulfuric acid (10 mL) dropwise, and the mixture was stirred at an external temperature of 95°C for 42 hours. The solvent of the reaction mixture was distilled off under reduced pressure, and the resulting residue was diluted with ethyl acetate, neutralized with an aqueous sodium hydroxide solution (2.0 M) under ice-cooling, and then subjected to extraction with ethyl acetate. The organic phase was washed with saturated brine and dried over magnesium sulfate, and the solvent was distilled off under reduced pressure to afford the target compound as a light yellow oil (3.3 g).

Reference Example 38: Synthesis of ethyl (2RS,4RS)-1-(5-bromopyrimidin-4-yl)-2-methylpiperidine-4-carboxylate

To a solution of ethyl 2-methylpiperidine-4-carboxylate (cis-racemate) (100 mg) in dimethyl sulfoxide (1.0 mL) were sequentially added DIPEA (0.20 mL) and 5-bromo-4-chloro-pyrimidine (140 mg), and the mixture was stirred at an external temperature of 140°C for 90 minutes. After allowing the reaction mixture to cool, the mixture was directly purified by column chromatography to afford 110 mg of the target compound as a yellow oil.

Reference Example 39: Synthesis of (2RS,4RS)-1-(5-bromopyrimidin-4-yl)-2-methylpiperidine-4-carboxylic acid

To a mixed solution of ethyl 1-(5-bromopyrimidin-4-yl)-2-methylpiperidine-4-carboxylate (cis-racemate) (110 mg) in tetrahydrofuran (2.5 mL) and water (2.5 mL) was added lithium hydroxide monohydrate (36 mg), and the mixture was stirred at room temperature for 2 hours. To the reaction mixture was added an aqueous hydrochloric acid solution (1.0 M) to adjust the pH to 4. The mixture subjected to extraction with ethyl acetate, the organic phase was washed with saturated brine and dried over magnesium sulfate, and the solvent was then distilled off under reduced pressure. The resulting residue was slurry-washed with a mixed solvent of ethyl acetate and diethyl ether to afford 90 mg of the target compound as a white solid.

Reference Example 40: Synthesis of (2RS,4RS)-[1-(5-bromopyrimidin-4-yl)-2-methylpiperidin-4-yl][5-chloro-6-(2H-1,2,3-triazol-2-yl)-1H-pyrrolo[2,3-b]pyridin-3-yl]methanone

Step 1

To a solution of 1-(5-bromopyrimidin-4-yl)-2-methylpiperidine-4-carboxylic acid (cis-racemate) (45 mg) in dichloromethane (2.5 mL) were added N,N-dimethylformamide (1.1 μL) and oxalyl chloride (15 μL), and the mixture was stirred at room temperature for 30 minutes. The reaction mixture was distilled off under reduced pressure to afford a white solid.

Step 2

To a solution of 5-chloro-6-(2H-1,2,3-triazol-2-yl)-1H-pyrrolo[2,3-b]pyridine (30 mg) in 1,2-dichloroethane (2.5 mL) was added aluminum trichloride (91 mg), and the mixture was stirred at room temperature for 10 minutes. Subsequently, a suspension of the white solid obtained in Step 1 in 1,2-dichloroethane (2.5 mL) was added dropwise to the reaction mixture, and the mixture was stirred at an external temperature of 50°C for 2 hours. After ice-cooling the reaction mixture, the mixture was diluted with water and subjected to extraction with ethyl acetate. The organic phase was washed with saturated brine and dried over magnesium sulfate, and the solvent was then distilled off under reduced pressure. The resulting residue was purified by column chromatography to afford 42 mg of the target compound as a white solid.

Reference Example 41: Synthesis of (2RS,4RS)-1-(4-fluoro-2-nitrophenyl)-2-methylpiperidine-4-carboxylic acid

To a solution of ethyl (2RS,4RS)-2-methylpiperidine-4-carboxylate (200 mg) in dimethyl sulfoxide (2.0 mL) were sequentially added DIPEA (0.40 mL) and 1,4-difluoro-2-nitrobenzene (270 mg), and the mixture was stirred at an external temperature of 50°C for 60 minutes under an argon atmosphere. Furthermore, the mixture was stirred at an external temperature of 120°C for 6 hours. After allowing the reaction mixture to cool, the mixture was diluted with water and subjected to extraction with ethyl acetate. The organic phase was washed with saturated brine and dried over sodium sulfate, and the solvent was distilled off under reduced pressure. The resulting residue was purified by column chromatography to afford 240 mg of the target compound as a yellow oil. To a mixed solution of the resulting yellow oil in tetrahydrofuran (2.5 mL) and water (2.5 mL) was added lithium hydroxide monohydrate (82 mg), and the mixture was stirred at room temperature for 3 hours. Lithium hydroxide monohydrate (65 mg) was added to the reaction mixture, and the mixture was stirred at room temperature for 1 hour. Lithium hydroxide monohydrate (82 mg) was added to the reaction mixture, and the mixture was stirred at room temperature for 2 hours. Lithium hydroxide monohydrate (98 mg) was added to the reaction mixture, and the mixture was stirred at room temperature overnight. To the reaction mixture was added an aqueous hydrochloric acid solution (1.0 M) to adjust the pH to 3. The mixture subjected to extraction with ethyl acetate, the organic phase was washed with saturated brine and dried over sodium sulfate, and the solvent was then distilled off under reduced pressure to afford 210 mg of the target compound as a yellow solid.

Reference Example 42: Synthesis of [5-chloro-6-(2H-1,2,3-triazol-2-yl)-1H-pyrrolo[2,3-b]pyridin-3-yl][(2RS,4RS)-1-(4-fluoro-2-nitrophenyl)-2-methylpiperidin-4-yl]methanone

Step 1

To a solution of (2RS,4RS)-1-(4-fluoro-2-nitrophenyl)-2-methylpiperidine-4-carboxylic acid (100 mg) in dichloromethane (2.0 mL) were added N,N-dimethylformamide (2.7 μL) and oxalyl chloride (37 μL), and the mixture was stirred at room temperature for 30 minutes. The reaction mixture was distilled off under reduced pressure to give a residue.

Step 2

To a solution of 5-chloro-6-(2H-1,2,3-triazol-2-yl)-1H-pyrrolo[2,3-b]pyridine (30 mg) in 1,2-dichloroethane (1.0 mL) was added aluminum trichloride (180 mg), and the mixture was stirred at room temperature for 10 minutes. Subsequently, to the reaction mixture was added dropwise a solution of the residue obtained in Step 1 in 1,2-dichloroethane (3.0 mL), and the mixture was stirred at an external temperature of 50°C for 2 hours under an argon atmosphere. After ice-cooling the reaction mixture, the mixture was diluted with water and subjected to extraction with ethyl acetate. The organic phase was washed with saturated brine and dried over sodium sulfate, and the solvent was distilled off under reduced pressure. The resulting residue was purified by column chromatography to give a crude residue. The resulting residue was slurry-washed with a mixed solvent of ethyl acetate and hexane to afford 71 mg of the target compound as a yellow solid.

Reference Example 43: Synthesis of [5-chloro-1-methyl-6-(2H-1,2,3-triazol-2-yl)-1H-pyrrolo[2,3-b]pyridin-3-yl][(2RS,4RS)-1-(4-fluoro-2-nitrophenyl)-2-methylpiperidin-4-yl]methanone

To a solution of [5-chloro-6-(2H-1,2,3-triazol-2-yl)-1H-pyrrolo[2,3-b]pyridin-3-yl][(2RS,4RS)-1-(4-fluoro-2-nitrophenyl)-2-methylpiperidin-4-yl]methanone (30 mg) in N,N-dimethylformamide (0.50 mL) were sequentially added potassium carbonate (25 mg) and methyl iodide (11 μL), and the mixture was stirred at an external temperature of 50°C for 90 minutes under an argon atmosphere. After allowing the reaction mixture to cool, the mixture was directly purified by reverse-phase column chromatography ($H_2O$/MeOH) to afford 27 mg of the target compound as a yellow solid.

Reference Example 44: Synthesis of methyl 5-chloro-1H-pyrrolo[2,3-b]pyridine-6-carboxylate

Step 1

To a solution of 5,6-dichloro-1H-pyrrolo[2,3-b]pyridine (1.3 g) in N,N-dimethylformamide (29 mL) was added zinc cyanide (1.3 g), and the mixture was stirred at an external temperature of 100°C for 6 hours under an argon atmosphere. After allowing the reaction mixture to cool, the mixture was directly purified by column chromatography to give a crude residue. The resulting residue was slurry-washed with a mixed solvent of diethyl ether and hexane to afford 950 mg as a white solid.

Step 2

To a solution of the white solid (610 mg) obtained in Step 1 in ethanol (30 mL) was added an aqueous sodium hydroxide solution (2.0 M, 30 mL), and the mixture was stirred under heating and reflux at an external temperature of 100°C overnight. After ice-cooling the reaction mixture, an aqueous hydrochloric acid solution (1.0 M) was added to adjust the pH to 3, and the resulting precipitated solid was collected by filtration to afford a white solid (710 mg).

Step 3

To a solution of the white solid (500 mg) obtained in Step 2 in methanol (25 mL) was added a 10% trimethylsilyldiazomethane solution in hexane (7.9 mL) dropwise over 5 minutes, and the mixture was stirred at room temperature for 4 hours. To the reaction mixture was added 10% trimethylsilyldiazomethane (5.3 mL), and the mixture was stirred at room temperature overnight. To the reaction mixture was added acetic acid (0.72 mL), and the mixture was stirred at room temperature for 30 minutes. After adding a saturated aqueous sodium bicarbonate solution, the mixture was extracted with ethyl acetate. The organic phase was washed with saturated brine and dried over magnesium sulfate, and the solvent was then distilled off under reduced pressure. The resulting residue was purified by column chromatography to afford 310 mg of the target compound as a white solid.

Reference Example 45: Synthesis of (2RS,4RS)-1-(3-chloro-5-fluoropyridin-2-yl)-2-methylpiperidine-4-carboxylic acid

Step 1

To a solution of ethyl (2RS,4RS)-2-methylpiperidine-4-carboxylate (3000 mg) in dimethyl sulfoxide (35 mL) were sequentially added DIPEA (6.0 mL) and 3-chloro-2,5-difluoropyridine (5.5 mL), and the mixture was stirred at an external temperature of 140°C for 18 hours. After allowing the reaction mixture to cool, the mixture was diluted with water and extracted with ethyl acetate. The organic phase was washed with saturated brine and dried over magnesium sulfate, and the solvent was then distilled off under reduced pressure. The resulting residue was purified by column chromatography to afford 2.9 g of the ethyl ester as a light yellow oil.

Step 2

To a mixed solution of the light yellow oil (2.9 g) obtained in Step 1 in tetrahydrofuran (30 mL) and water (30 mL) was added lithium hydroxide monohydrate (1.2 g), and the mixture was stirred at room temperature for 2 hours. The reaction mixture was adjusted to pH 3 by adding an aqueous hydrochloric acid solution (1.0 M). Extraction was carried out with ethyl acetate, the organic phase was washed with saturated brine and dried over magnesium sulfate, and the solvent was then distilled off under reduced pressure. The resulting residue was slurry-washed with a mixed solvent of diethyl ether and hexane to afford 1.6 g of the target compound as a white solid.

Reference Example 46: Synthesis of 5-chloro-3-[(2RS,4RS)-1-(3-chloro-5-fluoropyridin-2-yl)-2-methylpiperidine-4-carbonyl]-1H-pyrrolo[2,3-b]pyridine-6-carboxylic acid (cis-racemate)

Step 1

To a solution of (2RS,4RS)-1-(3-chloro-5-fluoropyridin-2-yl)-2-methylpiperidine-4-carboxylic acid (280 mg) in dichloromethane (10 mL) were added N,N-dimethylformamide (7.9 μL) and oxalyl chloride (130 μL), and the mixture was stirred at room temperature for 30 minutes. The reaction mixture was distilled off under reduced pressure to afford a light brown oil.

Step 2

To a solution of methyl 5-chloro-1H-pyrrolo[2,3-b]pyridine-6-carboxylate (180 mg) in 1,2-dichloroethane (10 mL) was added aluminum trichloride (560 mg), and the mixture was stirred at room temperature for 10 minutes. Subsequently, a solution of the light brown oil obtained in Step 1 in 1,2-dichloroethane (10 mL) was added dropwise to the reaction mixture, and the mixture was stirred at an external temperature of 70°C for 2 hours. After ice-cooling the reaction mixture, the mixture was diluted with water, and an aqueous hydrochloric acid solution (1.0 M) was added to adjust the pH to 3. The mixture was extracted with ethyl acetate. The organic phase was washed with saturated brine and dried over magnesium sulfate, and the solvent was then distilled off under reduced pressure. The resulting residue was purified by column chromatography to afford 110 mg of the target compound as a white solid.

Reference Example 47: Synthesis of tert-butyl 4-{5-chloro-3-[(2RS,4RS)-1-(3-chloro-5-fluoropyridin-2-yl)-2-methylpiperidine-4-carbonyl]-1H-pyrrolo[2,3-b]pyridine-6-carbonyl}piperazine-1-carboxylate

To a solution of 5-chloro-3-[(2RS,4RS)-1-(3-chloro-5-fluoropyridin-2-yl)-2-methylpiperidine-4-carbonyl]-1H-pyrrolo[2,3-b]pyridine-6-carboxylic acid (40 mg) in N,N-dimethylformamide (0.88 mL) were added O-(7-azabenzotriazol-1-yl)-N,N,N',N'-tetramethyluronium hexafluorophosphate (HATU) (100 mg), DIPEA (76 μL), and tert-butyl piperazine-1-carboxylate (21 mg), and the mixture was stirred at room temperature for 3 hours. A small amount of methanol was added to the reaction mixture, and the mixture was purified by reverse-phase column chromatography ($H_2O$/MeOH) to afford 41 mg of the target compound as a white solid.

Reference Example 48: Synthesis of (6-bromo-5-chloro-1H-pyrrolo[2,3-b]pyridin-3-yl)[(2RS,4RS)-1-(3-chloro-5-fluoropyridin-2-yl)-2-methylpiperidin-4-yl]methanone

Step 1

To a solution of (2RS,4RS)-1-(3-chloro-5-fluoropyridin-2-yl)-2-methylpiperidine-4-carboxylic acid (cis-racemate) (250 mg) in dichloromethane (10 mL) were added N,N-dimethylformamide (7.1 μL) and oxalyl chloride (110 μL), and the mixture was stirred at room temperature for 30 minutes. The reaction mixture was distilled off under reduced pressure to afford a light brown oil.

Step 2

To a solution of 6-bromo-5-chloro-1H-pyrrolo[2,3-b]pyridine (170 mg) in 1,2-dichloroethane (10 mL) was added aluminum trichloride (500 mg), and the mixture was stirred at room temperature for 10 minutes. Subsequently, a solution of the light brown oil obtained in Step 1 in 1,2-dichloroethane (10 mL) was added dropwise to the reaction mixture, and the mixture was stirred at an external temperature of 50°C for 2 hours. After ice-cooling the reaction mixture, the mixture was diluted with water and subjected to extraction with ethyl acetate. The organic phase was washed with saturated brine and dried over magnesium sulfate, and the solvent was then distilled off under reduced pressure. The resulting residue was purified by column chromatography to afford 210 mg of the target compound as a white solid.

Reference Example 49: Synthesis of [6-(benzylsulfanyl)-5-chloro-1-methyl-1H-pyrrolo[2,3-b]pyridin-3-yl][(2RS,4RS)-1-(3-chloro-5-fluoropyridin-2-yl)-2-methylpiperidin-4-yl]methanone

To a solution of (6-bromo-5-chloro-1H-pyrrolo[2,3-b]pyridin-3-yl)[(2RS,4RS)-1-(3-chloro-5-fluoropyridin-2-yl)-2-methylpiperidin-4-yl]methanone (64 mg) in 1,4-dioxane (2.6 mL) were sequentially added 4,5-bis(diphenylphosphino)-9,9-dimethylxanthene (15 mg), DIPEA (91 μL), benzyl mercaptan (31 μL), and tris(dibenzylideneacetone)(chloroform)dipalladium (12 mg), and the mixture was stirred under an argon atmosphere by microwave irradiation at 110°C for 30 minutes. The reaction mixture was directly purified by column chromatography to afford 68 mg of the target compound as a yellow solid. To a solution of the resulting yellow solid in N,N-dimethylformamide (1.2 mL) were sequentially added potassium carbonate (53 mg) and methyl iodide (23 μL), and the mixture was stirred at room temperature for 40 minutes. The reaction mixture was directly purified by column chromatography to afford 71 mg of the target compound as an orange oil.

Reference Example 50: Synthesis of tert-butyl 4-{5-chloro-3-[(2RS,4RS)-1-(3-chloro-5-fluoropyridin-2-yl)-2-methylpiperidine-4-carbonyl]-1-methyl-1H-pyrrolo[2,3-b]pyridine-6-sulfonyl}piperazine-1-carboxylate

To a mixed solvent of [6-(benzylsulfanyl)-5-chloro-1-methyl-1H-pyrrolo[2,3-b]pyridin-3-yl][(2RS,4RS)-1-(3-chloro-5-fluoropyridin-2-yl)-2-methylpiperidin-4-yl]methanone (70 mg) in acetic acid (1.8 mL) and water (0.2 mL) was added N-chlorosuccinimide (51 mg), and the mixture was stirred at room temperature for 1 hour. Furthermore, the mixture was stirred at an external temperature of 40°C for 2 hours. After the reaction mixture was distilled off under reduced pressure, the resulting residue was treated with an ice-cooled saturated aqueous sodium bicarbonate solution, followed by extraction with ethyl acetate. After the organic phase was washed with saturated brine and dried over magnesium sulfate, the solvent was distilled off under reduced pressure to afford a brownish oil. To a solution of the resulting brownish oil in tetrahydrofuran (1.0 mL) was added tert-butyl piperazine-1-carboxylate (47 mg), and the mixture was stirred at room temperature for 30 minutes. The reaction mixture was directly purified by column chromatography to afford 56 mg of the target compound as a white solid.

Example 1: [5-chloro-1-(oxetan-3-yl)-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone

To a solution of [5-chloro-6-(2H-1,2,3-triazol-2-yl)-1H-pyrrolo[2,3-b]pyridin-3-yl] [(2R,6R)-1-(5-fluoro-3-iodopyridin-2-yl)-2,6-dimethylpiperidin-4-yl]methanone (50 mg) in N,N-dimethylformamide (0.50 mL) were sequentially added potassium carbonate (30 mg) and 3-iodooxetane (0.12 g), and the mixture was stirred at an external temperature of 100°C for 10 hours. After allowing the reaction mixture to cool, the mixture was directly purified by reverse-phase column chromatography ($H_2O$/MeOH) to afford 44 mg of the target compound as a white solid. MS(ESI+)m/z 636.1(M+1)

Example 2: [5-chloro-1-methyl-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone

To a solution of [5-chloro-6-(2H-1,2,3-triazol-2-yl)-1H-pyrrolo[2,3-b]pyridin-3-yl] [(2R,6R)-1-(5-fluoro-3-iodopyridin-2-yl)-2,6-dimethylpiperidin-4-yl]methanone (20 mg) in N,N-dimethylformamide (0.50 mL) were sequentially added potassium carbonate (14 mg) and methyl iodide (6.4 μL), and the mixture was stirred at an external temperature of 50°C for 30 minutes. After allowing the reaction mixture to cool, the mixture was directly purified by reverse-phase column chromatography ($H_2O$/MeOH) to afford 19 mg of the target compound as a white solid. MS(ESI+)m/z 594.0(M+1)

$$\text{Specific optical rotation } [\alpha]_D^{21} = +63.80° \text{ (c = 1.0, chloroform)}$$

Example 3: {5-chloro-3-[(2R,6R)-1-(5-fluoro-3-iodopyridin-2-yl)-2,6-dimethylpiperidin-4-carbonyl]-6-(2H-1,2,3-triazol-2-yl)-1H-pyrrolo[2,3-b]pyridin-1-yl}acetonitrile

To a solution of [5-chloro-6-(2H-1,2,3-triazol-2-yl)-1H-pyrrolo[2,3-b]pyridin-3-yl] [(2R,6R)-1-(5-fluoro-3-iodopyridin-2-yl)-2,6-dimethylpiperidin-4-yl]methanone (30 mg) in N,N-dimethylformamide (0.50 mL) were sequentially added potassium carbonate (21 mg) and 2-bromoacetonitrile (19 mg), and the mixture was stirred at an external temperature of 50°C for 30 minutes. After allowing the reaction mixture to cool, the mixture was directly purified by reverse-phase column chromatography ($H_2O$/MeOH) to afford 32 mg of the target compound as a light brown solid. MS(ESI+)m/z 619.1(M+1)

Example 4: [5-chloro-1-(2-hydroxyethyl)-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone

To a mixed solution of [1-(2-{[tert-butyl(dimethyl)silyl]oxy}ethyl)-5-chloro-6-(2H-1,2,3-triazol-2-yl)-1H-pyrrolo [2,3-b]pyridin-3-yl] [(2R,6R)-1-(5-fluoro-3-iodopyridin-2-yl)-2,6-dimethylpiperidin-4-yl]methanone (42 mg) in methanol (2.5 mL) and dichloromethane (0.50 mL) was added an aqueous hydrochloric acid solution (1.0 M, 0.57 mL), and the mixture was stirred at room temperature for 12 hours. After the reaction mixture was neutralized with an aqueous sodium hydroxide solution (1.0 M), the mixture was diluted with water and subjected to extraction with ethyl acetate. The organic phase was washed with saturated brine and dried over magnesium sulfate, and the solvent was then distilled off under reduced pressure. The resulting residue was purified by column chromatography to afford 31 mg of the target compound as a white solid. MS(ESI+)m/z 624.0(M+1)

Example 5: 1-(3-{5-chloro-3-[(2R,6R)-1-(5-fluoro-3-iodopyridin-2-yl)-2,6-dimethylpiperidin-4-carbonyl]-6-(2H-1,2,3-triazol-2-yl)-1H-pyrrolo[2,3-b]pyridin-1-yl}azetidin-1-yl)ethan-1-one

Step 1: To a solution of tert-butyl 3-{5-chloro-3-[(2R,6R)-1-(5-fluoro-3-iodopyridin-2-yl)-2,6-dimethylpiperidin-4-carbonyl]-6-(2H-1,2,3-triazol-2-yl)-1H-pyrrolo[2,3-b]pyridin-1-yl}azetidine-1-carboxylate (59 mg) in dichloromethane (1.0

mL) was added trifluoroacetic acid (0.50 mL), and the mixture was stirred at room temperature for 1 hour. After diluting the reaction mixture with ethyl acetate, the mixture was neutralized with an aqueous sodium hydroxide solution (2.0 M) to adjust the pH to 8. The reaction mixture was diluted with water and subjected to extraction with ethyl acetate. After the organic phase was washed with saturated brine and dried over magnesium sulfate, the solvent was distilled off under reduced pressure to afford the residue (77 mg) as a light yellow solid.

Step 2: To a solution of the residue (77 mg) obtained in Step 1 in tetrahydrofuran (1.5 mL) were sequentially added DIPEA (69 μL) and acetyl chloride (7.1 μL), and the mixture was stirred at room temperature for 2 hours. After adding a saturated aqueous sodium bicarbonate solution to the reaction mixture, the mixture was diluted with water and subjected to extraction with ethyl acetate. The organic phase was washed with saturated brine and dried over magnesium sulfate, and the solvent was then distilled off under reduced pressure. The resulting residue was purified by column chromatography to afford 40 mg of the target compound as a light brown solid. MS(ESI+)m/z 677.2(M+1)

Example 6: [5-chloro-1-methyl-6-(2H-1,2,3-triazol-2-yl)-1H-pyrrolo[2,3-b]pyridin-3-yl][1-(8-fluoroisoquinolin-4-yl)-5-(trifluoromethyl)-1H-pyrazol-4-yl]methanone

To a solution of [5-chloro-6-(2H-1,2,3-triazol-2-yl)-1H-pyrrolo[2,3-b]pyridin-3-yl][1-(8-fluoroisoquinolin-4-yl)-5-(trifluoromethyl)-1H-pyrazol-4-yl]methanone (40 mg) in N,N-dimethylformamide (0.50 mL) were sequentially added potassium carbonate (31 mg) and methyl iodide (14 μL), and the mixture was stirred at an external temperature of 50°C for 30 minutes. After allowing the reaction mixture to cool, the mixture was diluted with water, and the resulting precipitated solid was collected by filtration to obtain a light brown residue. The resulting residue was purified by column chromatography, followed by purification by reverse-phase column chromatography ($H_2O$/MeOH), to afford 21 mg of the target compound as a white solid. MS(ESI+)m/z 541.1(M+1)

Example 7: [5-chloro-1-ethyl-6-(2H-1,2,3-triazol-2-yl)-1H-pyrrolo[2,3-b]pyridin-3-yl][1-(8-fluoroisoquinolin-4-yl)-5-(trifluoromethyl)-1H-pyrazol-4-yl]methanone

To a solution of [5-chloro-6-(2H-1,2,3-triazol-2-yl)-1H-pyrrolo[2,3-b]pyridin-3-yl][1-(8-fluoroisoquinolin-4-yl)-5-(trifluoromethyl)-1H-pyrazol-4-yl]methanone (35 mg) in N,N-dimethylformamide (0.50 mL) were sequentially added potassium carbonate (28 mg) and ethyl iodide (16 μL), and the mixture was stirred at an external temperature of 50°C for 30 minutes. After allowing the reaction mixture to cool, the mixture was directly purified by reverse-phase column chromatography ($H_2O$/MeOH) to give a crude residue. The resulting residue was slurry-washed with methanol to afford 37 mg of the target compound as a white solid. MS 555.1(M+1)

Example 8: [5-chloro-1-propyl-6-(2H-1,2,3-triazol-2-yl)-1H-pyrrolo[2,3-b]pyridin-3-yl][1-(8-fluoroisoquinolin-4-yl)-5-(trifluoromethyl)-1H-pyrazol-4-yl]methanone

To a solution of [5-chloro-6-(2H-1,2,3-triazol-2-yl)-1H-pyrrolo[2,3-b]pyridin-3-yl][1-(8-fluoroisoquinolin-4-yl)-5-(trifluoromethyl)-1H-pyrazol-4-yl]methanone (35 mg) in N,N-dimethylformamide (0.50 mL) were sequentially added potassium carbonate (28 mg) and 1-bromopropane (18 μL), and the mixture was stirred at an external temperature of 50°C for 2 hours. After allowing the reaction mixture to cool, the mixture was directly purified by reverse-phase column chromatography ($H_2O$/MeOH) to afford 32 mg of the target compound as a white solid. MS(ESI+)m/z 569.1(M+1)

Example 9: [5-chloro-1-(3-hydroxypropyl)-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone

To a mixed solution of [1-(3-{[tert-butyl(dimethyl)silyl]oxy}propyl)-5-chloro-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone (47 mg) in tetrahydrofuran (1.0 mL) and methanol (1.0 mL) was added an aqueous hydrochloric acid solution (1.0 M, 1.0 mL), and the mixture was stirred at room temperature for 3 days. The reaction mixture was neutralized with a saturated aqueous sodium bicarbonate solution, and then subjected to extraction with ethyl acetate. The organic phase was washed with saturated brine and dried over magnesium sulfate, and the solvent was then distilled off under reduced pressure. The resulting residue was purified by reverse-phase column chromatography ($H_2O$/MeOH) to give a crude residue. The resulting residue was recrystallized from ethyl acetate/n-hexane to afford 26 mg of the target compound as a light yellow solid. MS(ESI+)m/z 649.2(M+1)

$$\text{Specific optical rotation } [\alpha]_D^{20} = +30.36° \text{ (c = 1.0, chloroform)}$$

Example 10: [5-chloro-1-(oxetan-3-yl)-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone

To a solution of [5-chloro-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone (30 mg) in N,N-dimethylformamide (1.0 mL) were sequentially added potassium carbonate (18 mg) and 3-iodooxetane (75 mg), and the mixture was stirred at an external temperature of 100°C for 11 hours. After allowing the reaction mixture to cool, the mixture was directly purified by reverse-phase column chromatography ($H_2O$/MeOH), followed by further purification by column chromatography to give a crude residue. The resulting residue was recrystallized from methanol/water to afford 18 mg of the target compound as a light yellow solid. MS(ESI+) m/z 647.2(M+1)

Example 11: [5-chloro-1-methyl-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone

To a solution of [5-chloro-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone (22 mg) in N,N-dimethylformamide (0.50 mL) were sequentially added potassium carbonate (26 mg) and methyl iodide (7.0 μL), and the mixture was stirred at room temperature for 1 hour. The reaction mixture was directly purified by reverse-phase column chromatography ($H_2O$/MeOH), followed by further purification by column chromatography to afford 15 mg of the target compound as a white solid. MS(ESI+)m/z 605.0(M+1)

Example 12: [5-chloro-1-methyl-6-(pyrimidin-2-yl)-1H-pyrrolo[2,3-b]pyridin-3-yl][1-(8-fluoroisoquinolin-4-yl)-5-(trifluoromethyl)-1H-pyrazol-4-yl]methanone

To a solution of [5-chloro-6-(pyrimidin-2-yl)-1H-pyrrolo[2,3-b]pyridin-3-yl][1-(8-fluoroisoquinolin-4-yl)-5-(trifluoromethyl)-1H-pyrazol-4-yl]methanone (40 mg) in N,N-dimethylformamide (1.0 mL) were sequentially added potassium carbonate (51 mg) and methyl iodide (14 μL), and the mixture was stirred at room temperature for 1 hour. The reaction mixture was directly purified by reverse-phase column chromatography ($H_2O$/MeOH) to give a crude residue. The resulting residue was recrystallized from ethyl acetate/n-hexane to afford 31 mg of the target compound as a light yellow solid. MS(ESI+)m/z 552.1(M+1)

Example 13: [5-chloro-1-ethyl-6-(pyrimidin-2-yl)-1H-pyrrolo[2,3-b]pyridin-3-yl][1-(8-fluoroisoquinolin-4-yl)-5-(trifluoromethyl)-1H-pyrazol-4-yl]methanone

To a solution of [5-chloro-6-(pyrimidin-2-yl)-1H-pyrrolo[2,3-b]pyridin-3-yl][1-(8-fluoroisoquinolin-4-yl)-5-(trifluoromethyl)-1H-pyrazol-4-yl]methanone (40 mg) in N,N-dimethylformamide (1.0 mL) were sequentially added potassium carbonate (51 mg) and ethyl iodide (18 μL), and the mixture was stirred at room temperature for 1 hour. The reaction mixture was directly purified by reverse-phase column chromatography ($H_2O$/MeOH) to obtain a crude residue (25 mg). The resulting residue (25 mg) was recrystallized from ethyl acetate/n-hexane to afford 19 mg of the target compound as a light yellow solid. MS(ESI+)m/z 566.2(M+1)

Example 14: [5-chloro-1-ethyl-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone

To a solution of [5-chloro-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone (30 mg) in N,N-dimethylformamide (1.0 mL) were sequentially added potassium carbonate (36 mg) and ethyl iodide (12 μL), and the mixture was stirred at room temperature for 2 hours. The reaction mixture was directly purified by reverse-phase column chromatography, followed by further purification by column chromatography to afford 30 mg of the target compound as a white solid. MS(ESI+)m/z 608.2(M+1)

Example 15: [5-chloro-1-(3-hydroxypropyl)-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone

Step 1: To a solution of [5-chloro-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone (25 mg) in N,N-dimethylformamide (1.0 mL) were sequentially added potassium carbonate (18 mg) and (3-bromopropoxy)(tert-butyl)dimethylsilane (33 mg), and the mixture was stirred at an external temperature of 50°C overnight. After allowing the reaction mixture to cool, the mixture was directly purified by reverse-phase column chromatography ($H_2O$/MeOH) to afford 33 mg of a white solid.

Step 2: The white solid (33 mg) obtained in Step 1 was dissolved in a mixed solution of tetrahydrofuran (2.0 mL)

and methanol (2.0 mL), an aqueous hydrochloric acid solution (1.0 M, 1.0 mL) was added, and the mixture was stirred at room temperature overnight. The reaction mixture was neutralized with a saturated aqueous sodium bicarbonate solution, followed by extraction with ethyl acetate. The organic phase was dried over magnesium sulfate, and the solvent was then distilled off under reduced pressure. The resulting residue was purified by column chromatography to give a crude residue. The resulting residue was recrystallized from ethyl acetate/n-hexane to afford 26 mg of the target compound as a white solid. MS(ESI+)m/z 638.2(M+1)

Example 16: [5-chloro-1-cyclopropyl-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone

To a solution of [5-chloro-6-(2H-1,2,3-triazol-2-yl)-1H-pyrrolo[2,3-b]pyridin-3-yl] [(2R,6R)-1-(5-fluoro-3-iodopyridin-2-yl)-2,6-dimethylpiperidin-4-yl]methanone (40 mg) in 1,2-dichloroethane (1.0 mL) were added cyclopropylboronic acid (12 mg), 2,2'-bipyridine (16 mg), copper(II) acetate (19 mg), and sodium carbonate (15 mg), and the mixture was stirred at an external temperature of 70°C for 5 hours. After allowing the reaction mixture to cool, the mixture was directly purified by column chromatography, followed by further purification by reverse-phase column chromatography ($H_2O$/MeOH) to give a crude residue. The resulting residue was recrystallized from ethyl acetate/n-hexane to afford 24 mg of the target compound as a white solid. MS(ESI+)m/z 620.0(M+1)

Example 17: [5-chloro-1-isopropyl-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone

To a solution of [5-chloro-6-(2H-1,2,3-triazol-2-yl)-1H-pyrrolo[2,3-b]pyridin-3-yl] [(2R,6R)-1-(5-fluoro-3-iodopyridin-2-yl)-2,6-dimethylpiperidin-4-yl]methanone (19 mg) in N,N-dimethylformamide (1.0 mL) were sequentially added potassium carbonate (22 mg) and 2-bromopropane (9.0 μL), and the mixture was stirred at an external temperature of 100°C for 1.5 hours. After allowing the reaction mixture to cool, the mixture was directly purified by reverse-phase column chromatography ($H_2O$/MeOH) to afford 18 mg of the target compound as a white solid. MS(ESI+)m/z 622.1(M+1)

Example 18: [5-chloro-1-ethyl-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone

To a solution of [5-chloro-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone (42 mg) in N,N-dimethylformamide (1.0 mL) were sequentially added potassium carbonate (49 mg) and ethyl iodide (17 μL), and the mixture was stirred at room temperature for 2 hours. The reaction mixture was directly purified by reverse-phase column chromatography ($H_2O$/MeOH), followed by further purification by column chromatography to afford 39 mg of the target compound as a white solid. MS(ESI+)m/z 619.1(M+1)

Example 19: [5-chloro-1-(1,1-dioxotetrahydro-2H-thiopyran-4-yl)-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone

To a solution of [5-chloro-6-(2H-1,2,3-triazol-2-yl)-1H-pyrrolo[2,3-b]pyridin-3-yl] [(2R,6R)-1-(5-fluoro-3-iodopyridin-2-yl)-2,6-dimethylpiperidin-4-yl]methanone (30 mg) in N,N-dimethylformamide (1.0 mL) were sequentially added potassium carbonate (36 mg) and 1,1-dioxotetrahydro-2H-thiopyran-4-yl methanesulfonate (0.12 g), and the mixture was stirred at an external temperature of 100°C for 16 hours. After allowing the reaction mixture to cool, the mixture was directly purified by reverse-phase column chromatography ($H_2O$/MeOH) to give a crude residue. The resulting residue was purified by column chromatography, followed by reverse-phase column chromatography ($H_2O$/MeOH), to afford 11 mg of the target compound as a white solid. MS(ESI+)m/z 712.3(M+1)

Example 20: Synthesis of [5-chloro-1-(oxan-4-yl)-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone

To a solution of [5-chloro-6-(2H-1,2,3-triazol-2-yl)-1H-pyrrolo[2,3-b]pyridin-3-yl] [(2R,6R)-1-(5-fluoro-3-iodopyridin-2-yl)-2,6-dimethylpiperidin-4-yl]methanone (36 mg) in N,N-dimethylformamide (0.80 mL) were sequentially added potassium carbonate (42 mg) and oxan-4-yl methanesulfonate (0.11 g), and the mixture was stirred at an external temperature of 100°C for 12 hours. After allowing the reaction mixture to cool, the mixture was directly purified by column chromatography to give a crude residue. The resulting residue was purified by reverse-phase column chromatography ($H_2O$/MeOH) to afford 24 mg of the target compound as a light yellow solid. MS(ESI+)m/z 664.2(M+1)

Example 21: Synthesis of {5-chloro-1-[(3S)-oxolan-3-yl]-6-(2H-1,2,3-triazol-2-yl)-1H-pyrrolo[2,3-b]pyridin-3-yl} [(2R,6R)-1-(5-fluoro-3-iodopyridin-2-yl)-2,6-dimethylpiperidin-4-yl]methanone

To a solution of [5-chloro-6-(2H-1,2,3-triazol-2-yl)-1H-pyrrolo[2,3-b]pyridin-3-yl] [(2R,6R)-1-(5-fluoro-3-iodopyridin-2-yl)-2,6-dimethylpiperidin-4-yl]methanone (36 mg) in N,N-dimethylformamide (0.80 mL) were sequentially added potassium carbonate (42 mg) and (3R)-oxolan-3-yl methanesulfonate (0.10 g), and the mixture was stirred at an external temperature of 100°C for 12 hours. After allowing the reaction mixture to cool, the mixture was directly purified by column chromatography to give a crude residue. The resulting residue was purified by reverse-phase column chromatography ($H_2O$/MeOH) to afford 30 mg of the target compound as a white solid. MS(ESI+)m/z 650.2(M+1)

Example 22: Synthesis of {5-chloro-1-[(3R)-oxolan-3-yl]-6-(2H-1,2,3-triazol-2-yl)-1H-pyrrolo[2,3-b]pyridin-3-yl} [(2R,6R)-1-(5-fluoro-3-iodopyridin-2-yl)-2,6-dimethylpiperidin-4-yl]methanone

To a solution of [5-chloro-6-(2H-1,2,3-triazol-2-yl)-1H-pyrrolo[2,3-b]pyridin-3-yl] [(2R,6R)-1-(5-fluoro-3-iodopyridin-2-yl)-2,6-dimethylpiperidin-4-yl]methanone (36 mg) in N,N-dimethylformamide (0.80 mL) were sequentially added potassium carbonate (42 mg) and (3S)-oxolan-3-yl methanesulfonate (0.10 g), and the mixture was stirred at an external temperature of 100°C for 12 hours. After allowing the reaction mixture to cool, the mixture was directly purified by column chromatography to give a crude residue. The resulting residue was purified by reverse-phase column chromatography ($H_2O$/MeOH) to afford 30 mg of the target compound as a white solid. MS(ESI+)m/z 650.2(M+1)

Example 23: Synthesis of [5-chloro-6-(2H-1,2,3-triazol-2-yl)-1H-pyrrolo[2,3-b]pyridin-3-yl][1-(5-fluoronaphthalen-1-yl)-5-(trifluoromethyl)-1H-pyrazol-4-yl]methanone

Step 1

To a solution of 1-(5-fluoronaphthalen-1-yl)-5-(trifluoromethyl)-1H-pyrazole-4-carboxylic acid (0.055 g) (synthesized according to WO2018/119036) in dichloromethane (2.5 mL) were added N,N-dimethylformamide (1.3 μL) and oxalyl chloride (17 μL), and the mixture was stirred at room temperature for 30 minutes. The reaction mixture was distilled off under reduced pressure to afford a brown oil.

Step 2

To a solution of 5-chloro-6-(2H-1,2,3-triazol-2-yl)-1H-pyrrolo[2,3-b]pyridine (30 mg) in 1,2-dichloroethane (2.5 mL) was added aluminum trichloride (91 mg), and the mixture was stirred at room temperature for 10 minutes. Subsequently, a solution of the brown oil obtained in Step 1 in 1,2-dichloroethane (2.5 mL) was added dropwise to the reaction mixture, and the mixture was stirred at an external temperature of 75°C for 4 hours. After ice-cooling the reaction mixture, the mixture was diluted with water and extracted with ethyl acetate. The organic phase was washed with saturated brine and dried over magnesium sulfate, and the solvent was then distilled off under reduced pressure. The resulting residue was purified by column chromatography to give a crude residue. The resulting residue was slurry-washed with ethyl acetate to afford 0.018 g of the target compound as a tan solid. MS(ESI+)m/z 526.1(M+1)

Example 24: Synthesis of [5-chloro-1-methyl-6-(2H-1,2,3-triazol-2-yl)-1H-pyrrolo[2,3-b]pyridin-3-yl][1-(thieno[2,3-b]pyridin-4-yl)-5-(trifluoromethyl)-1H-pyrazol-4-yl]methanone

To a solution of [5-chloro-6-(2H-1,2,3-triazol-2-yl)-1H-pyrrolo[2,3-b]pyridin-3-yl][1-(thieno[2,3-b]pyridin-4-yl)-5-(trifluoromethyl)-1H-pyrazol-4-yl]methanone (25 mg) in N,N-dimethylformamide (0.50 mL) were sequentially added potassium carbonate (20 mg) and methyl iodide (9.0 μL), and the mixture was stirred at an external temperature of 50°C for 15 minutes. After allowing the reaction mixture to cool, it was diluted with water, and the precipitated solid was collected by filtration to give a crude residue. The resulting residue was slurry-washed with methanol to afford 19 mg of the target compound as a white solid. MS(ESI+)m/z 529.1(M+1)

Example 25: Synthesis of [5-chloro-1-(oxetan-3-yl)-6-(2H-1,2,3-triazol-2-yl)-1H-pyrrolo[2,3-b]pyridin-3-yl][1-(thieno[2,3-c]pyridin-4-yl)-5-(trifluoromethyl)-1H-pyrazol-4-yl]methanone

To a solution of 1-(thieno[2,3-c]pyridin-4-yl)-5-(trifluoromethyl)-1H-pyrazole-4-carboxylic acid (30 mg) in N,N-dimethylformamide (1.0 mL) were sequentially added potassium carbonate (40 mg) and 3-iodooxetane (80 mg), and the mixture was stirred at an external temperature of 100°C for 12 hours, followed by stirring at room temperature over the weekend. After allowing the reaction mixture to cool, the mixture was directly purified by column chromatography to give a

crude residue. The resulting residue was slurry-washed with methanol to afford 24 mg of the target compound as a light yellow solid. MS(ESI+)m/z 571.1(M+1)

Example 26: Synthesis of [5-chloro-1-propyl-6-(pyrimidin-2-yl)-1H-pyrrolo[2,3-b]pyridin-3-yl][1-(8-fluoroisoquinolin-4-yl)-5-(trifluoromethyl)-1H-pyrazol-4-yl]methanone

To a solution of [5-chloro-6-(pyrimidin-2-yl)-1H-pyrrolo[2,3-b]pyridin-3-yl][1-(8-fluoroisoquinolin-4-yl)-5-(trifluoromethyl)-1H-pyrazol-4-yl]methanone (40 mg) in N,N-dimethylformamide (1.0 mL) were sequentially added potassium carbonate (51 mg) and 1-bromopropane (20 μL), and the mixture was stirred at an external temperature of 60°C for 2 hours. After allowing the reaction mixture to cool, the mixture was directly purified by reverse-phase column chromatography ($H_2O$/MeOH) to obtain 35 mg of crude residue. The resulting residue (35 mg) was recrystallized from ethyl acetate/n-hexane to afford 26 mg of the target compound as a light yellow solid. MS(ESI+)m/z 580.2(M+1)

Example 27: Synthesis of [5-chloro-1-methyl-6-(2H-1,2,3-triazol-2-yl)-1H-pyrrolo[2,3-b]pyridin-3-yl][1-(pyrazolo[1,5-a]pyridin-4-yl)-5-(trifluoromethyl)-1H-pyrazol-4-yl]methanone

To a solution of [5-chloro-6-(2H-1,2,3-triazol-2-yl)-1H-pyrrolo[2,3-b]pyridin-3-yl][1-(pyrazolo[1,5-a]pyridin-4-yl)-5-(trifluoromethyl)-1H-pyrazol-4-yl]methanone (25 mg) in N,N-dimethylformamide (0.50 mL) were sequentially added potassium carbonate (20 mg) and methyl iodide (9.3 μL), and the mixture was stirred at an external temperature of 50°C for 15 minutes. After allowing the reaction mixture to cool, the mixture was directly purified by reverse-phase column chromatography ($H_2O$/MeOH) to give a crude residue. The resulting residue was slurry-washed with diethyl ether to afford 15 mg of the target compound as a white solid. MS(ESI+)m/z 512.1(M+1)

Example 28: Synthesis of [1-(1,2,3-benzothiadiazol-7-yl)-5-(trifluoromethyl)-1H-pyrazol-4-yl][5-chloro-1-methyl-6-(2H-1,2,3-triazol-2-yl)-1H-pyrrolo[2,3-b]pyridin-3-yl]methanone

To a solution of [1-(1,2,3-benzothiadiazol-7-yl)-5-(trifluoromethyl)-1H-pyrazol-4-yl][5-chloro-6-(2H-1,2,3-triazol-2-yl)-1H-pyrrolo[2,3-b]pyridin-3-yl]methanone (16 mg) in N,N-dimethylformamide (0.50 mL) were sequentially added potassium carbonate (12 mg) and methyl iodide (5.7 μL), and the mixture was stirred at an external temperature of 50°C for 30 minutes. After allowing the reaction mixture to cool, the mixture was directly purified by reverse-phase column chromatography ($H_2O$/MeOH) to afford 16 mg of the target compound as a light yellow solid. MS(ESI+)m/z 530.1(M+1)

Example 29: Synthesis of [1-(1,2,3-benzothiadiazol-7-yl)-5-(trifluoromethyl)-1H-pyrazol-4-yl][5-chloro-1-methyl-6-(pyrimidin-2-yl)-1H-pyrrolo[2,3-b]pyridin-3-yl]methanone

Step 1

To a solution of 1-(1,2,3-benzothiadiazol-7-yl)-5-(trifluoromethyl)-1H-pyrazole-4-carboxylic acid (43 mg) (synthesized according to WO2018/119036) in dichloromethane (1.0 mL) were added N,N-dimethylformamide (1.0 μL) and oxalyl chloride (14 μL), and the mixture was stirred at room temperature for 30 minutes. The reaction mixture was distilled off under reduced pressure to afford a light brown solid.

Step 2

To a solution of 5-chloro-1-methyl-6-(pyrimidin-2-yl)-1H-pyrrolo[2,3-b]pyridine (30 mg) in 1,2-dichloroethane (1.2 mL) was added aluminum trichloride (81 mg), and the mixture was stirred at room temperature for 10 minutes. Subsequently, a solution of the light brown solid obtained in Step 1 in 1,2-dichloroethane (1.2 mL) was added dropwise to the reaction mixture, and the mixture was stirred under heating and reflux at an external temperature of 110°C for 5 hours. After ice-cooling the reaction mixture, the mixture was diluted with water and subjected to extraction with ethyl acetate. The organic phase was washed with saturated brine and dried over magnesium sulfate, and the solvent was then distilled off under reduced pressure. The resulting residue was purified by column chromatography to give a crude residue. The resulting residue was purified by reverse-phase column chromatography ($H_2O$/MeOH) to give a crude residue. The resulting residue was slurry-washed with isopropanol to afford 33 mg of the target compound as a light yellow solid. MS(ESI+)m/z 541.1(M+1)

Example 30: Synthesis of 5-{4-[5-chloro-1-cyclobutyl-6-(2H-1,2,3-triazol-2-yl)-1H-pyrrolo[2,3-b]pyridine-3-carbonyl]-5-(trifluoromethyl)-1H-pyrazol-1-yl}isoquinolin-1(2H)-one

Step 1

To a solution of [5-chloro-6-(2H-1,2,3-triazol-2-yl)-1H-pyrrolo[2,3-b]pyridin-3-yl][1-(1-methoxyisoquinolin-5-yl)-5-(trifluoromethyl)-1H-pyrazol-4-yl]methanone (15 mg) in N,N-dimethylformamide (0.50 mL) were sequentially added potassium carbonate (11 mg) and bromocyclobutane (7.8 μL), and the mixture was stirred at an external temperature of 100°C for 11 hours. To the reaction mixture were sequentially added potassium carbonate (38 mg) and bromocyclobutane (26 μL), and the mixture was stirred at the same temperature overnight. The mixture was stirred at an external temperature of 110°C for 5 hours. After allowing the reaction mixture to cool, the mixture was directly purified by reverse-phase column chromatography ($H_2O$/MeOH) to obtain a white solid.

Step 2

To a solution of the white solid obtained in Step 1 in dioxane (1.0 mL) was added 4N hydrochloric acid in dioxane (1.0 mL), and the mixture was stirred at an external temperature of 50°C for 5 hours. After allowing the reaction mixture to cool, the solvent was distilled off under reduced pressure. The resulting residue was purified by reverse-phase column chromatography ($H_2O$/MeOH) to afford 17 mg of the target compound as a white solid. MS(ESI+)m/z 579.2(M+1)

Example 31: Synthesis of 9-{4-[5-chloro-1-methyl-6-(2H-1,2,3-triazol-2-yl)-1H-pyrrolo[2,3-b]pyridine-3-carbonyl]-5-(trifluoromethyl)-1H-pyrazol-1-yl}-4H-quinolizin-4-one

To a solution of 9-{4-[5-chloro-6-(2H-1,2,3-triazol-2-yl)-1H-pyrrolo[2,3-b]pyridine-3-carbonyl]-5-(trifluoromethyl)-1H-pyrazol-1-yl}-4H-quinolizin-4-one (35 mg) in N,N-dimethylformamide (0.50 mL) were sequentially added potassium carbonate (27 mg) and methyl iodide (12 μL), and the mixture was stirred at an external temperature of 50°C for 15 minutes. After allowing the reaction mixture to cool, the mixture was directly purified by reverse-phase column chromatography ($H_2O$/MeOH) to give a crude residue. The resulting residue was slurry-washed with ethyl acetate to afford 29 mg of the target compound as a yellow solid. MS(ESI+)m/z 539.1(M+1)

Example 32: Synthesis of (5-chloro-6-methoxy-1-methyl-1H-pyrrolo[2,3-b]pyridin-3-yl)[1-(8-fluoroisoquinolin-4-yl)-5-(trifluoromethyl)-1H-pyrazol-4-yl]methanone

Step 1

To a solution of 1-(8-fluoroisoquinolin-4-yl)-5-(trifluoromethyl)-1H-pyrazole-4-carboxylic acid (35 mg) (synthesized according to WO2018/119036) in dichloromethane (1.0 mL) were added N,N-dimethylformamide (0.83 μL) and oxalyl chloride (11 μL), and the mixture was stirred at room temperature for 30 minutes. The reaction mixture was distilled off under reduced pressure to afford a light yellow solid.

Step 2

To a solution of 5-chloro-6-methoxy-1-methyl-1H-pyrrolo[2,3-b]pyridine (16 mg) in 1,2-dichloroethane (0.5 mL) was added aluminum trichloride (54 mg), and the mixture was stirred at room temperature for 5 minutes. Subsequently, a suspension of the light yellow solid obtained in Step 1 in 1,2-dichloroethane (0.5 mL) was added dropwise to the reaction mixture, followed by washing with 1,2-dichloroethane (0.5 mL), and the mixture was stirred at an external temperature of 75°C for 1 hour. The mixture was further stirred under heating and reflux at an external temperature of 105°C for 3 hours. After ice-cooling the reaction mixture, the mixture was diluted with water and subjected to extraction with ethyl acetate. The organic phase was washed with saturated brine and dried over magnesium sulfate, and the solvent was then distilled off under reduced pressure. The resulting residue was purified by column chromatography to give a crude residue. The resulting residue was purified by reverse-phase column chromatography ($H_2O$/MeOH) to afford 7.8 mg of the target compound as a white solid. MS(ESI+)m/z 503.8(M+1)

Example 33: Synthesis of [(2RS,4RS)-1-(5-bromopyrimidin-4-yl)-2-methylpiperidin-4-yl][5-chloro-1-methyl-6-(2H-1,2,3-triazol-2-yl)-1H-pyrrolo[2,3-b]pyridin-3-yl]methanone

To a solution of (2RS,4RS)-[1-(5-bromopyrimidin-4-yl)-2-methylpiperidin-4-yl][5-chloro-6-(2H-1,2,3-triazol-2-yl)-1H-pyrrolo[2,3-b]pyridin-3-yl]methanone (20 mg), obtained in Reference Example 40, in N,N-dimethylformamide (0.50 mL) were sequentially added potassium carbonate (16 mg) and methyl iodide (3.7 μL), and the mixture was stirred at an external temperature of 50°C for 30 minutes. After allowing the reaction mixture to cool, the mixture was directly purified by reverse-phase column chromatography ($H_2O$/MeOH) to afford 17 mg of the target compound as a white solid.

MS(ESI+)m/z 517.0(M+1)

Example 34: Synthesis of [(2RS,4RS)-1-(2-amino-4-fluorophenyl)-2-methylpiperidin-4-yl][5-chloro-1-methyl-6-(2H-1,2,3-triazol-2-yl)-1H-pyrrolo[2,3-b]pyridin-3-yl]methanone

To a solution of [5-chloro-1-methyl-6-(2H-1,2,3-triazol-2-yl)-1H-pyrrolo[2,3-b]pyridin-3-yl][(2RS,4RS)-1-(4-fluoro-2-nitrophenyl)-2-methylpiperidin-4-yl]methanone (25 mg), obtained in Reference Example 43, in methanol (5.0 mL) were added 1% platinum on activated carbon, Degussa type CF 105R/W (75 mg), and the mixture was stirred at room temperature under a hydrogen atmosphere (4.0 atm) overnight. The reaction mixture was filtered through Celite, and the solvent was distilled off under reduced pressure. The resulting residue was purified by reverse-phase column chromatography ($H_2O$/MeOH) to give a crude residue. The resulting residue was purified by reverse-phase column chromatography ($H_2O$/MeOH) to afford 14 mg of the target compound as a white solid. MS(ESI+)m/z 468.2(M+1)

Example 35: Synthesis of {5-chloro-3-[(2RS,4RS)-1-(3-chloro-5-fluoropyridin-2-yl)-2-methylpiperidine-4-carbonyl]-1H-pyrrolo[2,3-b]pyridin-6-yl}(piperazin-1-yl)methanone

To a solution of tert-butyl 4-{5-chloro-3-[(2RS,4RS)-1-(3-chloro-5-fluoropyridin-2-yl)-2-methylpiperidine-4-carbonyl]-1H-pyrrolo[2,3-b]pyridine-6-carbonyl}piperazine-1-carboxylate (41 mg), obtained in Reference Example 47, in dichloromethane (1.0 mL) was added trifluoroacetic acid (0.50 mL), and the mixture was stirred at room temperature for 2 hours. After the addition of a saturated aqueous sodium bicarbonate solution to the reaction mixture, the mixture was extracted with ethyl acetate. The organic layer was washed with saturated brine and dried over magnesium sulfate, and the solvent was then distilled off under reduced pressure. The resulting residue was purified by reverse-phase column chromatography ($H_2O$/MeOH) to afford 25 mg of the target compound as a beige solid. MS(ESI+)m/z 519.2(M+1)

Example 36: Synthesis of [(2RS,4RS)-1-(3-chloro-5-fluoropyridin-2-yl)-2-methylpiperidin-4-yl][5-chloro-1-methyl-6-(piperazin-1-ylsulfonyl)-1H-pyrrolo[2,3-b]pyridin-3-yl]methanone

To a solution of tert-butyl 4-{5-chloro-3-[(2RS,4RS)-1-(3-chloro-5-fluoropyridin-2-yl)-2-methylpiperidine-4-carbonyl]-1-methyl-1H-pyrrolo[2,3-b]pyridine-6-sulfonyl}piperazine-1-carboxylate (56 mg), obtained in Reference Example 50, in dichloromethane (2.0 mL) was added trifluoroacetic acid (1.0 mL), and the mixture was stirred at room temperature for 2 hours. After the addition of a saturated aqueous sodium bicarbonate solution to the reaction mixture, the mixture was extracted with ethyl acetate. The organic phase was washed with saturated brine and dried over magnesium sulfate, and the solvent was then distilled off under reduced pressure. The resulting residue was purified by reverse-phase column chromatography ($H_2O$/MeOH) to give a crude residue. The resulting residue was purified by amino-silica gel column chromatography to afford 30 mg of the target compound as a white solid. MS(ESI+)m/z 569.2(M+1)

Example 37: Synthesis of [5-chloro-1-methyl-6-(2H-1,2,3-triazol-2-yl)-1H-pyrrolo[2,3-b]pyridin-3-yl][1-(8-fluoroisoquinolin-4-yl)-5-(trifluoromethyl)-1H-pyrazol-4-yl]methanone sulfate

To a solution of [5-chloro-1-methyl-6-(2H-1,2,3-triazol-2-yl)-1H-pyrrolo[2,3-b]pyridin-3-yl][1-(8-fluoroisoquinolin-4-yl)-5-(trifluoromethyl)-1H-pyrazol-4-yl]methanone (5.0 g) in acetone (50 mL) were added a few milligrams of seed crystals, followed by dropwise addition of sulfuric acid (0.74 mL), and the mixture was stirred overnight at room temperature. The precipitated solid was collected by filtration to afford 5.6 g of the target compound as a beige solid.

Elemental analysis value for $C_{24}H_{13}ClF_4N_8O \cdot H_2SO_4$ + $0.2H_2O$
Calcd (%) C: 44.86 H: 2.41 N: 17.43
Found (%) C: 45.15 H: 2.23 N: 17.10

Example 38: Synthesis of [5-chloro-1-methyl-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone monophosphate

To a solution of [5-chloro-1-methyl-6-(pyrimidin-2-yl)-1H-pyrrolo[2,3-b]pyridin-3-yl][(2R,6R)-1-(5-fluoro-3-10odopyridin-2-yl)-2,6-dimethylpiperidin-4-yl]methanone (180 g) in acetonitrile (3.0 L) was added a 85% aqueous phosphoric acid solution (40 mL) dropwise, and the mixture was stirred overnight at room temperature. The precipitated solid was collected by filtration to afford 210 g of the target compound as a yellow solid.

Melting point: 186°C to 188°C
Specific optical rotation $[\alpha]_D^{20}$ = +57.99° (c = 0.6, chloroform)

Elemental analysis value for $C_{25}H_{23}ClFIN_6O \cdot H_3PO_4$
Calcd (%): C: 42.72 H: 3.73 N: 11.96
Found (%): C: 42.49 H: 3.51 N: 11.98

The structural formulae of Examples 1 to 38 are illustrated in Tables 1 to 8.

[Table 1]

| Example | Structural formula |
|---|---|
| 1 | 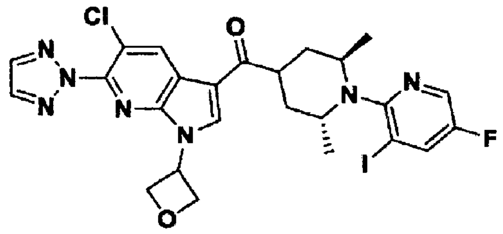 |
| 2 | 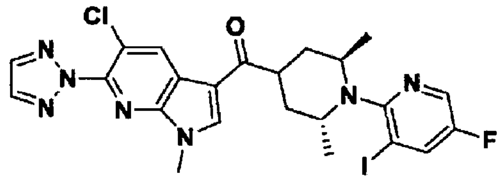 |
| 3 | 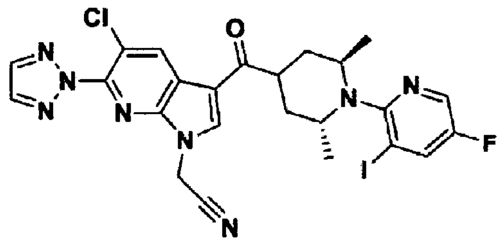 |
| 4 | 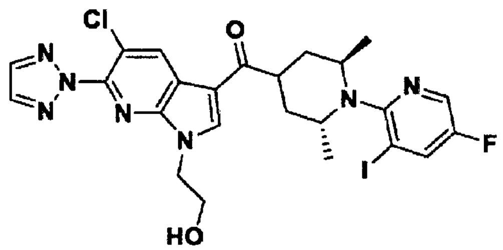 |
| 5 | 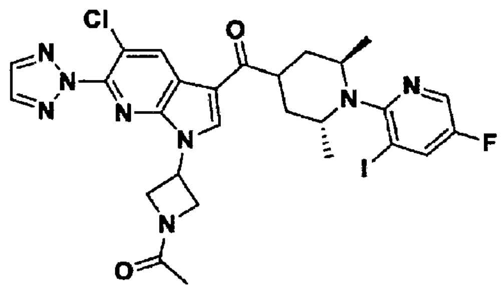 |

[Table 2]

| Example | Structural formula |
|---|---|
| 6 | 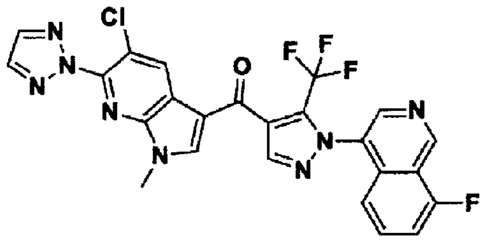 |
| 7 | 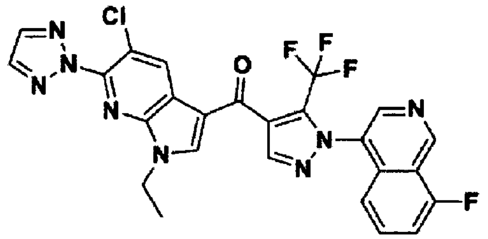 |
| 8 | 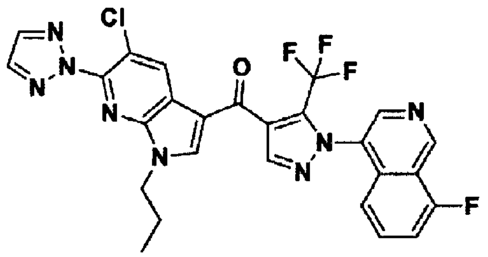 |
| 9 | 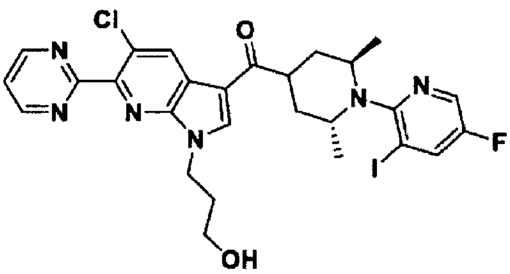 |
| 10 | 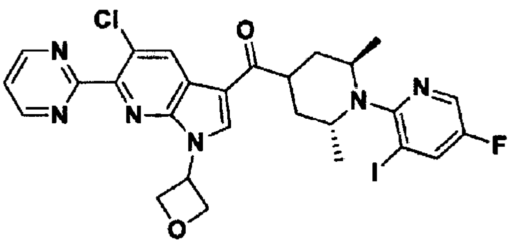 |

[Table 3]

| Example | Structural formula |
|---|---|
| 11 | 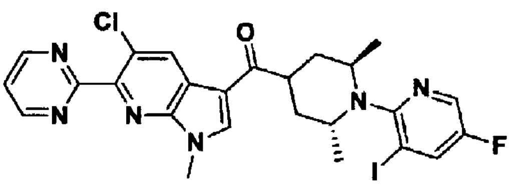 |

(continued)

| Example | Structural formula |
|---|---|
| 12 | 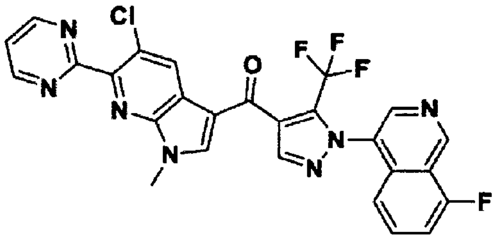 |
| 13 | 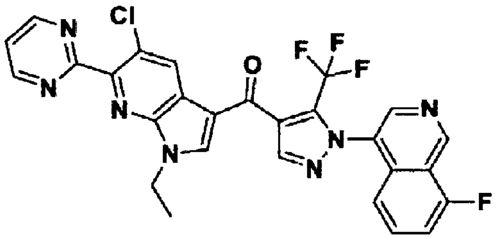 |
| 14 | 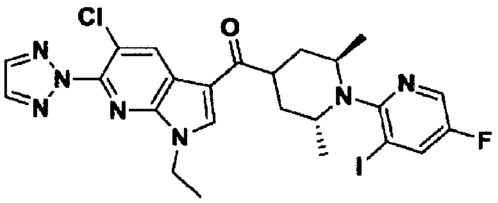 |
| 15 | 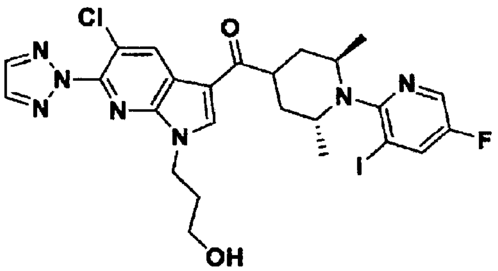 |

[Table 4]

| | Structural formula |
|---|---|
| 16 | 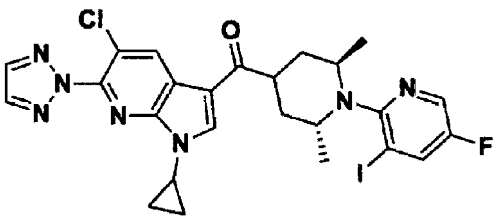 |
| 17 | 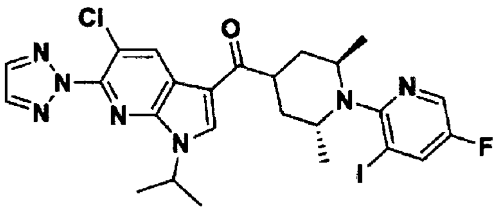 |

(continued)

| | Structural formula |
|---|---|
| 18 | 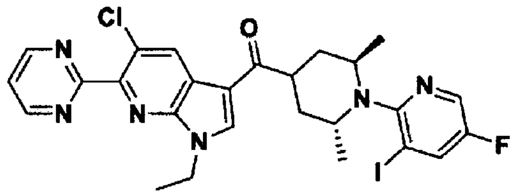 |
| 19 | 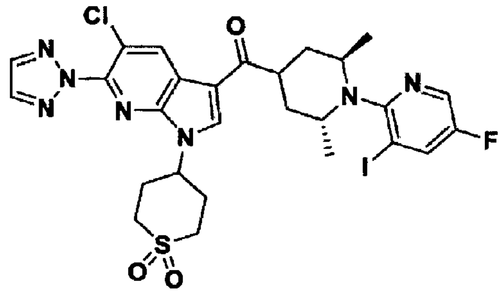 |

[Table 5]

| Example | Structural formula |
|---|---|
| 20 | 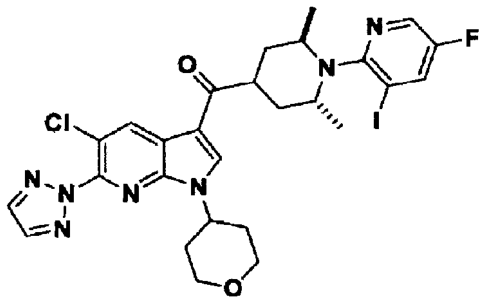 |
| 21 | 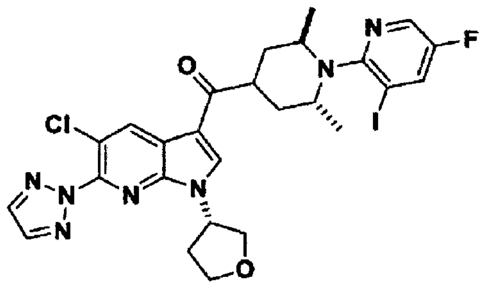 |
| 22 | 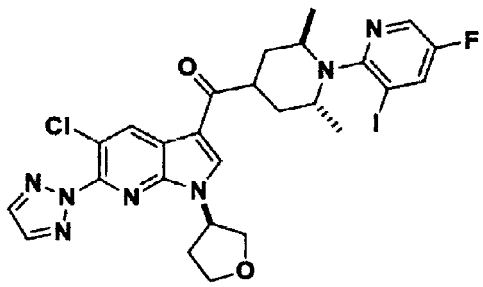 |

(continued)

| Example | Structural formula |
|---|---|
| 23 | 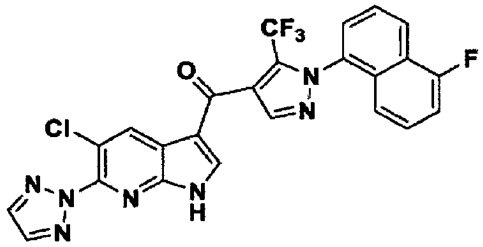 |
| 24 | 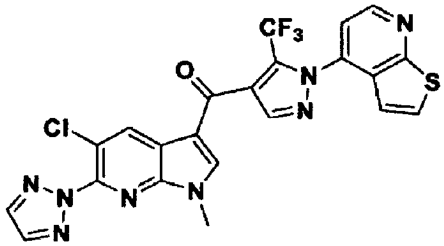 |

[Table 6]

| Example | Structural formula |
|---|---|
| 25 | 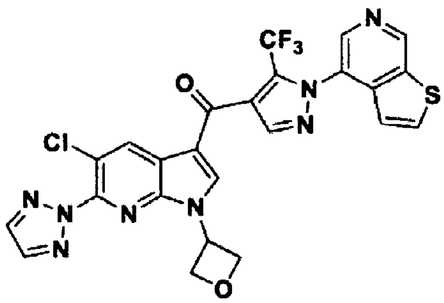 |
| 26 | 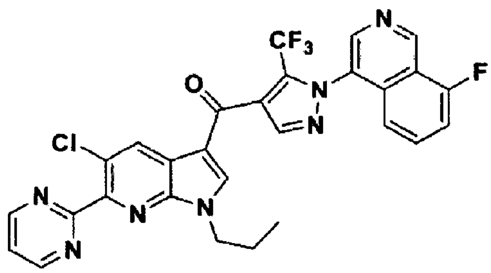 |
| 27 | 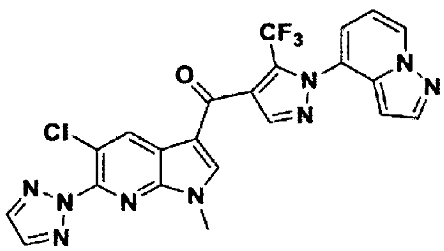 |

(continued)

| Example | Structural formula |
|---|---|
| 28 | 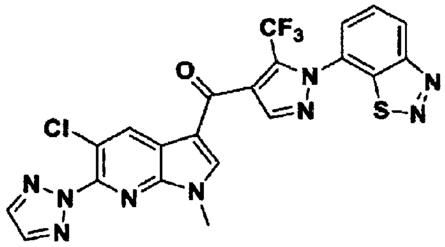 |
| 29 | 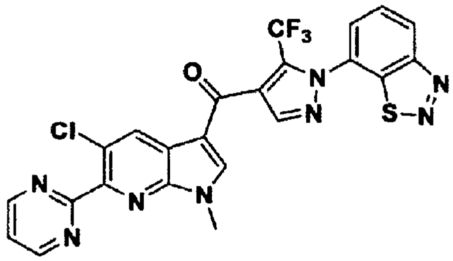 |

[Table 7]

| | Structural formula |
|---|---|
| 30 | 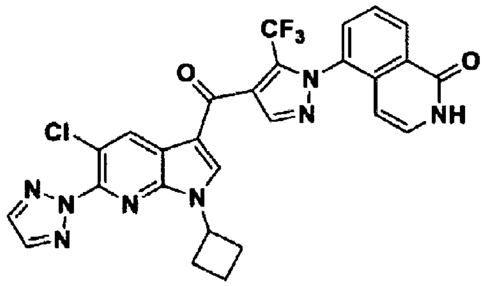 |
| 31 | 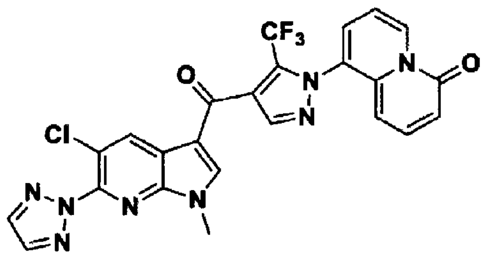 |
| 32 | 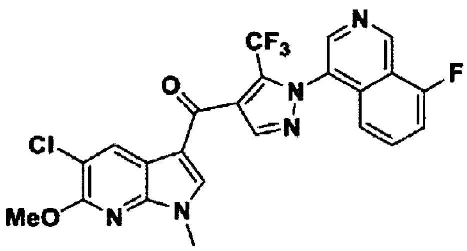 |

(continued)

| | Structural formula |
|---|---|
| 33 | 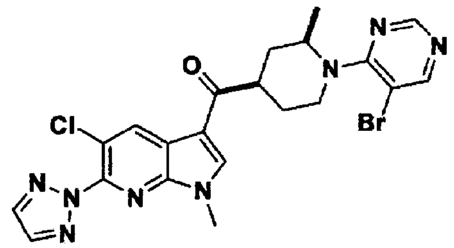 |
| 34 | 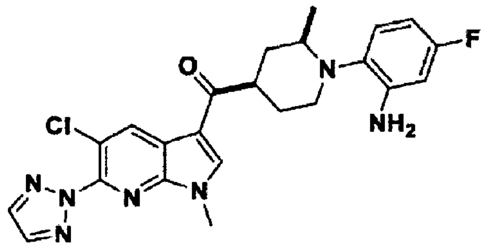 |

[Table 8]

| | Structural formula |
|---|---|
| 35 | 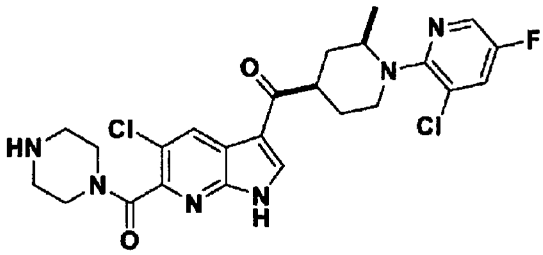 |
| 36 | 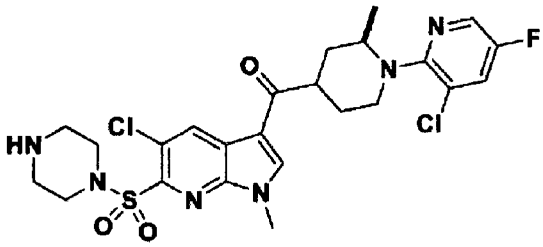 |
| 37 | 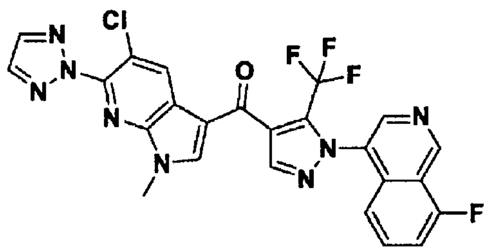 $H_2SO_4$ |
| 38 | 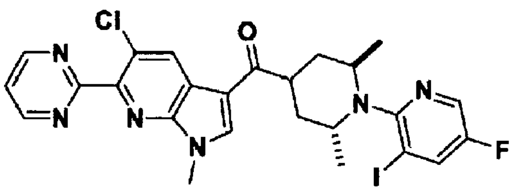 $H_3PO_4$ |

Biological test examples for the compounds of the present invention are described below.

<Test Example 1: MALT1 Protease Inhibitory Activity>

To evaluate the enzyme inhibitory activity of a test substance against MALT1, an enzyme activity inhibition assay was conducted using the C-terminal catalytic domain of MALT1 (amino acid residues 329-824).

1. Preparation of Test Substance

A test substance was prepared at a concentration of 10 mM in dimethyl sulfoxide (DMSO) and further diluted with DMSO to obtain final concentrations of 1000, 100, 10, 1, and 0.1 μM. The solutions were further diluted 25-fold with an assay buffer to prepare final test substance solutions. The composition of the assay buffer was 200 mM Tris/HCl HEPES (pH 7.5), 0.8 M Na citrate, 100 μM DTT, and 0.05% CHAPS.

2. Measurement of MALT 1 Protease Inhibitory Activity

The test substance solution was added to a 384-well black plate (#6007270, PerkinElmer) in an amount of 5 μL per well (n = 2; final concentrations of 10000, 1000, 100, 10, 1, and 0.1 nM), followed by the addition of 10 μL of MALT1 enzyme and finally 5 μL of a substrate solution (Ac-Leu-Arg-Ser-Arg-AMC, Peptide Institute, Inc.; final concentration in the reaction mixture: 50 μM), and after stirring, the mixture was allowed to react at 30°C for 2 hours. Fluorescence (excitation: 380 nm, emission: 460 nm) was detected using a microplate reader (EnVision, PerkinElmer).

3. Analysis of Measurement Results

Using the measurement data, non-linear regression analysis based on a 2-parameter logistic model was performed with Spotfire (PerkinElmer) to calculate $IC_{50}$. The results are illustrated in Table 9 below.

[Table 9]

| Example No. | MALT1 protease $IC_{50}$ (nM) | Example No. | MALT1 protease $IC_{50}$ (nM) |
|---|---|---|---|
| Example 1 | 45 | Example 19 | 92 |
| Example 2 | 18 | Example 20 | 110 |
| Example 3 | 43 | Example 21 | 44 |
| Example 4 | 44 | Example 22 | 70 |
| Example 5 | 35 | Example 23 | 19 |
| Example 6 | 13 | Example 24 | 12 |
| Example 7 | 25 | Example 25 | 19 |
| Example 8 | 39 | Example 26 | 51 |
| Example 9 | 69 | Example 27 | 5.8 |
| Example 10 | 67 | Example 28 | 24 |
| Example 11 | 54 | Example 29 | 17 |
| Example 12 | 11 | Example 30 | 44 |
| Example 13 | 15 | Example 31 | 25 |
| Example 14 | 58 | Example 32 | 54 |
| Example 15 | 35 | Example 33 | 10 |
| Example 16 | 310 | Example 34 | 13 |
| Example 17 | 160 | Example 35 | 37 |
| Example 18 | 65 | Example 36 | 20 |

<Test Example 2: Inhibitory Activity Against Reporter Activity in HEK293/AP12-MALT1/NF-κB Reporter Cells>

The API2-MALT1 fusion protein, expressed in MALT lymphoma, is known to constitutively activate the NF-κB pathway. To evaluate the inhibitory effect of the test substance on the NF-κB pathway in cells, a reporter activity inhibition test was conducted using HEK293/API2-MALT1/NF-κB reporter cells.

1. Preparation of Test Substance

The test substance was prepared at a concentration of 10 mM in dimethyl sulfoxide (DMSO) and further diluted with DMSO to each of concentrations of 1000, 300, 100, and 10 μM. The solutions were further diluted 100-fold with DMEM medium containing 10% FBS to prepare test substance solutions.

2. Measurement of Inhibitory Activity on Reporter Cell Proliferation

HEK293/API2-MALT1/NF-κB reporter cells and 293T/CMV reporter cells (used as a negative control) were cultured in DMEM medium containing 10% FBS. Into a 384-well plate (#781080, Greiner), 6,000 cells were seeded in 30 μL per well, and after culturing for 24 hours, 3.3 μL of the adjusted test substance solution was added thereto (final concentrations: 10,000, 1,000, 100, and 10 nM). After culturing for 24 hours at 37°C in a 5% $CO_2$ incubator, 33 μL of One-glo (Promega) was added to each well. After a 5-minute incubation at room temperature, luminescence was detected using a microplate reader (EnVision, PerkinElmer).

3. Analysis of Measurement Results

Using the measurement data, non-linear regression analysis was performed with the SAS system (SAS Institute Inc.) to estimate $IC_{50}$. The results are illustrated in Table 10 below.

[Table 10]

| Example No. | NF-κB reporter $IC_{50}$ (nM) | Example No. | NF-κB reporter $IC_{50}$ (nM) |
|---|---|---|---|
| Example 1 | 170 | Example 19 | 130 |
| Example 2 | 60 | Example 20 | 240 |
| Example 3 | 200 | Example 21 | 310 |
| Example 4 | 230 | Example 22 | 280 |
| Example 5 | 160 | Example 23 | 190 |
| Example 6 | 75 | Example 24 | 130 |
| Example 7 | 150 | Example 25 | 180 |
| Example 8 | 280 | Example 26 | 110 |
| Example 9 | 140 | Example 27 | 280 |
| Example 10 | 140 | Example 28 | 440 |
| Example 11 | 230 | Example 29 | 220 |
| Example 12 | 85 | Example 30 | 500 |
| Example 13 | 62 | Example 31 | 73 |
| Example 14 | 70 | Example 32 | 280 |
| Example 15 | 170 | Example 33 | 640 |
| Example 16 | | Example 34 | 200 |
| Example 17 | 300 | Example 35 | 390 |
| Example 18 | 100 | Example 36 | 280 |

<Test Example 3: Proliferation Inhibitory Activity against ABC-DLBCL Cell Lines>

In ABC-DLBCL cell lines, the NF-κB pathway is constitutively activated due to mutations in the B-cell receptor

pathway. To evaluate the proliferation inhibitory activity associated with the MALT1 inhibitory activity of the test substance, a proliferation inhibition test was conducted using ABC-DLBCL cell lines.

1. Preparation of Test Substance

The test substance was prepared at a concentration of 10 mM in dimethyl sulfoxide (DMSO) and further diluted with DMSO to each of concentrations of 3,000, 1,000, 300, 100, 30, and 10 μM. The solutions were further diluted 100-fold with IMDM medium containing 10% FBS to prepare test substance solutions.

2. Measurement of Proliferation Inhibitory Activity against ABC-DLBCL Cells

OCI-Ly3 cell lines (ABC-DLBCL, NF-κB pathway-dependent) were cultured in IMDM containing 10% FBS, and SU-DHL-4 cell lines (GCB-DLBCL, NF-κB pathway-independent, used as a negative control) were cultured in RPMI-1640 containing 10% FBS. Into a 96-well plate, 2,000 cells of the OCI-Ly3 cell line and 4,000 cells of the SU-DHL-4 cell line were seeded in 100 μL per well, and 11 μL of the adjusted test substance solution was added thereto (final concentrations: 10,000, 3,000, 1,000, 300, 100, 30, and 10 nM). After 96 hours of incubation at 37°C in a 5% $CO_2$ incubator, 10 μL of Cell Counting Kit-8 (Dojindo Laboratories) was added to each well. After 4 hours of incubation at 37°C, absorbance at 450 nm was detected using a microplate reader (EnVision, PerkinElmer).

3. Analysis of Measurement Results

Using the measurement data, non-linear regression analysis based on a 2-parameter logistic model was performed with Spotfire (PerkinElmer) to estimate $IC_{50}$ values. The results are illustrated in Tables 11 and 12 below.

[Table 11]

| Example No. | Proliferation inhibitory activity against OCI-Ly3 $IC_{50}$ (nM) | Example No. | Proliferation inhibitory activity against OCI-Ly3 $IC_{50}$ (nM) |
|---|---|---|---|
| Example 1 | 620 | Example 19 | 240 |
| Example 2 | 300 | Example 20 | 480 |
| Example 3 | 580 | Example 21 | 540 |
| Example 4 | 450 | Example 22 | 530 |
| Example 5 | 310 | Example 23 | 870 |
| Example 6 | 150 | Example 24 | 180 |
| Example 7 | 130 | Example 25 | 440 |
| Example 8 | 110 | Example 26 | 140 |
| Example 9 | 480 | Example 27 | 380 |
| Example 10 | 440 | Example 28 | 610 |
| Example 11 | 290 | Example 29 | 1000 |
| Example 12 | 210 | Example 30 | 870 |
| Example 13 | 110 | Example 31 | 150 |
| Example 14 | 470 | Example 32 | 140 |
| Example 15 | 720 | Example 33 | 790 |
| Example 16 | 720 | Example 34 | 520 |
| Example 17 | 690 | Example 35 | 630 |
| Example 18 | 480 | Example 36 | 410 |

[Table 12]

| Example No. | Proliferation inhibitory activity against SU-DHL-4 $IC_{50}$ (nM) | Example No. | Proliferation inhibitory activity against SU-DHL-4 $IC_{50}$ (nM) |
|---|---|---|---|
| Example 1 | 5400 | Example 19 | 2500 |
| Example 2 | 7200 | Example 20 | 6400 |
| Example 3 | 6300 | Example 21 | 9000 |
| Example 4 | 7200 | Example 22 | 8900 |
| Example 5 | 3600 | Example 23 | 9500 |
| Example 6 | 2300 | Example 24 | 9100 |
| Example 7 | 3200 | Example 25 | 9000 |
| Example 8 | 2400 | Example 26 | 1400 |
| Example 9 | 5100 | Example 27 | 7200 |
| Example 10 | 6000 | Example 28 | >10000 |
| Example 11 | 8900 | Example 29 | >10000 |
| Example 12 | 1900 | Example 30 | >10000 |
| Example 13 | 2000 | Example 31 | >10000 |
| Example 14 | 8500 | Example 32 | >10000 |
| Example 15 | 8400 | Example 33 | >10000 |
| Example 16 | 9700 | Example 34 | >10000 |
| Example 17 | 6700 | Example 35 | 9500 |
| Example 18 | 6700 | Example 36 | 2500 |

<Test Example 5: Central Nervous System (CNS) Penetration Study in Mice>

To evaluate the central nervous system (CNS) penetration of a test substance, a central nervous system penetration study was conducted using mice.

1. Administration of Test Substance and Sample Collection

Six-week-old female BALB/cCrSlc mice (Japan SLC) were used. The test substance suspended in 0.5% methylcellulose was intraperitoneally administered to mice at a dose of 30 mg/kg. Three hours later, blood was collected from the abdominal vena cava under isoflurane anesthesia, and plasma was obtained by centrifugation. After exsanguination from the abdominal vena cava, the cerebrum was collected.

2. Measurement and Analysis of Plasma and Brain concentrations

The drug concentrations of the collected plasma and cerebrum were measured by LC-MS/MS. To determine the brain penetration of the drug, the brain-to-plasma concentration ratio (Kp value) was calculated. The results are illustrated in Table 13 below.

[Table 13]

| Example No. | Brain-to-plasma concentration ratio (Kp value) | Example No. | Brain-to-plasma concentration ratio (Kp value) |
|---|---|---|---|
| Example 1 | 1.6 | Example 11 | 3.0 |
| Example 2 | 1.2 | Example 12 | 0.66 |
| Example 3 | 0.73 | Example 14 | 0.74 |

(continued)

| Example No. | Brain-to-plasma concentration ratio (Kp value) | Example No. | Brain-to-plasma concentration ratio (Kp value) |
|---|---|---|---|
| Example 4 | 0.71 | Example 15 | 0.81 |
| Example 5 | 0.12 | Example 16 | 0.28 |
| Example 6 | 0.83 | Example 17 | 0.26 |
| Example 7 | 0.52 | Example 18 | 0.63 |
| Example 8 | 0.52 | Example 26 | 0.27 |
| Example 9 | 1.4 | Example 28 | 1.75 |
| Example 10 | 1.7 | Example 29 | 0.98 |

## Claims

**1.** A compound represented by General Formula (1) or a pharmaceutically acceptable salt thereof:

[Chem. 1]

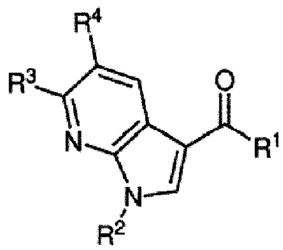

(1)

wherein

$R^1$ represents a group represented by General Formula (2) or General Formula (3);

[Chem. 2]

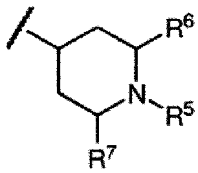

(2)

[Chem. 3]

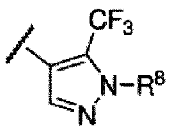

(3)

$R^2$ represents a hydrogen atom, a substituted or unsubstituted alkyl group, a substituted or unsubstituted cycloalkyl group, or a substituted or unsubstituted heterocyclic group;

$R^3$ represents a substituted or unsubstituted aryl group, a substituted or unsubstituted heteroaryl group, a substituted or unsubstituted alkoxy group, a substituted or unsubstituted aryloxy group, a substituted or unsubstituted heteroaryloxy group, a group represented by General Formula (4), or a group represented by General Formula (5);

[Chem. 4]

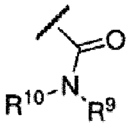

( 4 )

[Chem. 5]

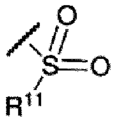

( 5 )

$R^4$ represents halogen;
$R^5$ represents a substituted or unsubstituted aryl group or a substituted or unsubstituted heteroaryl group;
$R^6$ and $R^7$ each independently represent a hydrogen atom or a substituted or unsubstituted alkyl group;
$R^8$ represents a substituted or unsubstituted aryl group, a substituted or unsubstituted heteroaryl group, or a substituted or unsubstituted heterocyclic group;
$R^9$ and $R^{10}$ each independently represent a substituted or unsubstituted alkyl group, a substituted or unsubstituted aryl group, or a substituted or unsubstituted heteroaryl group, and $R^9$ and $R^{10}$ may be linked to each other to form a ring;
$R^{11}$ represents a substituted or unsubstituted aryl group, a substituted or unsubstituted heteroaryl group, or a substituted or unsubstituted heterocyclic group; and
in General Formula (2), General Formula (3), General Formula (4), and General Formula (5), a wavy line represents a bond.

**2.** The compound or a pharmaceutically acceptable salt thereof according to claim 1, wherein

the compound is a compound represented by General Formula (6),

[Chem. 6]

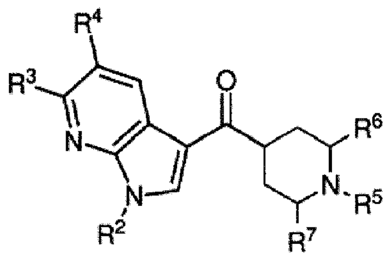

( 6 )

wherein $R^3$ represents a substituted or unsubstituted aryl group, a substituted or unsubstituted heteroaryl group, a group represented by General Formula (4), or a group represented by General Formula (5).

**3.** The compound or a pharmaceutically acceptable salt thereof according to claim 1, wherein

the compound is a compound represented by General Formula (7),

[Chem. 7]

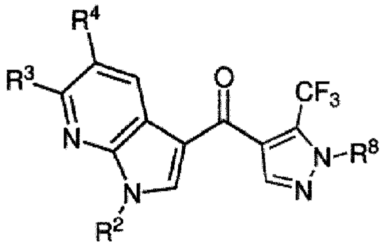

( 7 )

wherein $R^3$ represents a substituted or unsubstituted aryl group, a substituted or unsubstituted heteroaryl group, a substituted or unsubstituted alkoxy group, a substituted or unsubstituted aryloxy group, or a substituted or unsubstituted heteroaryloxy group.

**4.** A compound or a pharmaceutically acceptable salt thereof, wherein the compound is selected from the group consisting of compounds (1) to (36) below:

(1) [5-chloro-1-(oxetan-3-yl)-6-(2H-1,2,3-triazol-2-yl)-1H-pyrrolo[2,3-b]pyridin-3-yl][(2R,6R)-1-(5-fluoro-3-10odopyridin-2-yl)-2,6-dimethylpiperidin-4-yl]methanone;
(2) [5-chloro-1-methyl-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(3) {5-chloro-3-[(2R,6R)-1-(5-fluoro-3-iodopyridin-2-yl)-2,6-dimethylpiperidine-4-carbonyl]-6-(2H-1,2,3-triazol-2-yl)-1H-pyrrolo[2,3-b]pyridin-1-yl}acetonitrile;
(4) [5-chloro-1-(2-hydroxyethyl)-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(5) 1-(3-{5-chloro-3-[(2R,6R)-1-(5-fluoro-3-iodopyridin-2-yl)-2,6-dimethylpiperidine-4-carbonyl]-6-(2H-1,2,3-triazol-2-yl)-1H-pyrrolo[2,3-b]pyridin-1-yl}azetidin-1-yl)ethan-1-one;
(6) [5-chloro-1-methyl-6-(2H-1,2,3-triazol-2-yl)-1H-pyrrolo[2,3-b]pyridin-3-yl][1-(8-fluoroisoquinolin-4-yl)-5-(trifluoromethyl)-1H-pyrazol-4-yl]methanone;
(7) [5-chloro-1-ethyl-6-(2H-1,2,3-triazol-2-yl)-1H-pyrrolo[2,3-b]pyridin-3-yl][1-(8-fluoroisoquinolin-4-yl)-5-(trifluoromethyl)-1H-pyrazol-4-yl]methanone;
(8) [5-chloro-1-propyl-6-(2H-1,2,3-triazol-2-yl)-1H-pyrrolo[2,3-b]pyridin-3-yl][1-(8-fluoroisoquinolin-4-yl)-5-(trifluoromethyl)-1H-pyrazol-4-yl]methanone;
(9) [5-chloro-1-(3-hydroxypropyl)-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(10) [5-chloro-1-(oxetan-3-yl)-6-(pyrimidin-2-yl)-1H-pyrrolo[2,3-b]pyridin-3-yl][(2R,6R)-1-(5-fluoro-3-10odopyridin-2-yl)-2,6-dimethylpiperidin-4-yl]methanone;
(11) [5-chloro-1-methyl-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(12) [5-chloro-1-methyl-6-(pyrimidin-2-yl)-1H-pyrrolo[2,3-b]pyridin-3-yl][1-(8-fluoroisoquinolin-4-yl)-5-(trifluoromethyl)-1H-pyrazol-4-yl]methanone;
(13) [5-chloro-1-ethyl-6-(pyrimidin-2-yl)-1H-pyrrolo[2,3-b]pyridin-3-yl][1-(8-fluoroisoquinolin-4-yl)-5-(trifluoromethyl)-1H-pyrazol-4-yl]methanone;
(14) [5-chloro-1-ethyl-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(15) [5-chloro-1-(3-hydroxypropyl)-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(16) [5-chloro-1-cyclopropyl-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(17) [5-chloro-1-isopropyl-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(18) [5-chloro-1-ethyl-6-(pyrimidin-2-yl)-1H-pyrrolo[2,3-b]pyridin-3-yl][(2R,6R)-1-(5-fluoro-3-iodopyridin-2-yl)-2,6-dimethylpiperidin-4-yl]methanone;
(19) [5-chloro-1-(1,1-dioxidotetrahydro-2H-thiopyran-4-yl)-6-(2H-1,2,3-triazol-2-yl)-1H-pyrrolo[2,3-b]pyridin-3-yl] [(2R,6R)-1-(5-fluoro-3-iodopyridin-2-yl)-2,6-dimethylpiperidin-4-yl]methanone;
(20) [5-chloro-1-(oxan-4-yl)-6-(2H-1,2,3-triazol-2-yl)-1H-pyrrolo[2,3-b]pyridin-3-yl][(2R,6R)-1-(5-fluoro-3-iodopyridin-2-yl)-2,6-dimethylpiperidin-4-yl]methanone;

(21) {5-chloro-1-[(3S)-oxolan-3-yl]-6-(2H-1,2,3-triazol-2-yl)-1H-pyrrolo[2,3-b]pyridin-3-yl}[(2R,6R)-1-(5-fluoro-3-iodopyridin-2-yl)-2,6-dimethylpiperidin-4-yl]methanone;
(22) {5-chloro-1-[(3R)-oxolan-3-yl]-6-(2H-1,2,3-triazol-2-yl)-1H-pyrrolo[2,3-b]pyridin-3-yl}[(2R,6R)-1-(5-fluoro-3-iodopyridin-2-yl)-2,6-dimethylpiperidin-4-yl]methanone;
(23) [5-chloro-6-(2H-1,2,3-triazol-2-yl)-1H-pyrrolo[2,3-b]pyridin-3-yl][1-(5-fluoronaphthalen-1-yl)-5-(trifluoromethyl)-1H-pyrazol-4-yl]methanone;
(24) [5-chloro-1-methyl-6-(2H-1,2,3-triazol-2-yl)-1H-pyrrolo[2,3-b]pyridin-3-yl][1-(thieno[2,3-b]pyridin-4-yl)-5-(trifluoromethyl)-1H-pyrazol-4-yl]methanone;
(25) [5-chloro-1-(oxetan-3-yl)-6-(2H-1,2,3-triazol-2-yl)-1H-pyrrolo[2,3-b]pyridin-3-yl][1-(thieno[2,3-c]pyridin-4-yl)-5-(trifluoromethyl)-1H-pyrazol-4-yl]methanone;
(26) [5-chloro-1-propyl-6-(pyrimidin-2-yl)-1H-pyrrolo[2,3-b]pyridin-3-yl][1-(8-fluoroisoquinolin-4-yl)-5-(trifluoromethyl)-1H-pyrazol-4-yl]methanone;
(27) [5-chloro-1-methyl-6-(2H-1,2,3-triazol-2-yl)-1H-pyrrolo[2,3-b]pyridin-3-yl][1-(pyrazolo[1,5-a]pyridin-4-yl)-5-(trifluoromethyl)-1H-pyrazol-4-yl]methanone;
(28) [1-(1,2,3-benzothiadiazol-7-yl)-5-(trifluoromethyl)-1H-pyrazol-4-yl][5-chloro-1-methyl-6-(2H-1,2,3-triazol-2-yl)-1H-pyrrolo[2,3-b]pyridin-3-yl]methanone;
(29) [1-(1,2,3-benzothiadiazol-7-yl)-5-(trifluoromethyl)-1H-pyrazol-4-yl] [5-chloro-1-methyl-6-(pyrimidin-2-yl)-1H-pyrrolo[2,3-b]pyridin-3-yl]methanone;
(30) 5-{4-[5-chloro-1-cyclobutyl-6-(2H-1,2,3-triazol-2-yl)-1H-pyrrolo[2,3-b]pyridine-3-carbonyl]-5-(trifluoromethyl)-1H-pyrazol-1-yl}isoquinolin-1(2H)-one;
(31) 9-{4-[5-chloro-1-methyl-6-(2H-1,2,3-triazol-2-yl)-1H-pyrrolo[2,3-b]pyridine-3-carbonyl]-5-(trifluoromethyl)-1H-pyrazol-1-yl}-4H-quinolizin-4-one;
(32) (5-chloro-6-methoxy-1-methyl-1H-pyrrolo[2,3-b]pyridin-3-yl)[1-(8-fluoroisoquinolin-4-yl)-5-(trifluoromethyl)-1H-pyrazol-4-yl]methanone;
(33) [(2RS,4RS)-1-(5-bromopyrimidin-4-yl)-2-methylpiperidin-4-yl][5-chloro-1-methyl-6-(2H-1,2,3-triazol-2-yl)-1H-pyrrolo[2,3-b]pyridin-3-yl]methanone;
(34) [(2RS,4RS)-1-(2-amino-4-fluorophenyl)-2-methylpiperidin-4-yl][5-chloro-1-methyl-6-(2H-1,2,3-triazol-2-yl)-1H-pyrrolo[2,3-b]pyridin-3-yl]methanone;
(35) {5-chloro-3-[(2RS,4RS)-1-(3-chloro-5-fluoropyridin-2-yl)-2-methylpiperidine-4-carbonyl]-1H-pyrrolo[2,3-b]pyridin-6-yl}(piperazin-1-yl)methanone; and
(36) [(2RS,4RS)-1-(3-chloro-5-fluoropyridin-2-yl)-2-methylpiperidin-4-yl][5-chloro-1-methyl-6-(piperazin-1-sulfonyl)-1H-pyrrolo[2,3-b]pyridin-3-yl]methanone.

**5.** A pharmaceutical composition comprising, as an active ingredient, the compound or a pharmaceutically acceptable salt thereof according to any one of claims 1 to 4.

**6.** A MALT1 inhibitor comprising, as an active ingredient, the compound or a pharmaceutically acceptable salt thereof according to any one of claims 1 to 4.

**7.** A prophylactic or therapeutic agent for a disease involving MALT1, the agent comprising, as an active ingredient, the compound or a pharmaceutically acceptable salt thereof according to any one of claims 1 to 4.

**8.** A therapeutic agent for multiple sclerosis, rheumatoid arthritis, psoriasis, immune thrombocytopenia, spinal cord injury, graft-versus-host disease associated with bone marrow transplantation, rejection associated with organ transplantation, aplastic anemia, Behcet's disease, nephrotic syndrome, generalized myasthenia gravis, atopic dermatitis, diffuse large B-cell lymphoma, MALT lymphoma, mantle cell lymphoma, follicular lymphoma, marginal zone B-cell lymphoma, Burkitt lymphoma, multiple myeloma, BENTA syndrome, adult T-cell leukemia/lymphoma, peripheral T-cell lymphoma, Sezary's syndrome, primary effusion lymphoma, chronic lymphocytic leukemia/small lymphocytic lymphoma, primary central nervous system malignant lymphoma, intraocular lymphoma, primary macroglobulinemia, lymphoplasmacytic lymphoma, brain tumor, malignant melanoma, non-small cell lung cancer, renal cell carcinoma, head and neck cancer, gastric cancer, malignant pleural mesothelioma, colon and rectal cancer, or esophageal cancer,
the agent comprising, as an active ingredient, the compound or a pharmaceutically acceptable salt thereof according to any one of claims 1 to 4.

**INTERNATIONAL SEARCH REPORT**

International application No.
**PCT/JP2024/034463**

**A. CLASSIFICATION OF SUBJECT MATTER**

***C07D 471/04***(2006.01)i; ***A61K 31/437***(2006.01)i; ***A61K 31/496***(2006.01)i; ***A61K 31/506***(2006.01)i; ***A61K 31/4375***(2006.01)i; ***A61K 31/4545***(2006.01)i; ***A61K 31/4725***(2006.01)i; ***A61P 29/00***(2006.01)i; ***A61P 35/00***(2006.01)i; ***A61P 35/02***(2006.01)i; ***A61P 37/02***(2006.01)i; ***A61P 37/04***(2006.01)i; ***A61P 37/06***(2006.01)i; ***A61P 43/00***(2006.01)i; ***C07D 519/00***(2006.01)i

FI: C07D471/04 CSP; A61K31/437; A61K31/4375; A61K31/4545; A61K31/4725; A61K31/496; A61K31/506; A61P29/00; A61P35/00; A61P35/02; A61P37/02; A61P37/04; A61P37/06; A61P43/00 105; A61P43/00 111; C07D519/00 301; C07D519/00 311

According to International Patent Classification (IPC) or to both national classification and IPC

**B. FIELDS SEARCHED**

Minimum documentation searched (classification system followed by classification symbols)

C07D471/04; A61K31/437; A61K31/4375; A61K31/4545; A61K31/4725; A61K31/496; A61K31/506; A61P29/00; A61P35/00; A61P35/02; A61P37/02; A61P37/04; A61P37/06; A61P43/00; C07D519/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2006/058338 A2 (JANSSEN PHARMACEUTICA N.V.) 01 June 2006 (2006-06-01) claims, examples | 1-8 |
| A | WO 2017/081641 A1 (NOVARTIS AG) 18 May 2017 (2017-05-18) claims, examples | 1-8 |
| A | SCHLAPBACH, Achim et al. N-aryl-piperidine-4-carboxamides as a novel class of potent inhibitors of MALT1 proteolytic activity. Bioorganic & Medicinal Chemistry Letters. 2018, vol. 28, no. 12, pp. 2153-2158 tables 1-4, scheme 1-2 | 1-8 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 October 2024** | **29 October 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**

International application No.
**PCT/JP2024/034463**

**C. DOCUMENTS CONSIDERED TO BE RELEVANT**

| Category* | Citation of document, with indication, where appropriate, of the relevant passages | Relevant to claim No. |
|---|---|---|
| A | WO 2018/119036 A1 (JANSSEN BIOTECH, INC.) 28 June 2018 (2018-06-28) claims, examples | 1-8 |
| A | WO 2021/138298 A1 (RHEOS MEDICINES, INC.) 08 July 2021 (2021-07-08) claims, examples | 1-8 |
| A | WO 2022/262855 A1 (TUOJIE BIOTECH (SHANGHAI) CO., LTD.) 22 December 2022 (2022-12-22) claims, examples | 1-8 |

Form PCT/ISA/210 (second sheet) (July 2022)

**INTERNATIONAL SEARCH REPORT**
**Information on patent family members**

International application No.
**PCT/JP2024/034463**

| Patent document cited in search report | Publication date (day/month/year) | Patent family member(s) | Publication date (day/month/year) |
|---|---|---|---|
| WO 2006/058338 A2 | 01 June 2006 | US 2006/0116368 A1 | |
| | | TW 200635909 A | |
| WO 2017/081641 A1 | 18 May 2017 | US 2020/0289514 A1 | |
| | | EP 3374361 A1 | |
| | | AU 2016352813 A | |
| | | CA 3003820 A | |
| | | MX 2018005390 A | |
| | | BR 112018009511 A | |
| | | JP 2018-533610 A | |
| WO 2018/119036 A1 | 28 June 2018 | US 2018/0170909 A1 | |
| | | EP 3558969 A1 | |
| | | CA 3048027 A | |
| | | KR 10-2019-0093669 A | |
| | | CN 110214136 A | |
| | | BR 112019012355 A | |
| | | JP 2020-514267 A | |
| WO 2021/138298 A1 | 08 July 2021 | (Family: none) | |
| WO 2022/262855 A1 | 22 December 2022 | CN 117120427 A | |
| | | TW 202317537 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018119036 A
- WO 2021062316 A


### Non-patent literature cited in the description


- **JAWORSKI et al.** *Cellular and Molecular Life Sciences*, 2016, vol. 73, 459-473
- **STAUDT et al.** *Cold Spring Harbor Perspectives in Biology*, 2010, vol. 2, 1-30
- **CHIHARA et al.** *Clinical Lymphoma, Myeloma & Leukemia*, 2020, vol. 21, 73-79
- **WILSON et al.** *Nature Medicine*, 2015, vol. 21, 922-927
- **LOW et al.** *CNS Oncology*, 2020, vol. 9, CNS51
- **FONTAN et al.** *The Journal of Clinical Investigation*, 2018, vol. 128, 4397-4412
- **RUEFLI-BRASSE et al.** *Science*, 2003, vol. 302, 1581-1584
- **JABARA et al.** *Journal of Allergy and Clinical Immunology*, 2003, vol. 132, 151-158
- **BARDET et al.** *Immunology & Cell Biology*, 2018, vol. 96, 81-99
- **BRUSTLE et al.** *The Journal of Clinical Investigation*, 2012, vol. 122, 4698-4709
- **JAWORSKI et al.** *The EMBO Journal*, 2014, vol. 33, 2765-2781
- **PILATO et al.** *Nature*, 2019, vol. 570, 112-116
- **DEMEYER et al.** *Trends in Molecular Medicine*, 2016, vol. 22, 135-150
- **NAKAMURA et al.** *International Immunopharmacology*, 2018, vol. 56, 193-196
- **ZHANG et al.** *Science Bulletin*, 2019, vol. 64, 1179-1194
- **JUILLAND et al.** *Oncotarget*, 2018, vol. 9, 12542-12543
- **SABA et al.** *Cancer Research*, 2017, vol. 77, 7038-7048
- **LIU et al.** *Journal of Cellular and Molecular Medicine*, 2020, vol. 24, 7550-7562
- **WUTS ; GREENE**. Greene's Protective Groups in Organic Synthesis. John Wiley & Sons Inc., 2006
- **P.J. KOCIENSKI**. Protecting Groups. Thieme, 2005
- **R.C. LAROCK**. Comprehensive Organic Transformations: A Guide to Functional Group Preparations. John Wiley & Sons Inc., 1999
- The Fourth Series of Experimental Chemistry. Chemical Society of Japan, 1992
- **L. KUERTI ; B. CZAKO**. *Strategic Applications of Named Reactions in Organic Synthesis*, 2006
- **G.S. ZWEIFEL ; M.H. NANTZ**. *Modern Organic Synthesis: An Introduction*, 2009
- **P. J. KOCIENSKI**. Protecting Groups. Thiemes, 2005
- **WUTS ; GREENE**. Greene's Protective Groups in Organic Synthesis. John Wiley & Sons Inc, 2006
- **FERINGA et al.** *Org. Biomol. Chem.*, 2008, vol. 6, 3464-3466